# EUROPEAN PATENT SPECIFICATION

(11) **EP 2 101 747 B1**
(45) Date of publication and mention of the grant of the patent: **25.03.2015**
(21) Application number: 07869063.3
(22) Date of filing: 07.12.2007
(51) Int. Cl.: C12Q 1/68

(54) **CANCER CHEMOPREVENTION STRATEGY BASED ON LOSS OF IMPRINTING OF IGF2**
AUF VERLUST VON IGF2-PRÄGUNG BASIERENDE CHEMOPRÄVENTIONSSTRAGIE FÜR KREBS
STRATÉGIE DE CHIMIOPRÉVENTION DU CANCER BASÉE SUR LA PERTE D'EMPREINTE D'IGF2

(30) Priority: 08.12.2006 US 873830 P
(43) Date of publication of application: 23.09.2009
(73) Proprietor: The Johns Hopkins University, Baltimore, MD 21201 (US); The Government of the United States of America as represented by the Secretary of the Department of Health and Human Services, Rockville, MD 20852-3804 (US)
(72) Inventor: FEINBERG, Andrew P., Lutherville, MD 21093 (US); LEVCHENKO, Andre, Ellicott City, MD 21043 (US); LONGO, Dan L., Kensington, MD 20895 (US); KO, Minoru S.H., Cockeysville, MD 21030 (US)
(74) Representative: Lee, Nicholas John
(86) International application number: PCT/US2007/086889
(87) International publication number: WO 2008/073858

(56) References cited:
- WO-A2-2004/010850
- WO-A2-2005/082415
- US-A1- 2006 039 904
- SASAKI JUN-ICHI ET AL: "Clinicopathological characteristics of colorectal cancers with loss of imprinting of insulin-like growth factor 2" INTERNATIONAL JOURNAL OF CANCER, vol. 119, no. 1, July 2006 (2006-07), pages 80-83, XP002562116 ISSN: 0020-7136
- CHEN HUI LING ET AL: "Correction of aberrant imprinting of IGF2 in human tumors by nuclear transfer-induced epigenetic reprogramming" EMBO (EUROPEAN MOLECULAR BIOLOGY ORGANIZATION) JOURNAL, vol. 25, no. 22, November 2006 (2006-11), pages 5329-5338, XP002562117 ISSN: 0261-4189
- KANEDA ATSUSHI ET AL: "Loss of imprinting of IGF2: A common epigenetic modifier of intestinal tumor risk" CANCER RESEARCH, vol. 65, no. 24, December 2005 (2005-12), pages 11236-11240, XP002562118 ISSN: 0008-5472
- CUI H ET AL: "Loss of imprinting in colorectal cancer linked to hypomethylation of H19 and IGF2" CANCER RESEARCH, AMERICAN ASSOCIATION FOR CANCER RESEARCH, BALTIMORE, MD., US, vol. 62, 15 November 2002 (2002-11-15), pages 6442-6446, XP002953235 ISSN: 0008-5472
- CUI H ET AL: "Loss of imprinting in normal tissue of colorectal cancer patients with microsatellite instability" NATURE MEDICINE, NATURE PUBLISHING GROUP, NEW YORK, NY, US, vol. 4, no. 11, 1 November 1998 (1998-11-01), pages 1276-1280, XP002233806 ISSN: 1078-8956
- NAKAGAWA H ET AL: "Loss of imprinting of the insulin-like growth factor II gene occurs by biallelic methylation in a core region of H19-associated CTCF-binding sites in colorectal cancer" PROCEEDINGS OF THE NATIONAL ACADEMY OF SCIENCES OF USA, NATIONAL ACADEMY OF SCIENCE, WASHINGTON, DC, US, vol. 98, no. 2, 16 January 2001 (2001-01-16), pages 591-596, XP002904777 ISSN: 0027-8424
- YUN KANKATSU ET AL: "Analysis of IGF2 gene imprinting in breast and colorectal cancer by allele specific-PCR" JOURNAL OF PATHOLOGY, vol. 187, no. 5, April 1999 (1999-04), pages 518-522, XP002953234 ISSN: 0022-3417
- GARCIA-ECHEVERRIA CARLOS ET AL: "In vivo antitumor activity of NVP-AEW541 - A novel, potent, and selective inhibitor of the IGF-IR kinase" CANCER CELL, vol. 5, no. 3, March 2004 (2004-03), pages 231-239, XP002562119 ISSN: 1535-6108

## Description

### BACKGROUND OF THE INVENTION

### FIELD OF THE INVENTION

The invention relates generally to cancer and, more specifically, to methods of chemoprevention of tumor development by inhibiting signal pathways associated with loss of imprinting (LOI) of insulin-like growth factor 2 (IGF2) gene.

### BACKGROUND INFORMATION

Genomic imprinting is an epigenetic modification in the gamete or zygote that leads to relative silencing of a specific parental allele in somatic cells of the offspring. Loss of imprinting (LOI) of the insulin-like growth factor II gene (IGF2) is defined as aberrant expression of the normally silent maternally inherited allele, which has been found to be associated with a five-fold increased frequency of intestinal neoplasia in humans and a five-fold increased frequency of first degree relatives with colorectal cancer (CRC), suggesting that LOI of IGF2 contributes substantially to the population risk of CRC. Previously, a combined epigenetic-genetic model of intestinal neoplasia was developed, crossing female mice with a deletion of the differentially methylated region (DMR) of *H19* as well as *H19* itself, with male mice harboring a mutation in the *adenomatous polyposis coil* (*Apc*) gene (Min mice). Maternal transmission of the DMR deletion leads to aberrant activation of the maternal *Igf2* allele and LOI, a two-fold increased expression of IGF2 in the intestine, and a 1.8- to 2.5-fold increase in the frequency of intestinal adenomas in LOI(+) Min double heterozygotes. LOI leads to an increase in the progenitor cell (crypt) compartment and increased staining with progenitor cell markers. However, the mechanism for increased tumorigenesis may involve increased proliferation, decreased apoptosis, or an altered maturation program in the crypt, and no differences were seen using proliferation or apoptosis-specific immunostains in that mouse model that might clarify the mechanism. Furthermore, it is not clear that IGF2 itself is responsible for increased tumorigenesis, as alternatively it might reflect other epigenetic disruption, either of *H19* at the locus, or even through trans-effects that have been observed between the *H19* DMR and loci on other chromosomes.

IGF2 is an important autocrine and paracrine growth factor in development and cancer, signaling primarily through the insulin-like growth factor-I receptor (IGF1R), a transmembrane receptor tyrosine kinase. Activation of IGF1R leads to autophosphorylation of the receptor and activation of signaling cascades including the IRS-1/PI3K/AKT and GRB2/Ras/ERK pathways. IGF2 is overexpressed in a wide variety of malignancies, including CRC.

However, how LOI could lead to cancer remains enigmatic. Besides the question of specificity of LOI for the IGF2 signaling cascade as opposed to other cis or trans epigenetic effects associated with LOI, it is not clear how a simple doubling of dosage of IGF2, especially at the relatively low levels of expression found in normal colon, could lead to increased tumor risk. At the same time, if such a mechanistic link could be established, it would open the possibility of chemoprevention similar to the use of statins for reducing cardiovascular risk. That is because an epigenetic state in normal tissue that increases cancer risk might theoretically be reversed, lowering the risk of malignancy even before neoplasms arise.

### SUMMARY OF THE INVENTION

The present invention relates to LOI genes and their gene products in pre-malignant tissues, where methods of inhibiting those LOI gene products can be used to to prevent tumor development in subjects at risk for developing cancer. The present invention also relates to methods of identifying an increased risk in developing certain cancers in a subject, including methods of assessing the efficacy of a chemotherapeutic regimen for that subject. Further, the present invention relates to methods for identifying anti-neoplastic agents.

In one embodiment, a method of preventing tumor development in a subject is disclosed including administering an inhibitor of signal pathway activation by insulin-like growth factor 2 (IGF2), where the subject aberrantly expresses IGF2 due to loss of imprinting.

In one aspect, the subject is at risk of developing colorectal cancer (CRC) as compared with a subject not having LOI in IGF2. In another aspect, the inhibitor is selected from the group consisting of a tyrphostin, a pyrrolo[2,3-d]-pyrimidine, a monoclonal antibody, and a combination thereof. In a related aspect, the pyrrolo[2,3-d]-pyrimidine is NVP-AEW541.

In another aspect, the method of preventing tumor development further includes administering a chemotherapeutic agent, including, but not limited to, Aclacinomycins, Actinomycins, Adriamycins, Ancitabines, Anthramycins, Azacitidines, Azaserines, 6-Azauridines, Bisantrenes, Bleomycins, Cactinomycins, Carmofurs, Carmustines, Carubicins, Carzinophilins, Chromomycins, Cisplatins, Cladribines, Cytarabines, Dactinomycins, Daunorubicins, Denopterins, 6-Diazo-5-Oxo-L-Norleucines, Doxifluridines, Doxorubicins, Edatrexates, Emitefurs, Enocitabines, Fepirubicins, Fludarabines, Fluorouracils, Gemcitabines, Idarubicins, Loxuridines, Menogarils, 6-Mercaptopurines, Methotrexates, Mithramycins, Mitomycins, Mycophenolic Acids, Nogalamycins, Olivomycines, Peplomycins, Pirarubicins, Piritrexims, Plicamycins, Porfiromycins, Pteropterins, Puromycins, Retinoic Acids, Streptonigrins, Streptozocins, Tagafurs, Tamoxifens, Thiamiprines, Thioguanines, Triamcinolones, Trimetrexates, Tubercidins, Vinblastines, Vincristines, Zinostatins, and Zorubicins.

In one aspect, the inhibitor prevents the formation of aberrant crypt foci (ACF).

In another embodiment, a method of identifying an increased risk of developing colorectal cancer in a subject is disclosed including contacting a progenitor cell in a sample from a subject with insulin-like growth factor 2 (IGF2) and determining the sensitivity of the cell to IGF2 as determined by measuring a change in a signal pathway associated with DNA replication, DNA metabolism, cell cycling, apoptosis, and cell proliferation or by measuring a change in gene expression, protein levels, protein modification, or kinetics of protein modification, where an increase in the sensitivity of the progenitor cells to IGF2 correlates with increased risk of developing colorectal cancer.

In one aspect, the method includes determining gene expression changes between LOI positive (LOI(+)) and LOI negative (LOI(-)) progenitor cells, where the progenitor cells are associated with colorectal cancer, identifying genes which are overexpressed in the LOI(+) progenitor cells, contacting LOI (+) and LOI(-) cells with a mutagenic agent, contacting the cells with a ligand which is aberrantly expressed due to loss of imprinting (LOI) of the gene encoding the ligand in the presence and absence of a test agent. In another aspect, the signal pathway is IRS-1/PI3K/AKT or GRB2/Ras/ERK pathway. In a related aspect, the method includes determining the kinetics of modification of a AKT or ERK. In a further related aspect, the modification of AKT or ERK is phosphorylation.

In another aspect, measuring changes in gene expression, protein levels, protein modification, or kinetics of protein modification may be accomplished by monitoring such changes in the genes as set forth in Tables 3 and 5-7. In a related aspect, the genes as recited in Table 3 and 5-7 may be used as a diagnostic for determining risk. In another aspect, the method as discosed may be used in conjunction with methods for diagnosing cancers, including but not limited to, detection of tumor specific antigens/markers, biopsy, cytoscopy, X-rays, CT scans, PAP smears, detection of serum proteins, and the like.

In a related aspect, the method of identifying an increased risk includes contacting the cell with IGF2 in the presence of an inhibitor of IGF1 receptor, where a further decrease in signal pathway activation in the presence of the inhibitor correlates with increased risk of developing colorectal cancer.

In one aspect, the inhibitor is NVP-AEW541. In another aspect, the signal pathway is IRS-1/PI3K/AKT or GRB2/Ras/ERK pathway. In a related aspect, the signal pathway is measured via pathway activation of Akt/PKB.

In one embodiment, a method for identifying an anti-neoplastic agent is disclosed including determining gene expression changes between LOI positive (LOI(+)) and LOI negative (LOI(-)) progenitor cells, wherein the progenitor cells are associated with a neoplastic disorder, identifying genes which are overexpressed in the LOI(+) progenitor cells, contacting LOI+ and LOI- cells with a mutagenic agent, contacting the LOI(+) progenitor cells with a ligand which is aberrantly expressed due to loss of imprinting (LOI) of the gene encoding the ligand in the presence and absence of a test agent; where the ligand is associated with the neoplastic disorder, and determining the sensitivity of the LOI(+) and LOI(-) cells to the ligand in the presence and absence of the test agent, where sensitivity is measured by determining changes in a signal pathway associated with DNA replication, DNA metabolism, cell cycling, apoptosis, and cell proliferation, where a decrease in the sensitivity of the LOI(+) cells to the ligand is inversely proportional to the anti-neoplastic activity of the agent.

In one aspect, the ligand is IGF2. In another aspect, the neoplastic disorder is cancer. In a related aspect, the neoplastic disorder is colorectal cancer (CRC).

In another aspect, the agent reduces the sensitivity of signal transduction induced by the ligand via a cognate receptor for the ligand. In one aspect, the mutagenic agent is a physical agent or chemical agent.

In one aspect, the cells are contained in a microfluidic chip. In another aspect, the cells are contained in a non-human animal.

In another embodiment, a method of assessing the efficacy of a chemotherapeutic regimen is disclosed including periodically isolating a progenitor cell in a sample from a subject receiving a chemotherapeutic agent, contacting the progenitor cell in the sample with insulin-like growth factor 2 (IGF2), and determining the sensitivity of the progenitor cell to IGF2, where sensitivity is measured by determining changes in a signal pathway associated with DNA replication, DNA metabolism, cell cycling, apoptosis, and cell proliferation, where a reduction of the progenitor cell to form aberrant crypt foci (ACF) correlates with the efficacy of the regimen.

### BRIEF DESCRIPTION OF THE DRAWINGS

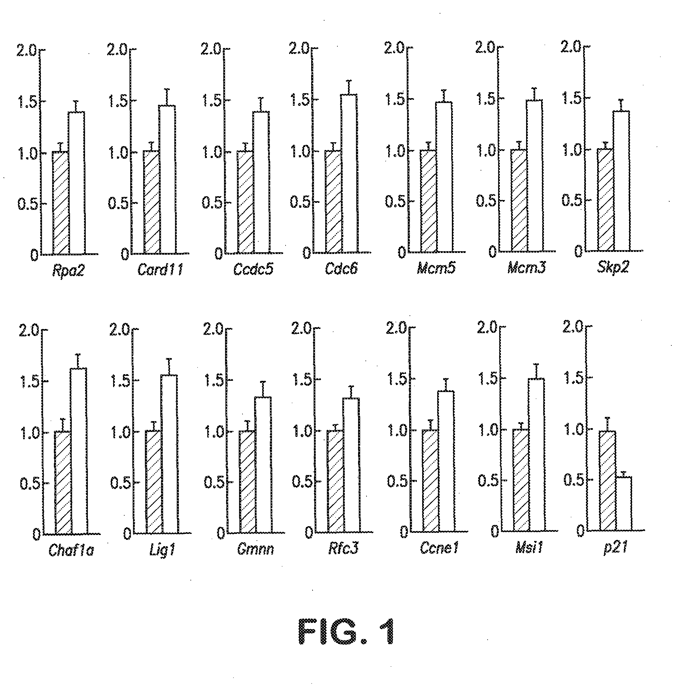

Figure 1 is a bar graph which illustrates the validation of altered expression of genes identified by microarray analysis of intestinal crypts of LOI(+) and LOI(-) mice. Analysis was by quantitative real-time PCR of 10,000 crypts laser capture microdissected from 12 LOI(+) and 9 LOI(-) mice. Expression was normalized to β-actin, and the expression level in LOI(+) samples (grey box) relative to LOI(-) samples (black box) is shown. The bars indicate standard error. *Rpa2,* 1.38-fold (P = 0.03); *Card11,* 1.44-fold (P = 0.04); *Ccdc5,* 1.39-fold (P = 0.03); *Cdc6,* 1.55-fold (P = 0.003); *Mcm5,* 1.47-fold (P = 0.007); *Mcm3,* 1.49-fold (P = 0.002); *Skp2,* 1.37-fold (P = 0.02); *Chaf1a,* 1.61-fold (P = 0.009); *Lig1,* 1.54-fold (P = 0.008) *Gmnn,* 1.33-fold (P = 0.1); *Rfc3,* 1.31-fold (P = 0.04); *Ccne1,* 1.38-fold (P = 0.04). *Msi1* and *p21*/*Cdkn1a* expression were also examined.

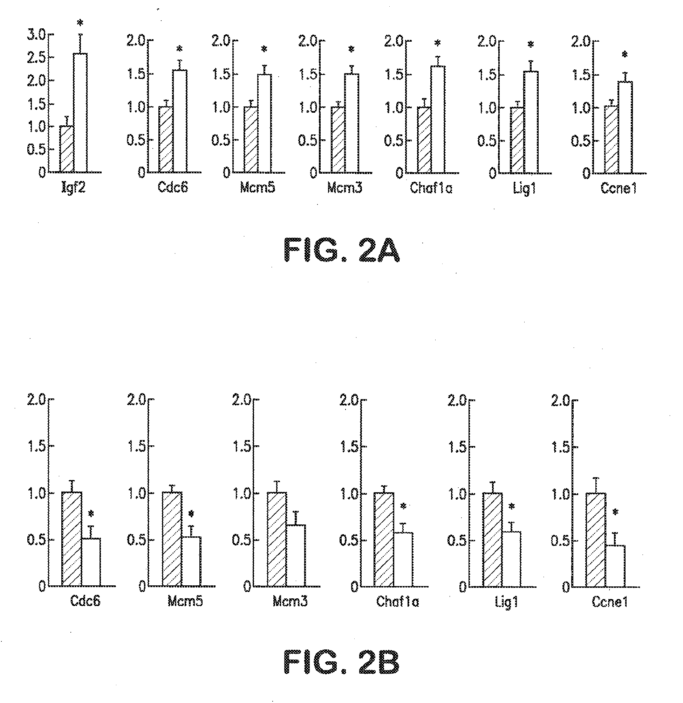

Figure 2 is a bar graph which shows gene expression levels in microdissected intestinal crypts. Qunatitative real-time PCR was performed on laser capture microdissected intestinal crypts from 12 LOI(+) and 9 LOI(-) mice. Expression was normalized to β-actin, and the expression level in LOI(+) samples (gray) relative to LOI(-) samples (black) is shown. The bars indicate standard error. (A) The up-regulated genes in the top ranking GO annotation categories (DNA replication per cell cycle genes listed in Table S 1 3). (B) Receptor inhibition by NVP-AEW541 had a differential effect on proliferation-related gene expression in LOI(+) crypts. Analysis was by quantitative real-time PCR of laser capture microdissected crypts from four LOI(+) mice and four LOI(-) mice treated with NVP-AEW541 for 3 weeks. LOI(+) (gray bars), LOI(-) mice (black bars normalized to 1.0).

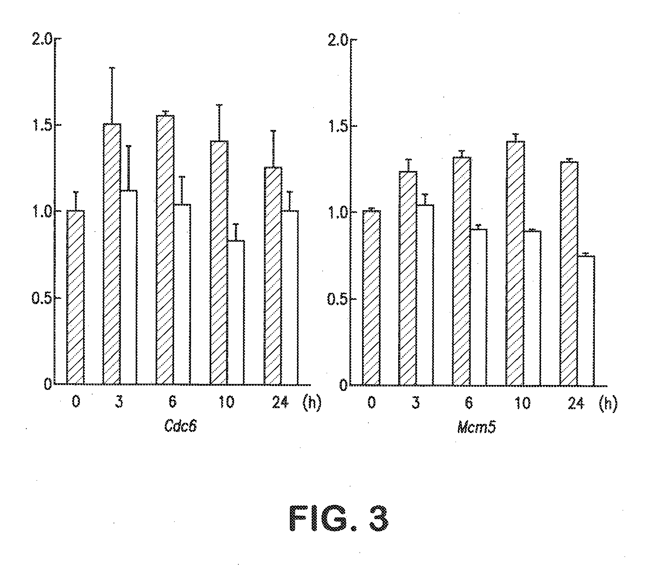

Figure 3 shows bar graph data which demonstrates the induction of *Cdc6* and *Mcm5* gene expression by exogenous IGF2 protein, and inhibition by NVP-AEW541. Mouse ES cells were cultured in defined medium and treated with 800 ng/ml mouse *lgf2* protein. Gene expression was analyzed by real-time RT-PCR and normalized to β-actin. Shown is the expression level without (black boxes) and with (gray boxes) the IGF1R inhibitor 3 µM NVP-AEW541 (a pyrrolo-2,3d-pyrimidine), normalized to time 0. The bars indicate standard error.

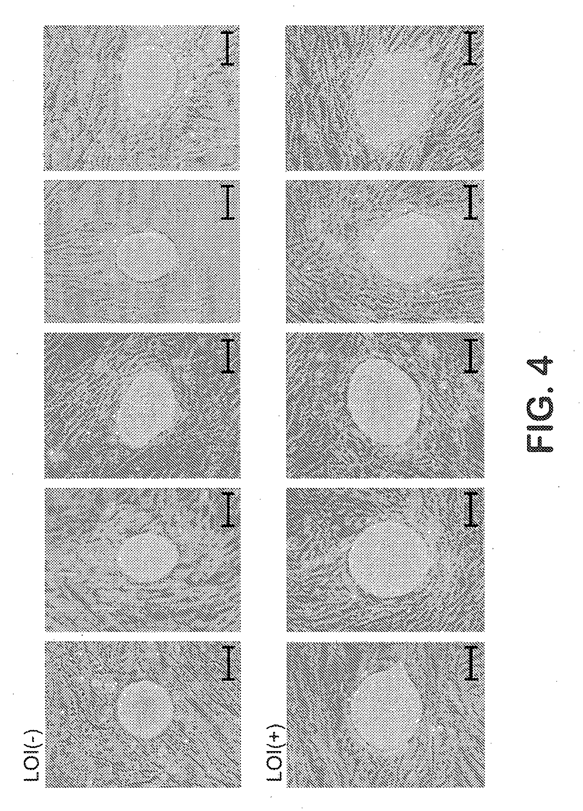

Figure 4 is a photomicrograph which shows the colony size of LOI(-) and LOI(+) ES cells grown on feeder layer cells. 1,000 ES cells each were seeded on 3.5-cm dishes, and the size of 15-30 colonies was measured by photomicrosopy on days 1 through 6. Representative colonies on day 6 are shown. The bar represents 100 µm.

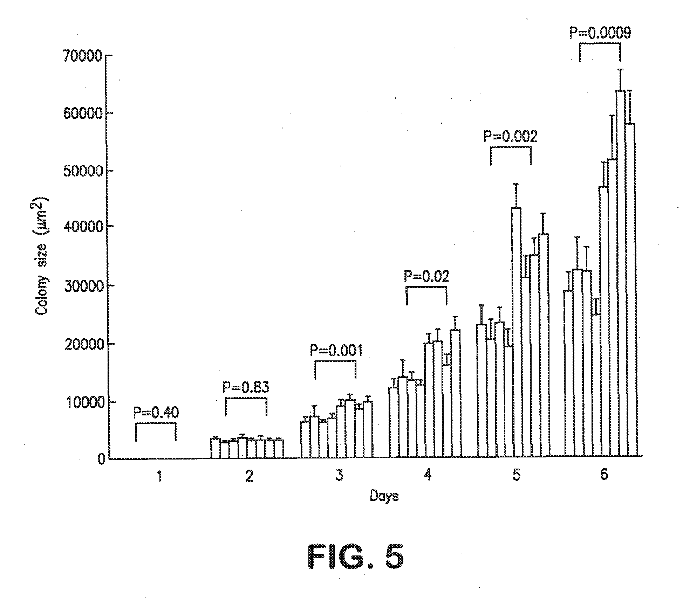

Figure 5 is a graph showing the growth rate of LOI(-) and LOI(+) ES cell colonies grown on feeder layer cells. Four experiments were performed for each cell type using 4 independent LOI(-) and LOI(+) ES cell lines [black boxes, LOI(-); grey boxes, LOI(+)]. Sizes of 15-30 ES cell colonies each were measured on days 1 through 6. The bars indicate standard error.

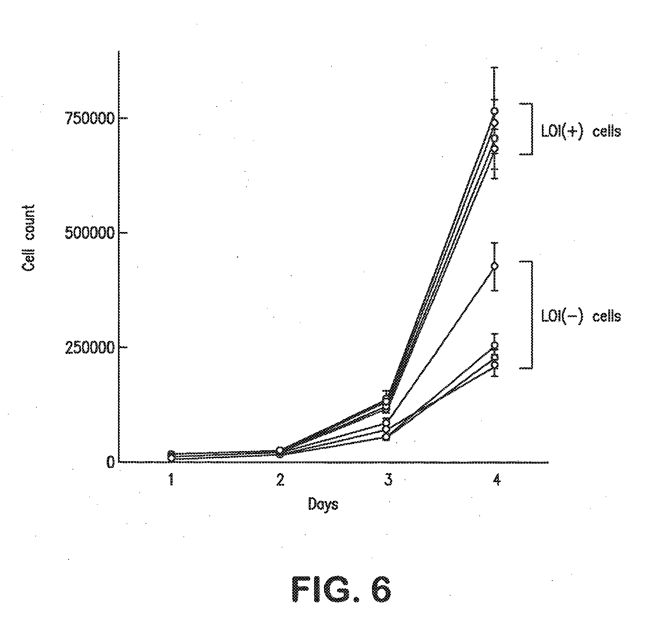

Figure 6 is a graph which shows the growth rate of LOI(-) and LOI(+) ES cells. Four experiments were performed on each cell type, culturing undifferentiated ES cells on gelatin-coated plates without feeder layer cells, using ESGRO Complete Clonal Grade defined medium (Chemicon) without serum or IGF2. Cell growth was determined by counting cells from 3 wells each for days 1 through 6, for four independent LOI(-) and LOI(+) ES cell lines. Doubling time of LOI(+) ES cells was 9.6 ± 0.1 hours (mean ± standard error), 26% faster than LOI(-) ES cells (12.1 ± 0.5 hours, P = 0.01). The bars indicate standard error.

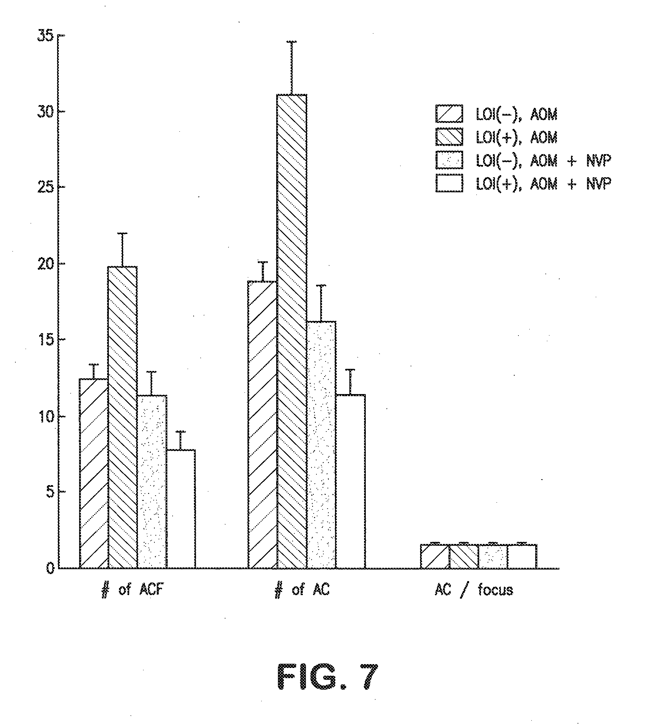

Figure 7 is a graph which demonstrates the inhibition of azoxymethane (AOM)-induced aberrant crypt foci (ACF) by NVP-AEW541. ACF formation in the colon was induced by AOM intraperitoneal injection, and treatment with NVP-AEW541 was by gastric gavage. Each ACF was formed of 1-4 aberrant crypts, and the number of ACF (# of ACF), the number of total aberrant crypts (# of AC), and the average number of aberrant crypts per ACF were measured.

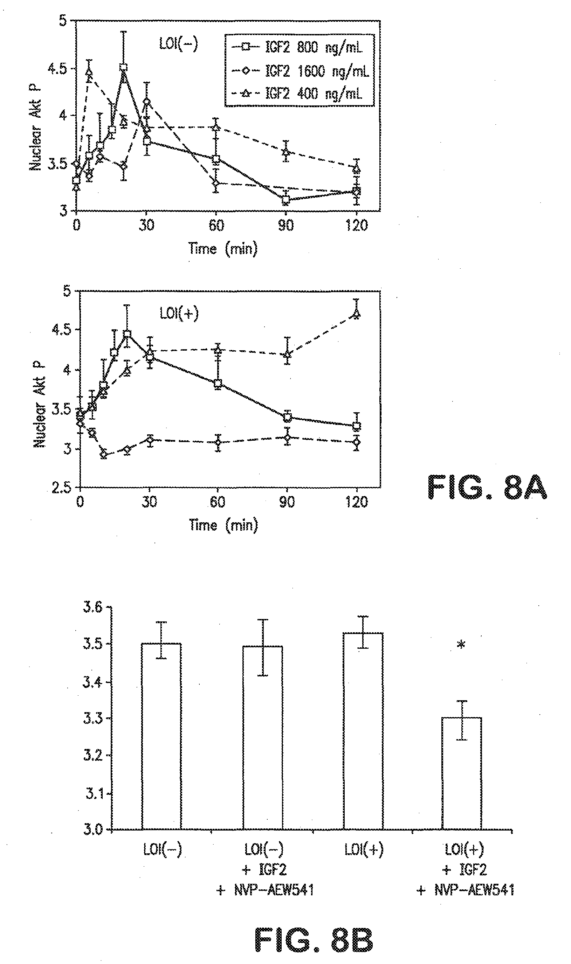

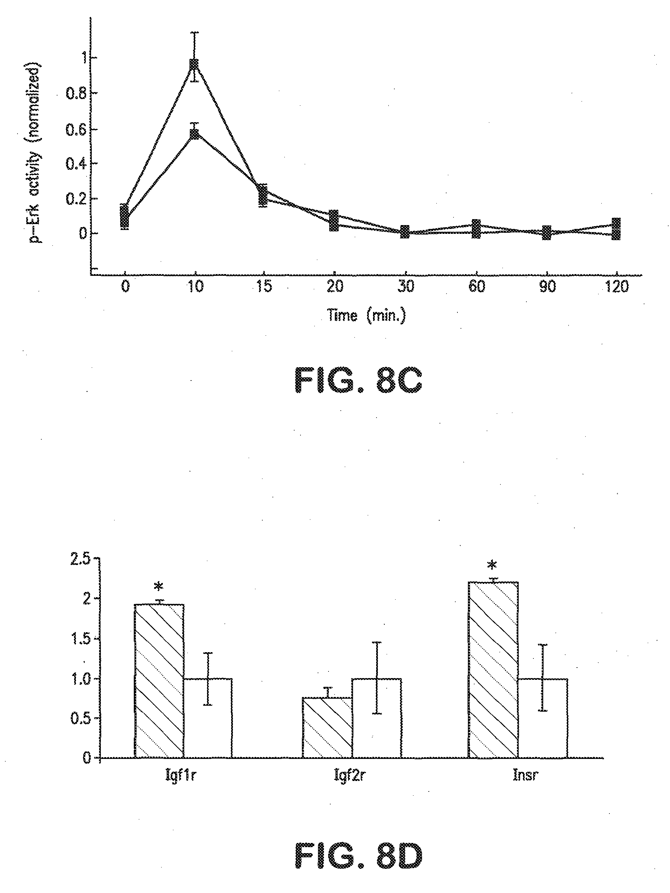

Figure 8 shows a single cell analysis of Akt activation by IGF2 in LOI(+) and LOI(-) mouse embryonic fibroblasts. (A) Akt/PKB activation was assayed by single cell immunocytochemistry with an antibody to phosphorylated Akt (Ser 473), in a monolithic 2-layer PDMS chip sealed with a glass coverslip, with defined media delivery controlled by a multiplexed system of valves. Live LOI(+) and LOI(-) MEF cells were stimulated within the microfluidic chips with varying doses of IGF2, with measurements at multiple time points at each IGF2 concentration. The Y axis shows the ratio of nuclear to background fluorescence. For each cell type, IGF2 concentration, and time point, at least 200 individual cellular measurements were obtained by digital imaging and analysis. (B) Inhibition of Akt activation by NVP-AEW541. The cells were assayed as in (A) at 60 min after coincubation with 400 ng/ml IGF2 and 3 µM NVP-AEW541 and compared with the unstimulated control. Each bar is based on measurements of > 400 cells. Asterisk indicates statistical difference vs LOI(+) control (*t* test, P < 0.001) (C) Single cell analysis of ERK activation by IGF2 in LOI(+) and LOI(-) MEF cells. Erk activation was assayed by single immunocytochemistry within microfluidic chips using an antibody to phosphorylated Erk2 from Upstate (Charlottesville, VA). LOI(+) cells (gray) and LOI(-) cells (black) were exposed to 400 ng/ml IGF2 for indicated times. The y axis shows the ratio of nucleoar to background fluorescence normalized to the maximum level achieved in the LOI(+) cells. Error bars represent SD. Standard error bars are completely subsumed by the symbols on this scale. (D) Gene expression levels in mouse embryonic fibroblasts. Quantitative real-time PCR was performed on LOI(+) and LOI(-) MEF cells, with expression normalized to transferring receptor expression. The expression level in LOI(+) samples (black) relative to LOI(-) samples (gray) is shown. The bars indicate standard error.

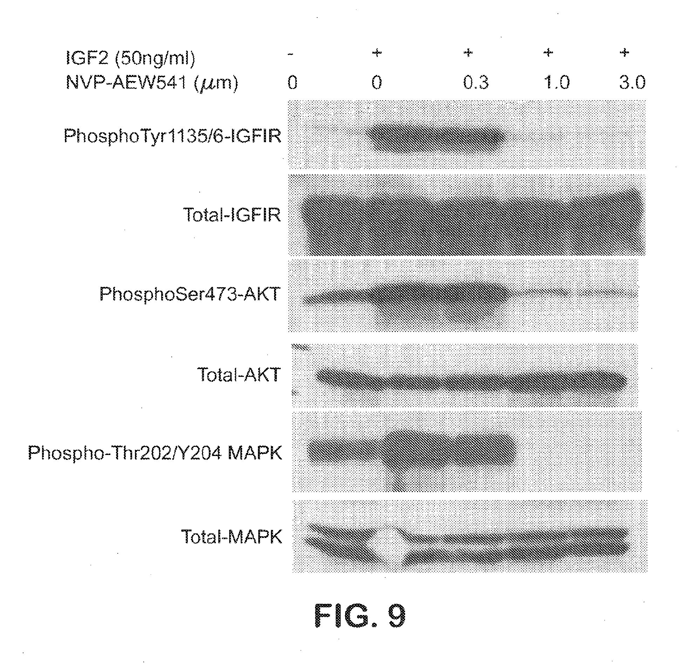

Figure 9 is a western blot which shows the effect of NVP-AEW541 on IGF2 signaling. Confirmation that NVP-AEW541 inhibits IGF2 signaling at the IGF1 receptor was done identically to those performed for IGF1³¹. NIH 3T3 cells were passaged every 3 days and maintained with low glucose DMEM plus 10% CBS in 5% CO₂. The day prior to transfection cells were trypinsized and seeded into PLL-coated glass bottom Mattek dishes. Cells typically reached about 70% confluence the next day, when they were transfected with eGFP-Akt-PH plasmid with Fugene lipid following the manufacturer's instructions. 8 hours after transfection, cells were starved in 0.2% CBS in low glucose DMEM (with no antibiotics) for at least 12 hours. Western blots were performed with the antibodies shown, and varying concentrations of NVP-AEW541, with or without IGF2.

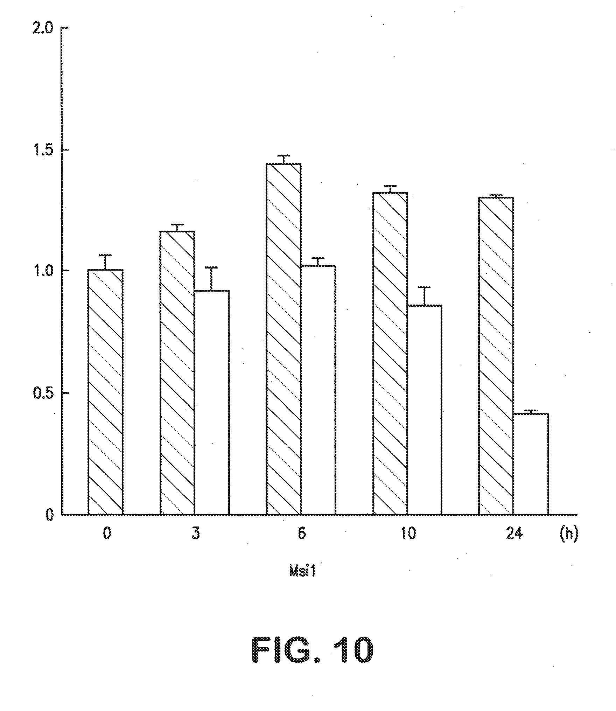

Figure 10 is a bar graph which shows the induction of *Msi1* gene expression by exogenous IGF2 protein, and inhibition by NVP-AEW541. Mouse ES cells were cultured in defined medium and treated with 800 ng/ml mouse 1gf2 protein. Gene expression was analyzed by real-time RT-PCR and normalized to β-actin. Shown is the expression level without (black boxes) and with (pink boxes) the IGF1R R inhibitor 3 µM NVP-AEW541, normalized to time 0. The bar represents standard error.

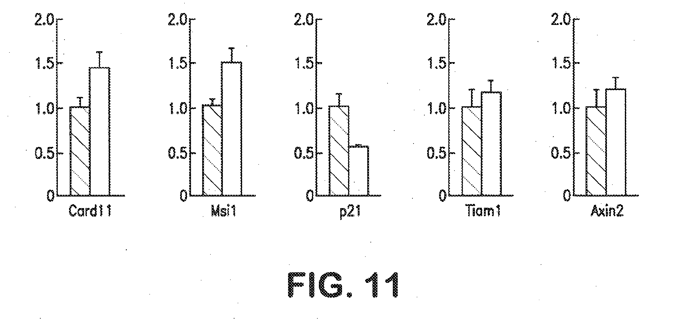

Figure 11 shows bar graphs for gene expression levels in microdissected intestinal crypts. Quantitative real-time PCR was performed on laser capture microdissected intestinal crypts from 12 LOI(+) and 9 LOI(-) mice. Expression was normalized to β-actin, and the expression level in LOI(+) samples (grey) relative to LOI(-) samples (black) is shown. The bars indicate standard error.

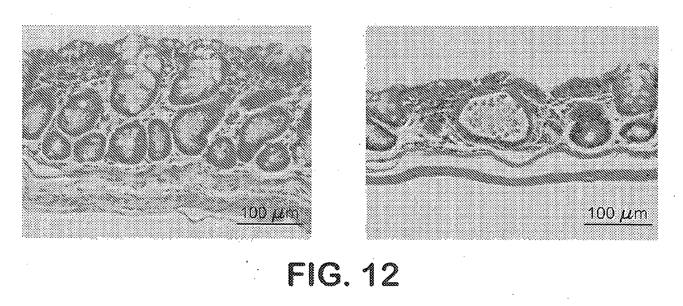

Figure 12 is a photograph showing the histology of the colon in AOM-treated LOI(+) mice. On the left, a representative aberrant crypt focus shows hyperproliferative features including crypt multiplicity, enlargement and elevation over surrounding mucosa. On the right is a cystically dilated crypt lined by enlarged cells with atypical nuclei and containing necrotic debris, reminiscent of sessile serrated adenomas found in the human colon.

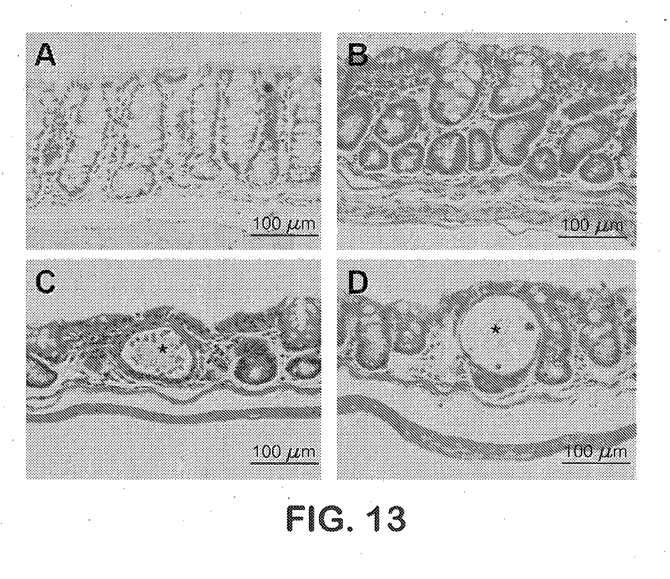

Figure 13 is a photograph showing the histology of the colon in AOM-treated LOI(+) mice. Compared to the normal colonic mucosa shown in panel A that contains crypts of uniform size and orientation, the representative aberrant crypt focus shown in panel B demonstrates hyperproliferative features including crypt multiplicity, enlargement and elevation over surrounding mucosa. In panels C and D, two different examples of cystically dilated crypts lined by enlarged cells with atypical nuclei and containing necrotic debris are shown (indicated by asterisks).

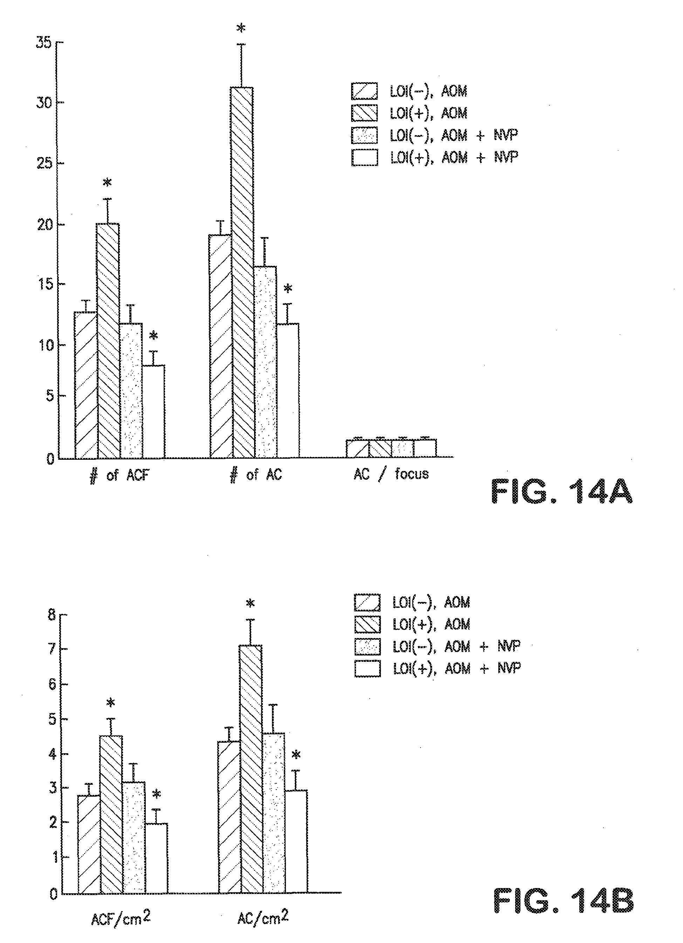

Figure 14 are bar graphs demonstrating the inhibition of azoxymethane (AOM)-induced aberrant crypt foci (ACF) by NVP-AEW541. (A) ACF formation in the colon was induced by AOM i.p. injection, and treatment with NVP-AEW541 was by gastric lavage. Each ACF was formed of one of four aberrant crypts, and the number of ACF (# of ACF), the number of total aberrant crypts (# of AC), and the average number of aberrant crypts per ACF were measured. LOI(+) AOM mice (dark gray bars), LOI(-) AOM mice (black bars), LOI(+) AOM NVP mice (light gray bars), LOI(-) AOM NVP mice (gray bars). (B) The number of ACF and the number of total aberrant crypts (AC) were corrected by colon surface area (cm²).

### DETAILED DESCRIPTION OF THE INVENTION

Before the present compositions and methods are described, it is to be understood that the invention is not limited to the particular methodologies, protocols, cell lines, assays, and reagents described, as these may vary. It is also to be understood that the terminology used herein is intended to describe particular embodiments of the present invention, and is in no way intended to limit the scope of the present invention as set forth in the appended claims.

It must be noted that as used herein and in the appended claims, the singular forms "a," "an," and "the" include plural references unless context clearly dictates otherwise. Thus, for example, a reference to "a ligand" includes a plurality of such ligands, a reference to a "cell" is a reference to one or more cells and to equivalents thereof known to those skilled in the art, and so forth.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described.

The practice of the present invention will employ, unless otherwise indicated, conventional methods of chemistry, biochemistry, molecular biology, cell biology, genetics, immunology and pharmacology, within the skill of the art. Such techniques are explained fully in the literature. See, e.g., Gennaro, A.R., ed. (1990) Remington's Pharmaceutical Sciences, 18th ed., Mack Publishing Co.; Colowick, S. et al., eds., Methods In Enzymology, Academic Press, Inc.; Handbook of Experimental Immunology, Vols. I-IV (D.M. Weir and C.C. Blackwell, eds., 1986, Blackwell Scientific Publications); Maniatis, T. et al., eds. (1989) Molecular Cloning: A Laboratory Manual, 2nd edition, Vols. I-III, Cold Spring Harbor Laboratory Press; Ausubel, F. M. et al., eds. (1999) Short Protocols in Molecular Biology, 4th edition, John Wiley & Sons; Ream et al., eds. (1998) Molecular Biology Techniques: An Intensive Laboratory Course, Academic Press); PCR (Introduction to Biotechniques Series), 2nd ed. (Newton & Graham eds., 1997, Springer Verlag).

Genomic imprinting is a parent of origin-specific gene silencing that is epigenetic in origin, i.e., not involving the DNA sequence per se but methylation and likely other modifications heritable during cell division (Feinberg, A. P., in The Metabolic and Molecular Bases of Inherited Disease, C. R. Scriver, et al., Eds. (McGraw-Hill, New York, 2002)). Loss of imprinting (LOI) of IGF2 was first discovered in embryonal tumors of childhood, such as Wilms tumor (WT), but is one of the most common alterations in cancer, including ovarian, lung, liver, and colon (Feinberg, A. P., in The Metabolic and Molecular Bases of Inherited Disease, C. R. Scriver, et al., Eds. (McGraw-Hill, New York, 2002)). The consequence of LOI is best understood in WT. Here it serves as a gatekeeper in about half of tumors, especially those that occur with relatively late onset, and leads to increased expression of IGF2 (Ravenel, J. D., et al., J Natl. Cancer Inst. 93, 1698-1703 (2001)), an important autocrine growth factor in a wide variety of cancers including CRC (Lahm, H., et al., Br. J. Cancer 65, 341-346 (1992); M. C. Gelato and J. Vassalotti, J. Clin. Endocrinol. Metab. 71, 1168-1174 (1990); El-Badry, O. M., et al., Cell Growth Diff. 1, 325-331 (1990); Yee, D., et al., Cancer Res. 48, 6691-6696 (1988); Lamonerie, T., et al., Int. J. Cancer 61, 587-592 (1995); and Pommier, G. J., et al., Cancer Res. 52, 3182-3188 (1992)).

Epigenetic alterations in human cancers include global DNA hypomethylation, gene hypomethylation and promoter hypermethylation, and loss of imprinting (LOI) of the insulin-like growth factor-II gene (IGF2).

The present invention discloses that LOI increases the expression of proliferation-specific genes in specific tissues, including but not limited to, intestinal crypts. For example, this may be shown by LCM microarray and real-time quantitative PCR, and by in vitro stimulation with IGF2 and its inhibition by IGF1R blockade. Further, the present invention demonstrates that LOI(+) progenitor cells proliferate more rapidly in vitro, as measured by colony size and by growth in defined media. Moreover, IGF1R blockade also reduces the numbers of aberrant crypt foci in LOI(+) subjects exposed to AOM below that of AOM-treated LOI(-) subjects, suggesting that LOI(+) cells are inherently more sensitive to IGF2 signaling, which may be confirmed in vitro using a microfluidic chip (See Examples).

While not being bound by theory, the abrogation of AOM-induced aberrant crypt foci by an IGF2 signaling receptor inhibitor has been exploited to develop a chemopreventive strategy for subjects having neoplastic disorders, including but not limited to, colorectal cancer (CRC) in subjects with LOI. This approach may have a significant public health impact, since 5-10% of the population shows this epigenetic alteration (Cui et al., (2003) Science 299:1752-1755; Woodson et al., J Natl Cancer Inst (2004) 96:407-410), and may include the use of other compounds as disclosed below.

The present invention represents a fundamentally different approach for cancer mortality reduction, compared to screening for the presence of early tumors. For example, in cardiovascular disease prevention, there has been a shift in emphasis toward pharmacologically mediated risk reduction, even (and preferably) in those subjects with no apparent end organ disease at all (Cannon et al., N Engl J Med (2004) 350:1495-1504). In one embodiment, a method of preventing tumor development in a subject is disclosed including administering an inhibitor of signal pathway activation by insulin-like growth factor 2 (IGF2), where the subject aberrantly expresses IGF2 due to loss of imprinting. In one aspect, the inhibitor includes, but is not limited to, a tyrphostin, a pyrrolo[2,3-d]-pyrimidine, a monoclonal antibody and a combination thereof. In a related aspect, the pyrrolo[2,3-d]-pyrimidine is NVP-AEW541.

In one aspect, the inhibitor may be combined with know chemotherapeutic agents, including but not limited to, Aclacinomycins, Actinomycins, Adriamycins, Ancitabines, Anthramycins, Azacitidines, Azaserines, 6-Azauridines, Bisantrenes, Bleomycins, Cactinomycins, Carmofurs, Carmustines, Carubicins, Carzinophilins, Chromomycins, Cisplatins, Cladribines, Cytarabines, Dactinomycins, Daunorubicins, Denopterins, 6-Diazo-5-Oxo-L-Norleucines, Doxifluridines, Doxorubicins, Edatrexates, Emitefurs, Enocitabines, Fepirubicins, Fludarabines, Fluorouracils, Gemcitabines, Idarubicins, Loxuridines, Menogarils, 6-Mercaptopurines, Methotrexates, Mithramycins, Mitomycins, Mycophenolic Acids, Nogalamycins, Olivomycines, Peplomycins, Pirarubicins, Piritrexims, Plicamycins, Porfiromycins, Pteropterins, Puromycins, Retinoic Acids, Streptonigrins, Streptozocins, Tagafurs, Tamoxifens, Thiamiprines, Thioguanines, Triamcinolones, Trimetrexates, Tubercidins, Vinblastines, Vincristines, Zinostatins, and Zorubicins.

In one aspect, the subject is at risk of developing colorectal cancer (CRC) as compared with a subject not having LOI in IGF2. Thus, the present invention provides for a method of screening the general population for LOI, and providing pharmacological intervention that may reduce those at high risk to average or even reduced risk of colon cancer.

In another embodiment, a method of identifying an increased risk of developing colorectal cancer in a subject is disclosed including contacting a progenitor cell in a sample from a subject with insulin-like growth factor 2 (IGF2) and determining the sensitivity of the cell to IGF2 as determined by measuring a change in a signal pathway associated with DNA replication, DNA metabolism, cell cycling, apoptosis, and cell proliferation or by measuring changes in gene expression, protein levels, protein modification, or kinetics of protein modification, where an increase in the sensitivity of the progenitor cells to IGF2 correlates with increased risk of developing colorectal cancer. In one aspect, the method includes determining gene expression changes between LOI positive (LOI(+)) and LOI negative (LOI(-)) progenitor cells, where the progenitor cells are associated with colorectal cancer, identifying genes which are overexpressed in the LOI(+) progenitor cells, contacting LOI (+) and LOI(-) cells with a mutagenic agent, contacting the cells with a ligand which is aberrantly expressed due to loss of imprinting (LOI) of the gene encoding the ligand in the presence and absence of a test agent; wherein the ligand is associated with colorectal cancer, and determining the sensitivity of the LOI(+) and LOI(-) cells to the ligand in the presence and absence of the test agent. In another aspect, the signal pathway is IRS-1/PI3K/AKT or GRB2/Ras/ERK pathway. In a related aspect, the method includes determining the kinetics of modification of a AKT or ERK. In a further related aspect, the modification of AKT or ERK is phosphorylation.

In another aspect, measuring changes in gene expression, protein levels, protein modification, or kinetics of protein modification may be accomplished by monitoring such changes in the genes as set forth in Tables 3 and 5-7. In a related aspect, the genes as recited in Table 3 and 5-7 may be used as a diagnostic for determining risk in conjunction with methods as disclosed for cancers including, but not limited to, breast cancer, prostate cancer, cervical cancer, pancreatic cancer, gastric cancers, esophageal cancer, ovarian cancer, skin cancer, including methods as disclosed in, but not limited to, U.S. Pat. Nos. 7,264,928; 7,063,944; 6,890,514; 6,696,262; 6,720,189; 6,645,770; 6,410,335; 6,383,817; 6,282,305; 5,773,215. For example, such methods may include, but are not limited to, detection of tumor specific antigens/markers, biopsy, cytoscopy, X-rays, CT scans, PAP smears, detection of serum proteins, and the like.

In another embodiment, a method of assessing the efficacy of a chemotherapeutic regimen is disclosed including periodically isolating a progenitor cell in a sample from a subject receiving a chemotherapeutic, contacting the progenitor cell in the sample with insulin-like growth factor 2 (IGF2), and determining the sensitivity of the progenitor cell to IGF2, where sensitivity is measured by determining changes in a signal pathway associated with DNA replication, DNA metabolism, cell cycling, apoptosis, and cell proliferation, where a reduction of the progenitor cell to form aberrant crypt foci (ACF) correlates with the efficacy of the regimen.

The present invention also provides data for an "epigenetic progenitor model", which demonstrates that there is a polyclonal change in the numbers and states of progenitor cells that arises prior to the genetic mutation, and increases the risk of cancer when a mutation occurs stochastically (Feinberg et al., Nat Rev Genet (2006) 7:21-33). Thus, LOI and cancer risk has been confirmed in a second animal model, and while not being bound by theory, a plausible mechanism for this progenitor cell expansion has been offered, namely increased IGF2 sensitivity in LOI(+) cells, leading to increased proliferation of progenitor cells. The present invention demonstrates that LOI is a paradigm for other epigenetic changes in apparently normal cells of subjects at risk of cancer, and includes other genes aberrantly expressed due to LOI, DNA methylation, or chromatin differences that distinguish non-tumor cells of subjects with cancer, or subjects at risk of cancer, from their non-cancer cohorts.

The present invention also demonstrates the absence of any ACF reduction by the IGF2 inhibitor in LOI(-) mice, and the presence of a reduction by the inhibitor of the numbers of ACF in LOI(+) mice below that seen in LOI(-) mice. While not being bound by theory, this result suggests that cells with LOI have enhanced sensitivity to low doses of IGF2, which is confirmed by studying downstream Akt/PKB signaling in a novel microfluidic chip system (See Examples). This in vitro analysis showed a marked potentiation of downstream IGF2 signaling in cells with LOI.

Again, not to be bound by theory, the increased sensitivity to IGF2 at low dose could help to explain the relationship between receptor-mediated signaling and cell growth. Based on the results described herein, proliferating cells may be more sensitive to a ligand at low density, with a relatively low accumulated IGF2. As cell density increases, autocrine and paracrine stimulation progressively increases local interstitial concentration of IGF2 causing a diminished effect on cellular proliferation, similar to that seen in in vitro experiments (see Examples), and providing an important check on growth control as the tissue reaches a critical size. In one embodiment, the difference in sensitivity of LOI(+) cells is used to favor an increased therapeutic ratio of IGF2 inhibitors for chemoprevention, since subjects (or cells) with normal imprinting would be relatively refractory to the drug. In a related aspect, for cancer risk control, subjects with higher risk might be lowered to an even lower risk category than baseline through targeted intervention as disclosed herein.

As used herein, when hypomethylation is measured, "the degree of LOI" means the percentage of methylation compared to a fully methylated DMR. As used herein, when expression of different polymorphisms is compared, "the degree of LOI" means total expression (as measured by actual expression or transcription) attributable to the allele which is normally imprinted. The degree of LOI may be calculated by allele ratio, i.e., the more abundant allele divided by the less abundant allele. The degree of LOI may be determined by any method which allows the determination of the relative expressions of the two alleles. For example, a degree of LOI of 100% reflects complete LOI (equal expression of both alleles), while a degree of LOI of 0% reflects no LOI (expression of only one allele). Any method of measuring the relative expression of the two alleles is considered to be included in the present invention.

Methods for detecting loss of imprinting are typically quantitative methods for analyzing imprinting status. The presence or absence of LOI may be detected by examining any condition, state, or phenomenon which causes LOI or is the result of LOI. Such conditions, states, and phenomena include, but are not limited to:
1. Causes of LOI, such as the state or condition of the cellular machinery for DNA methylation, the state of the imprinting control region on chromosome 11, the presence of trans-acting modifiers of imprinting, the degree or presence of histone deacetylation;
2. State of the genomic DNA associated with the genes or gene for which LOI is being assessed, such as the degree of DNA methylation;
3. Effects of LOI, such as:
   a. Relative transcription of the two alleles of the genes or gene for which LOI is being assessed;
   b. Post-transcriptional effects associated with the differential expression of the two alleles of the genes or gene for which LOI is being assessed;
   c. Relative translation of the two alleles of the genes or gene for which LOI is being assessed;
   d. Post-translational effects associated with the differential expression of the two alleles of the genes or gene for which LOI is being assessed;
   e. Other downstream effects of LOI, such as altered gene expression measured at the RNA level, at the splicing level, or at the protein level or post-translational level (i.e., measure one or more of these properties of an imprinted gene's manifestation into various macromolecules); changes in function that could involve, for example, cell cycle, signal transduction, ion channels, membrane potential, cell division, or others (i.e., measure the biological consequences of a specific imprinted gene being normally or not normally imprinted (for example, QT interval of the heart). Another group of macromolecular changes include processes associated with LOI such as histone acetylation, histone deacetylation, or RNA splicing.

The degree of LOI can be measured for the IGF2 gene when screening for the presence of colorectal cancer, or other cancers, e.g., the degree of LOI is measured for the IGF2 gene when screening for the presence of stomach cancer, esophageal cancer, or leukemia.

A linear detection platform can be employed to quantitate LOI. A linear detection platform is a detection platform that allows quantitation because the amount of target present and signal detected are linearly related. In this regard, a PhosphorImager (model 4458I, manufactured by Molecular Dynamics), which detects radioactive emissions directly from a gel, can be used. Other linear detection systems include carefully titrated autoradiography followed by image analysis, beta-emission detection analysis (Betascan). Another linear detection platform is an automated DNA sequencer such as ABI 377 analyzer. Another linear detection platform is an array based system with appropriate software. Another is SNuPE.

In addition to measuring the degree of imprinting when an imprinted polymorphism is present in a gene, it is possible to assess the degree of LOI in a particular gene even when an imprinted polymorphism is not present in that gene. For example, imprinting can be assessed by the degree of methylation of CpG islands in or near an imprinted gene (e.g., Barletta, Cancer Research, op. cit). In addition, imprinting can be assessed by changes in DNA replication timing asynchrony, e.g., White L M, Rogan P K, Nicholls R D, Wu B L, Korf B. Knoll J H, Allele-specific replication of 15q11-q 13 loci: a diagnostic test for detection of uniparental disomy. American Journal of Human Genetics. 59:423-30, 1996.

On the other hand, certain techniques are more conveniently used when there is a polymorphism in the two alleles of the gene or genes for which the presence or absence of LOI is being measured. For example, RT-PCR, followed by gel electrophoresis to distinguish length polymorphisms, or RT-PCR followed by restriction enzyme digestion, or by automated DNA sequencing, or by single strand conformational polymorphism (SSCP) analysis, or denaturing gradient gel electrophoresis, etc.; or, completely DNA based methods that exploit, for example DNA methylation, which require no RT step, to convert RNA to cDNA prior to PCR.

Once the degree of LOI, such as the level of hypomethylation, has been measured for the gene or genes in question, the risk of having cancer is then assessed by comparing the degree of LOI for that gene or genes to a known relationship between the degree of LOI and the probability of the presence of the particular type of cancer or other disease. The relationship between the degree of LOI and the probability of the presence of a particular type of cancer may be determined for any combination of a normally imprinted gene or genes and a particular type of cancer by determining.

When the degree of LOI is measured, such as the degree of IGF2 hypomethylation, the measured degree of LOI is compared to a known relationship between the degree of LOI and the probability of contracting the particular type of cancer. The relationship between the degree of LOI and the probability of contracting a particular type of cancer may be determined by one of ordinary skill in the art for any combination of a normally imprinted gene or genes and a particular type of cancer by determining the degree of LOI in a statistically meaningful number of tissue samples obtained from patients with cancer, and determining the degree of LOI in a statistically meaningful number of tissue samples obtained from patients without cancer, and then calculating an odds ratio as a function of the degree of LOI.

It should also be understood that measuring the degree of LOI, can be carried out by comparing the degree of LOI against one or more predetermined threshold values, such that, if the degree of LOI is below a given threshold value, which can be manifested in a regular methylation pattern, then the subject is assigned to a low risk population for having cancer, contracting cancer, and/or having replication error repair defects. Alternatively, the analytical technique may be designed not to yield an explicit numerical value for the degree of LOI, but instead yield only a first type of signal when the degree of LOI is below a threshold value and/or a second type of signal when the degree of LOI is below a threshold value. It is also possible to carry out the present methods by means of a test in which the degree of LOI is signaled by means of a non-numeric spectrum such as a range of colors encountered with litmus paper.

Although many conventional genetic mutations have been observed in human cancer, most do not occur at high frequency in the general population. Certain embodiments of the present invention are based on the finding of an association between loss of imprinting (LOI) of the IGF2 gene and family history of colorectal cancer (CRC) and between LOI of the IGF2 gene and present or past personal history of colorectal neoplasia. Accordingly, methods of the present invention analyze common molecular markers of cancer risk to identify an increased risk of developing cancer in a subject.

Certain embodiments of the present invention are based on the finding that loss of imprinting of the IGF2 gene is associated with cancers such as colorectal cancer, and that loss of imprinting of the IGF2 gene is correlated with hypomethylation of both the IGF2 gene and the H 19 gene.

Accordingly, one aspect of the present invention relates to a method for identifying an increased risk of developing cancer in a subject.

A method of the present invention can also be used to infer a cancer risk of a subject.

As illustrated in the Example section, the present invention in certain embodiments, provides a method of identifying an increased risk of developing colorectal cancer in a subject including contacting a LOI(+) progenitor cell in a sample from a subject with insulin-like growth factor 2 (IGF2) and determining the sensitivity of the cell to IGF2 as determined by measuring a change in a signal pathway associated with DNA replication, DNA metabolism, cell cycling, apoptosis, and cell proliferation, where an increase in the sensitivity of the LOI(+) progenitor cell to IGF2 correlates with increased risk of developing colorectal cancer.

Loss of imprinting, an epigenetic alteration affecting the insulin-like growth factor II gene (IGF2), is found in normal colonic mucosa of approximately 30% of colorectal cancer (CRC) patients, compared to 10% of those without colorectal neoplasia (Cui, H., et al., Nat. Med. 4, 1276-1280 (1998)). Therefore, LOI occurs at a relatively high rate in CRC patients and in patients without colorectal neoplasia.

In the study provided in Example 1, 11 of 123 (9.0%) of patients with no family history of CRC showed LOI in lymphocytes, compared to 13 of 49 (27%) with a positive family history (adjusted odds ratio 4.41, 95% CI 1.62-12.0, p=0.004). Similarly, 7 of 106 (6.6%) patients without past or present colonic neoplasia showed LOI, compared to 12 of 56 (21%) patients with adenomas, and 5 of 9 (56%) patients with CRC (adjusted odds ratios 4.10 [95% CI 1.30-12.8, p=0.016] and 34.4 [95% CI 6.10-194, p<0.001], respectively). These data support the usefulness and effectiveness of methods of the present invention in identifying an increased risk of developing cancer.

A method according to the present invention can be performed during routine clinical care, for example as part of a general regular checkup, on a subject having no apparent or suspected neoplasm such as cancer. Therefore, the present invention in certain embodiments, provides a screening method for the general population. The methods of the present invention can be performed at a younger age than present cancer screening assays, for example where the method can be performed on a subject under 65, 55, 50, 40, 35, 30, 25, or 20 years of age.

If the biological sample of the subject in question is found to exhibit LOI, for example as the result of contacting a progenitor cell in a sample from a subject with a gene that is aberrantly expressed due to LOI and determining the sensitivity of the cell to LOI(+) gene, as determined by measuring a change in a signal pathway associated with DNA replication, DNA metabolism, cell cycling, apoptosis, and cell proliferation, where an increase in the sensitivity of the progenitor cells to IGF2 correlates with increased risk of developing cancer, then that subject is identified as having an increased probability of having cancer. In these embodiments, further diagnostic tests may be carried out to probe for the possibility of cancer being present in the subject. Examples of such further diagnostic tests include, but are not limited to, chest X-ray, carcinoembryonic antigen (CEA) or prostate specific antigen (PSA) level determination, colorectal examination, endoscopic examination, MRI, CAT scanning, or other imaging such as gallium scanning, and barium imaging. Furthermore, the method of the invention can be coincident with routine sigmoidoscopy/colonoscopy of the subject. The method could involve use of a very thin tube, or a digital exam to obtain a colorectal sample.

The method of the present invention, especially when used to detect local LOI, can be repeated at regular intervals. While not wanting to be limited to a particular theory, methods directed to detecting local LOI by analyzing a blood sample for LOI, typically identify germline mutations. Therefore, typically one test is sufficient. However, for methods used to detect local LOI, a third sample can be isolated, for example from colorectal tissue, for example at least 2 months after isolation of the second sample. For example, the third sample can be isolated at about 1 year after the second sample was isolated. In fact, the method can be repeated annually, for example at an annual routine physical exam. Using this regular testing, a method of the present invention is used to screen for an increased risk of developing colorectal cancer by a method that includes contacting a LOI(+) progenitor cell in a sample from a subject with insulin-like growth factor 2 (IGF2) and determining the sensitivity of the cell to IGF2 as determined by measuring a change in a signal pathway associated with DNA replication, DNA metabolism, cell cycling, apoptosis, and cell proliferation, where an increase in the sensitivity of the LOI(+) progenitor cells to IGF2 correlates with increased risk of developing colorectal cancer.

Additional diagnostic tests can be performed in the future, even if no cancer is present at the time LOI is detected. For example, if LOI is detected in a biological sample of a subject and indicates an increased risk of contracting cancer, periodic (e.g., every 1 to 12 months) chest X-rays, colorectal examinations, endoscopic examination, MRI, CAT scanning, other imaging such as gallium scanning, and/or barium imaging can be scheduled for that subject. Therefore, in these embodiments, LOI is used as a screening assay to identify subjects for whom more frequent monitoring is justified.

According to the present invention, the biological or tissue sample can be drawn from any tissue that is susceptible to cancer. For example, the tissue may be obtained by surgery, biopsy, swab, stool, or other collection method. The biological sample for methods of the present invention can be, for example, a sample from colorectal tissue, or in certain embodiments, can be a blood sample, or a fraction of a blood sample such as a peripheral blood lymphocyte (PBL) fraction. Methods for isolating PBLs from whole blood are well known in the art. In addition, it is possible to use a blood sample and enrich the small amount of circulating cells from a tissue of interest, e.g., colon, breast, etc. using a method known in the art.

When the method of the present invention provides a method for identifying an increased risk of developing colorectal cancer, a biological sample can be isolated from the colon. Such a tissue sample can be obtained by any of the above described methods, or by the use of a swab or biopsy. In the case of stomach and esophageal cancers, the tissue sample may be obtained by endoscopic biopsy or aspiration, or stool sample or saliva sample. In the case of leukemia, the tissue sample is typically a blood sample.

As disclosed above, the biological sample can be a blood sample. The blood sample can be obtained using methods known in the art, such as finger prick or phlebotomy. Suitably, the blood sample is approximately 0.1 to 20 ml, or alternatively approximately 1 to 15 ml with the volume of blood being approximately 10 ml.

Accordingly, in one embodiment, the identified cancer risk is for colorectal cancer, and the biological sample is a tissue sample obtained from the colon, blood, or a stool sample. In another embodiment, the identified cancer risk is for stomach cancer or esophageal cancer, and the tissue may be obtained by endoscopic biopsy or aspiration, or stool sample or saliva sample. In another embodiment, the identified cancer risk is esophageal cancer, and the tissue is obtained by endoscopic biopsy, aspiration, or oral or saliva sample. In another embodiment, the identified cancer risk is leukemia/lymphoma and the tissue sample is blood.

In the present invention, the subject is typically a human but also can be any mammalian organism, including, but not limited to, a dog, cat, rabbit, cow, bird, rat, horse, pig, or monkey.

As mentioned above, for certain embodiments of the present invention, the method is performed as part of a regular checkup. Therefore, for these methods the subject has not been diagnosed with cancer, and typically for these present embodiments it is not known that a subject has a hyperproliferative disorder, such as a colorectal neoplasm.

Methods of the present invention identify a risk of developing cancer for a subject. A cancer can include, but is not limited to, colorectal cancer, esophageal cancer, stomach cancer, leukemia/lymphoma, lung cancer, prostate cancer, uterine cancer, breast cancer, skin cancer, endocrine cancer, urinary cancer, pancreas cancer, other gastrointestinal cancer, ovarian cancer, cervical cancer, head cancer, neck cancer, and adenomas. In one aspect, the cancer is colorectal cancer.

A hyperproliferative disorder includes, but is not limited to, neoplasms located in the following: abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital. Typically, as used herein, the hyperproliferative disorder is a cancer. In certain aspects, the hyperproliferative disorder is colorectal cancer.

The method can further include analysis of a second biological sample from the subject at a target tissue for loss of imprinting of the IGF2 gene, wherein a loss of imprinting in the second sample is indicative of an increased risk of developing cancer in the target tissue. In certain embodiments, the second biological sample is not a blood sample. For example, the first biological sample can be a blood sample and the second biological sample can be isolated from colorectal tissue.

In another embodiment, the present invention provides a method for managing health of a subject. The method includes performing the method for identifying an increased risk of developing cancer discussed above and performing a traditional cancer detection method. For example, a traditional cancer detection method can be performed if the method for identifying cancer risk indicates that the subject is at an increased risk for developing cancer. Many traditional cancer detection methods are known and can be included in this aspect of the invention. The traditional cancer detection method can include, for example, one or more of chest X-ray, carcinoembryonic antigen (CEA) level determination, colorectal examination, endoscopic examination, MRI, CAT scanning, or other imaging such as gallium scanning, and barium imaging, and sigmoidoscopy/colonoscopy, a breast exam, or a prostate specific antigen (PSA) assay.

In another embodiment, the present invention provides a method for prognosing cancer risk of a subject. The method includes contacting a LOI(+) progenitor cell in a sample from a subject with insulin-like growth factor 2 (IGF2) and determining the sensitivity of the cell to IGF2 as determined by measuring a change in a signal pathway associated with DNA replication, DNA metabolism, cell cycling, apoptosis, and cell proliferation; wherein an increase in the sensitivity of the LOI(+) progenitor cells to IGF2 correlates with increased risk of developing colorectal cancer

In another aspect, the present invention provides a method for identifying predisposition to colorectal cancer of a subject. The method includes contacting the cell with IGF2 in the presence of an inhibitor of IGF1 receptor, wherein a further decrease in signal pathway activation in the presence of the inhibitor correlates with increased risk of developing colorectal cancer. In this aspect of the invention, the first biological sample is typically a colorectal sample.

When detecting the presence or absence of LOI by relying on any one of these conditions, states, or phenomena, it is possible to use a number of different specific analytical techniques. In particular, it is possible to use any of the methods for determining the pattern of imprinting known in the art. It is recognized that the methods may vary depending on the gene to be analyzed.

Conditions, states, and phenomena which may cause LOI and may be examined to assess the presence or absence of LOI include the state or condition of the cellular machinery for DNA methylation, the state of the imprinting control region on chromosome 11, the presence of trans-acting modifiers of imprinting, the degree or presence of histone deacetylation or histone deacetylation, imprinting control center, transacting modulatory factors, changes in chromatin caused by polycomb-like proteins, trithorax-like proteins, human homologues of other chromatin-affecting proteins in other species such as Su(var) proteins in Drosophila, SIR proteins in yeast, mating type silencing in yeast, or XIST-like genes in mammals.

It is also possible to detect LOI by examining the DNA associated with the gene or genes for which the presence or absence of LOI is being assessed. By the term "the DNA associated with the gene or genes for which the presence or absence of LOI is being assessed" it is meant the gene, the DNA near the gene, or the DNA at some distance from the gene (as much as a megabase or more away, e.g., methylation changes can be that far away, since they act on chromatin over long distances). Typically, for the present invention LOI is identified or analyzed or detected by detecting hypomethylation of a DMR of the IGF2 gene and/or of a DMR of the H 19 gene, as described herein.

The degree of methylation in the DNA, associated with the gene or genes for which the presence or absence of LOI is being assessed, can be measured or identified using a number of analytical techniques.

Numerous methods for analyzing methylation status of a gene are known in the art and can be used in the methods of the present invention to identify either hypomethylation or hypermethylation of the IGF2 gene. For example, analysis of methylation can be performed by bisulfite genomic sequencing. Accordingly, denatured genomic DNA can be treated with freshly prepared bisulfite solution at 55°C. in the dark overnight, followed by column purification and NaOH treatment. Bisulfite treatment modifies DNA converting unmethylated, but not methylated, cytosines to uracil.

It will be recognized primers may be designed depending on the site bound by the primer and the direction of extension from a primer. The regions amplified and/or otherwise analyzed using primer pairs can be readily identified by a skilled artisan using sequence comparison tools and/or by analyzing nucleotides fragments that are replicated using the primers. Therefore, it will be understood that identification of the binding sites for these primers using computational methods, will take into account that the primers can preferably bind to a polynucleotide whose sequence is modified by bisulfite treatment.

Bisulfite treatment can be carried out using the CpG Genome DNA Modification kit (Intergen, Purchase, N.Y.). For sequencing individual clones, the PCR products can be subcloned into a TA Cloning vector (Invitrogen, Carlsbad, Calif.) according to the manufacturer's instructions, and a series of clones, such as 10-15 clones, can be selected for sequencing.

PCR products can be purified using the QIAEX II gel extraction kit (Qiagen) and directly sequenced with an ABI Prism 377 DNA sequencer using the BIGDYE^{™}. Terminator Cycle Sequencing kit following the manufacturer's protocol (PE Applied Biosystems, Foster City, Calif.).

Altered methylation can be identified by identifying a detectable difference in methylation. For example, hypomethylation can be determined by identifying whether after bisulfite treatment a uracil or a cytosine is present at specific residues. If uracil is present after bisulfite treatment, then the residue is unmethylated. Hypomethylation is present when there is a measurable decrease in methylation, or a measurable decrease in methylation of residues corresponding to methylated positions within the polynucleotides analyzed using select primers.

In an alternative embodiment, an amplification reaction can be preceded by bisulfite treatment, and the primers can selectively hybridize to target sequences in a manner that is dependent on bisulfite treatment. For example, one primer can selectively bind to a target sequence only when one or more base of the target sequence is altered by bisulfite treatment, thereby being specific for a methylated target sequence.

Other methods are known in the art for determining methylation status of a gene, such as the IGF2 gene, including, but not limited to, array-based methylation analysis and Southern blot analysis.

Methods using an amplification reaction, for example methods above for detecting hypomethylation of the IGF2 DMR can utilize a real-time detection amplification procedure. For example, the method can utilize molecular beacon technology (Tyagi S., et al., Nature Biotechnology, 14: 303 (1996)) or TAQMAN^{™} technology (Holland, P. M., et al., Proc. Natl. Acad. Sci. USA, 88:7276 (1991)).

Also methyl light (Trinh B N, Long T I, Laird P W. DNA methylation analysis by MethyLight technology, Methods, 25(4):456-62 (2001), Methyl Heavy (Epigenomics, Berlin, Germany), or SNuPE (single nucleotide primer extension) (See e.g., Watson D., et al., Genet Res. 75(3):269-74 (2000)). Can be used in the methods of the present invention related to identifying altered methylation of IGF2.

As used herein, the term "selective hybridization" or "selectively hybridize" refers to hybridization under moderately stringent or highly stringent physiological conditions, which can distinguish related nucleotide sequences from unrelated nucleotide sequences.

As known in the art, in nucleic acid hybridization reactions, the conditions used to achieve a particular level of stringency will vary, depending on the nature of the nucleic acids being hybridized. For example, the length, degree of complementarity, nucleotide sequence composition (for example, relative GC:AT content), and nucleic acid type, i.e., whether the oligonucleotide or the target nucleic acid sequence is DNA or RNA, can be considered in selecting hybridization conditions. An additional consideration is whether one of the nucleic acids is immobilized, for example, on a filter. Methods for selecting appropriate stringency conditions can be determined empirically or estimated using various formulas, and are well known in the art (see, for example, Sambrook et al., supra, 1989).

An example of progressively higher stringency conditions is as follows: 2 X SSC/0.1% SDS at about room temperature (hybridization conditions); 0.2 X SSC/0.1% SDS at about room temperature (low stringency conditions); 0.2 X SSC/0.1% SDS at about 42°C. (moderate stringency conditions); and 0.1 X SSC at about 68°C. (high stringency conditions). Washing can be carried out using only one of these conditions, for example, high stringency conditions, or each of the conditions can be used, for example, for 10 to 15 minutes each, in the order listed above, repeating any or all of the steps listed.

The degree of methylation in the DNA associated with the gene or genes for which the presence or absence of LOI is being assessed, may be measured by fluorescent in situ hybridization (FISH) by means of probes which identify and differentiate between genomic DNAs, associated with the gene for which the presence or absence of LOI is being assessed, which exhibit different degrees of DNA methylation. FISH is described in the Human chromosomes: principles and techniques (Editors, Ram S. Verma, Arvind Babu Verma, Ram S.) 2nd ed., New York: McGraw-Hill, 1995, and de Capoa A., Di Leandro M., Grappelli C., Menendez F., Poggesi I., Giancotti P., Marotta, M. R., Spano A., Rocchi M., Archidiacono N., Niveleau A. Computer-assisted analysis of methylation status of individual interphase nuclei in human cultured cells. Cytometry. 31:85-92, 1998. In this case, the biological sample will typically be any which contains sufficient whole cells or nuclei to perform short term culture. Usually, the sample will be a tissue sample that contains 10 to 10,000, or, for example, 100 to 10,000, whole somatic cells.

Additionally, as mentioned above, methyl light, methyl heavy, and array-based methylation analysis can be performed, by using bisulfite treated DNA that is then PCR-amplified, against microarrays of oligonucleotide target sequences with the various forms corresponding to unmethylated and methylated DNA.

As mentioned above, methods for detecting LOI can identify altered methylation patterns. However, other methods for detecting LOI are known. For example, certain methods for detecting LOI identify allele-specific gene expression and rely upon the differential transcription of the two alleles. For these methods, RNA is reverse transcribed with reverse transcriptase, and then PCR is performed with PCR primers that span a site within an exon where that site is polymorphic (i.e., normally variable in the population), and this analysis is performed on an individual that is heterozygous (i.e., informative) for the polymorphism. A number of detection schemes can be used to determine whether one or both alleles is expressed. See also, Rainier et al. (1993) Nature 362:747-749; which teaches the assessment of allele-specific expression of IGF2 by reverse transcribing RNA and amplifying cDNA by PCR using new primers that permit a single round rather than nested PCR; Matsuoka et al. (1996) Proc. Natl. Acad Sci USA 93:3026-3030 which teaches the identification of a transcribed polymorphism in p57^{K1P2}; Thompson et al. (1996) Cancer Research 56:5723-5727 which teaches determination of mRNA levels by RPA and RT-PCR analysis of allele-specific expression of p57^{K1P2} ; and Lee et al. (1997) Nature Genet. 15:181185 which teaches RT-PCR SSCP analysis of two polymorphic sites. In this case, the biological sample will be any which contains sufficient RNA to permit amplification and subsequent reverse transcription followed by polymerase chain reaction. Typically, the biological sample will be a tissue sample which contains 1 to 10,000,000, 1000 to 10,000,000, or 1,000,000 to 10,000,000, somatic cells.

LOI may also be detected by reliance on other allele-specific downstream effects. For example, depending on the metabolic pathway in which lies the product of the imprinted gene; the difference will be 2 X versus 1 X (or some number in between) of the product, and therefore the function or a variation in function specific to one of the alleles. For example, for IGF2, increased mitogenic signaling at the IGF1 receptor, increased occupancy of the IGF1 receptor, increased activity at the IGF2 catabolic receptor, decreased apoptosis due to the dose of IGF2; for KvLQT1, change in the length of the QT interval depending on the amount and isoform of protein, or change in electrical potential, or change in activity when the RNA is extracted and introduced into Xenopus oocytes.

The term "nucleic acid molecule" is used broadly herein to mean a sequence of deoxyribonucleotides or ribonucleotides that are linked together by a phosphodiester bond. As such, the term "nucleic acid molecule" is meant to include DNA and RNA, which can be single stranded or double stranded, as well as DNA/RNA hybrids. Furthermore, the term "nucleic acid molecule" as used herein includes naturally occurring nucleic acid molecules, which can be isolated from a cell, for example, the IGF2 gene, as well as synthetic molecules, which can be prepared, for example, by methods of chemical synthesis or by enzymatic methods such as by the polymerase chain reaction (PCR), and, in various embodiments, can contain nucleotide analogs or a backbone bond other than a phosphodiester bond.

The terms "polynucleotide" and "oligonucleotide" also are used herein to refer to nucleic acid molecules. Although no specific distinction from each other or from "nucleic acid molecule" is intended by the use of these terms, the term "polynucleotide" is used generally in reference to a nucleic acid molecule that encodes a polypeptide, or a peptide portion thereof, whereas the term "oligonucleotide" is used generally in reference to a nucleotide sequence useful as a probe, a PCR primer, an antisense molecule, or the like. Of course, it will be recognized that an "oligonucleotide" also can encode a peptide. As such, the different terms are used primarily for convenience of discussion.

A polynucleotide or oligonucleotide comprising naturally occurring nucleotides and phosphodiester bonds can be chemically synthesized or can be produced using recombinant DNA methods, using an appropriate polynucleotide as a template. In comparison, a polynucleotide comprising nucleotide analogs or covalent bonds other than phosphodiester bonds generally will be chemically synthesized, although an enzyme such as T7 polymerase can incorporate certain types of nucleotide analogs into a polynucleotide and, therefore, can be used to produce such a polynucleotide recombinantly from an appropriate template

In another aspect, the present invention includes kits that are useful for carrying out the methods of the present invention. The components contained in the kit depend on a number of factors, including: the condition, state, or phenomenon relied on to detect LOI or measure the degree of LOI, the particular analytical technique used to detect LOI or measure the degree of LOI, and the gene or genes for which LOI is being detected or the degree of LOI is being measured.

The following examples are intended to illustrate but not limit the invention.

### EXAMPLES

### Materials and Methods

### Mice and Genotyping.

Mice with C57BL/6J background carrying a deletion in the H19 gene (3 kb) and 10 kb of the upstream region including the differentially methylated region (DMR) regulating IGF2 silencing were obtained from S. Tilghman (Princeton University) and maintained by breeding female wild-type C57B/6J and male H19+/-. Mice with biallelic IGF2 expression, and control littermates were isolated by crossing female H19+/- with male wild-type mice. Mice were genotyped by PCR which identified an 847 bp product for the wild type allele and a 1,000-bp product for the mutant allele, using the following primers: H19-F, TCC CCT CGC CTA GTC TGG AAG CA (SEQ ID NO:1); Mutant-F, GAA CTG TTC GCC AGG CTC AAG (SEQ ID NO:2); Common-R, ACA GCA GAC AGC AAG GGG AGG GT (SEQ ID NO:3).

Since strain variation was known to be associated with the progression of the lesions, the littermate controls were treated with and without LOI, in which the dams were heterozygous for a deletion of the *H19* differentially methylated region (DMR); inheritance of a maternal allele lacking the DMR leads to activation of normally silent allele of *Igf2* [LOI(+)], whereas inheritance of a wild-type maternal allele leads to normal imprinting [LOI(-)].

### Microarray Analysis.

In an initial pilot evaluation, total RNA was extracted from fresh frozen full thickness intestine using the RNeasy Kit, assessed using a Bioanalyzer (Agilent), and 2.7 µg of total RNA was labeled and hybridized to a National Institute on Aging (NIA) mouse 44k microarray (Version 2.0, manufactured by Agilent, #12463). Initially two sets of 6 samples were compared, three LOI(+) from males to three LOI(-) from females, and a separate analysis of three LOI(+) from females and three LOI(-) from males, confirming the sensitivity of the comparison by the detection of known gender-specific differences including Xist, Eif2s3y, and Ddx3y. Statistical analysis was done using NIA array analysis software (Sharove et al., Bioinformatic (2005) 21-2548-2549). Genes showing consistent and statistically significant changes (P ≤ 0.05) in both sets were analyzed for enrichment in Gene Ontogeny categories using the NIA Mouse Gene Index (Ver. Mm5) *Sharov et al., Genome Res (2005) 15:748-754). This can be found at the NIA Mouse Gene Index website hosted by the National Institutes of Health, Bethesda Maryland. For validation, 14 LOI(-) and 14 LOI(+) RNA samples were collected similarly and used for real-time RT-PCR.

To detect gene expression change in intestinal progenitor cells more definitively, laser capture microdissection (LCM) was performed to isolate intestinal crypt cells. Slides were pretreated with RNAzap (Ambion), rinsed with DEPC-treated water, dried, and UV-irradiated, then frozen intestines were embedded in OCT, sectioned at 10 µm, and fixed with 70% ethanol on the slides. Slides were stained with hematoxylin (Sigma), dehydrated and used for LCM within one week. 5,000-13,000 intestinal crypts were dissected by LCM from each of three LOI(+) and LOI(-) mice, and 2-6 µg of RNA were collected using the RNeasy Kit (Qiagen). 1.7 µg of total RNA from each sample was used for labelling, and gene expression was analyzed with a NIA mouse 44k microarray (Ver 2.1, manufactered by Agilent, #014117). Genes were examined for statistically significant enrichment in Gene Ontogeny categories.

For validation, LCM was performed on an additional 12 LOI(+) and 9 LOI(-) mice, isolating approximately 800 crypts yielding more than 300 ng of total RNA from each. RNA samples were reverse-transcribed using SUPERSCRIPT II (Invitrogen), and quantified using SYBR Green PCR Core Reagents and an ABI Prism 7700 Sequence Detection System (Applied Biosystems), and normalized to β-actin. Primers and annealing temperatures are provided in Table 1.

**Table 1. Primers for real-time RT-PCR.**

| Genes | Primers (forward; reverse; annealing temperature; product length) |
|---|---|
| *Igf2* | cat cgt gga aga gtg ctg ct (SEQ ID NO:4); ggg tat ctg ggg aag tcg t (SEQ ID NO:5); 62 °C; 132 bp |
| *Axin2* | aac aca gaa gac agc tcc tca (SEQ ID NO:6); gtc tga atc gat ggt aaa cct g (SEQ ID NO:7); 59 °C; 166 bp |
| *Tiam1* | cac ttc aag gag cag ctc agc (SEQ ID NO:8); gct cag tcg atc ctc tcc ac (SEQ ID NO:9); 59 °C; 190 bp |
| *Rpa2* | atg gat gtt cgt cag tgg gtt (SEQ ID NO:10); cca gag gaa tga tct taa agg c (SEQ ID NO:11); 60°C; 145 bp |
| *Card11* | gaa gac gag gtg ctc aat gc (SEQ ID NO:12); cct ttg tcc ctt ggt gtg aa (SEQ ID NO:13); 60°C; 90 bp |
| *Ccdc5* | ggg aca tca gcc tgg taa tag a (SEQ ID NO:14); ctt aga cag att ggc agg tga a (SEQ ID NO:15); 60°C; 122 bp |
| *Cdc6* | tgt gga gtc gga tgt cag ga (SEQ ID NO:16); ggg ata tgt gag caa gac caa (SEQ ID NO:17); 60°C; 107 bp |
| *Mcm5* | cca ggt cat gct caa gtc aga (SEQ ID NO:18); gaa tgg aga tac gag tag cct t (SEQ ID NO:19); 60°C; 140 bp |
| *Mcm3* | cgc aga gag act act tgg act tc (SEQ ID NO:20); agc cga tac tgg ttg tca ctg (SEQ ID NO:21); 60°C; 97 bp |
| *Skp2* | agt caa ggg caa agg gag tg (SEQ ID NO:22); gag gca cag aca gga aaa gat (SEQ ID NO:23); 60°C; 136 bp |
| *Chaf1a* | tcc cag tga aga ggt taa tac aag (SEQ ID NO:24); gat gtg tct tcc tca act ttc tc (SEQ ID NO:25); 60°C; 85 bp |
| *Lig1* | cgg aca ttt gag aag att gcg g (SEQ ID NO:26); aga tag aga aca ggg agc aag tc (SEQ ID NO:27); 60°C; 119 bp |
| *Gmnn* | tga aaa taa gga tgt tgg aga cc (SEQ ID NO:28); gcc act tct ttc caa tac tga g (SEQ ID NO:29); 60°C; 90 bp |
| *Rfc3* | cca cct tga agt taa tcc cag t (SEQ ID NO:30); tgt cca cct ctg tca ata ata cc (SEQ ID NO:31); 60°C; 143 bp |
| *Ccne1* | agt tct tct gga ttg gct gat g (SEQ ID NO:32); gta acg atc aaa gaa gtc ctg tg (SEQ ID NO:33); 60°C; 91 bp |
| *Msil* | tgc tgg gta ttg gga tgc t (SEQ ID NO:34); tcg ggg aac tgg tag gtg ta (SEQ ID NO:35); 60°C; 103 bp |
| *p21* | aca gcg ata tcc aga cat tca ga (SEQ ID NO:36); cga aga gac aac ggc aca ct (SEQ ID NO:37); 60°C; 99bp |
| *β-actin* | tac cac cat gta ccc agg ca (SEQ ID NO:38); gga gga gca atg atc ttg at (SEQ ID NO:39); 60°C; 93 bp |

### Establishment of Mouse Embryonic Stem (ES) Cells and Mouse Embryonic Fibroblasts (MEFs).

Timed mating was performed between female H19 mutant mice and male wild type mice after intraperitoneal injection of 5 IU pregnant mare serum gonadotropin, followed two days later with 5 IU of human chorionic gonadotropin. On embryonic day 13.5, embryos were isolated, digested with trypsin, seeded onto 10-cm cell culture dishes, and split twice at 1:3-1:4 before being frozen. Genomic DNA was extracted for genotyping *H19* (thus identifying LOI status). For ES cells, timed mating was performed between 4-week old female *H19* mutant mice and 8-10 week old male wild type mice. On embryonic day 3.5, the uteri were flushed and embryos were collected and cultured as described Cowan et al., ES Cell Targeting Core Laboratory, 2006). Inner cell mass outgrowths were aspirated and plated. Eight clones were successfully expanded to 3.5-cm dishes. For ES colony size assays, ES cells and feeder layer cells were trypsinized and seeded on gelatin-coated plates for 30 minutes to let the feeder layer cells attach, and the supernatant was aspirated and underwent this procedure once more. The predominantly ES nonadhered population was counted, and 1,000 cells were seeded on a feeder layer in 6-well plate on day 0, measuring the sizes of 15-30 randomly chosen ES colonies on days 1 through 6. ES growth rate assays were performed in ESGRO Complete Clonal Grade Medium (Chemicon). After collecting predominantly ES nonadhered population as above, cells were split at 1:4 density twice more in ESGRO Complete medium to eliminate any feeder layer cells. 200,000 ES cells were then seeded on gelatin-coated 6-well plate without a feeder layer on day 0, and cell number was determined on days 1, 2, 3 and 4. The analysis was performed in triplicate, and blinded for genotype.

For IGF2 stimulation, wild type ES cells were isolated as described above, and cultured in ESGRO Complete Basal Medium with 800 ng /ml of mouse Igf2 recombinant protein (StemCell Technologies), with or without 3 µM NVP-AEW541 (Novartis). Cells were washed with PBS twice and RNA was extracted using the RNeasy Kit (QIAGEN) at 0h, 3h, 6h, 10h, and 24 h. Gene expression was assayed by real-time quantitative RT-PCR as described above.

### Azoxymethane and NVP-AEW541 Administration In Vivo.

7 week old LOI(+) and LOI(-) littermate controls were treated with AOM at 10mg/kg body weight intraperitoneally once per week for three weeks, and euthanized 5 weeks after the first dose of AOM. The entire colon was resected from mice after laparotomy, flushed with PBS, filled with 10% buffered formalin (Sigma) for one minute, opened longitudinally, fixed flat between filter paper in formalin at 4 °C overnight, rinsed with PBS, and stained with 0.2% methylene blue in saline. The number of ACF per colon and the number of aberrant crypts per ACF were scored under a light microscopy as previously described Bird, Cancer Lett (1987) 37:147-151). NVP-AEW541 (50 mg/kg) was administered by oral gavage, beginning 1 week before AOM treatment, and NVP-AEW541 was administered 7 days/week, twice a day for 5 weekdays and once a day for 2 weekend days, for 6 weeks until sacrifice.

### Microfluidic Chamber Assays.

The immunostaining automation device consists of a monolithic 2-layer PDMS chip sealed with a glass coverslip, fabricated using techniques described previously (Unger et al., Science 288 (2000) 288:113-116). Multiplexing valves were actuated by pressure lines connected to off-device solenoid valves. The chip architecture, performance and validation are all described in a separate manuscript submitted for publication. The glass substrate of the cell culture chamber was coated by introducing 0.1% gelatin (Sigma) into the device for at least 30 minutes, then cells were introduced and allowed to attach for 3 hours. IGF2 (StemCell Technologies) dissolved in cell media was introduced to each chamber at different times so that the end of all stimulation periods coincided. To end the experiment, ice-cold PBS (Invitrogen) was introduced into all chambers followed immediately by 4% paraformaldehyde (Electron Microscopy Systems) for 20 minutes. Cells were permeabilized with 0.1% Triton X-100 (Sigma) for 5 min and blocked with 10% goat serum (Sigma), with PBS washes in between. The cells were incubated with 1:100 anti-phospho-Akt primary antibody (Ser 473, Upstate) in blocking solution for 1 hour, washed in PBS, then incubated with 1:200 Alexa 488-conjugated goat anti-rabbit secondary antibody (Invitrogen), 1:500 Hoechst 33258 (Sigma), plus 1:40 Texas Red phalloidin (Invitrogen) in blocking solution for 1 hour. Finally, cells were washed and kept in PBS to prevent drying during imaging. Imaging was performed using a motorized Zeiss inverted epifluorescence microscope equipped with a Cascade 512B CCD camera. Using custom MATLAB (Mathworks) programs, the images were Hatfield-corrected, stitched together, and quantified. Briefly, the Hoechst image was used to determine the nuclear region for each cell and the average staining intensity in this region was compared to background. For each cell type and time point, 200-300 cells were analyzed in this manner to ensure statistical significance.

Since both the endogenous IGF2 and IGF2 used in the MEF experiments are susceptible to IGF2 binding protein (IBP)-based inactivation, the dose of an IBP-resistant IGF2 variant was determined producing similar levels of Akt activation. This level was found to be approximately 50 ng/ml (6.5 nM; Akt activation is equivalent to that obtained in approximately 600 ng/ml of the IBP sensitive IGF1), comparably to the previously estimated kD of IGF1R for IGF2 (1-10 nM), indicating that receptor saturation was not achieved. These results suggest that the experiments were carried out in a sub-saturation regime, including the case when the does of IBP-sensitive IGF2 was increased up to 1600 ng/ml.

Given that the effect of LOI of IGF2 is only a two-fold change in gene expression and protein (Sakatani et al., Science 307 (2005) 307:1976-1978) an approach was designed to detect with sufficient statistical power modest to moderate changes in downstream gene expression. Preliminary experiments were first performed comparing gene expression in full thickness intestine derived from 6 LOI(+) and 6 LOI(-) littermates, by extracting RNA and hybridizing to a mouse 44K microarray of 60-mer oligos representing 23,933 genes (Carter et al., Genome Biol (2005) 6:R61). 508 genes were found with increased expression and 147 genes with decreased expression in the intestine of LOI(+) mice. Gene Ontology (GO) annotations showed that among genes overexpressed in the intestine of LOI(+) mice, there was an overrepresentation in intestinal expression in LOI(+) mice of genes showing increased expression involved in the DNA replication (P < 10⁻¹⁰), cell cycle (P < 10⁻⁷), and cholesterol biosynthesis and metabolism (each P < 10⁻¹²) categories, and of genes showing decreased expression involved in carbohydrate, glucose and monosaccharide metabolism (each P < 10⁻¹⁵)(Table 2).

**Table 2. Genes with significant differences in expression in LOI(+) mice found by microarray analysis of laser capture microdissected crypts.**

| Upregulated in LOI | | | | | |
|---|---|---|---|---|---|
| GO-annotation Category | Genes upregulated in category | Total GO-annotated upregulated genes | Total GO-annotated genes in category | Total GO-annotated genes | P value |
| Cholesterol biosynthesis | 5 | 312 | 17 | 12933 | 10⁻¹² |
| Cholesterol metabolism | 8 | 312 | 41 | 12933 | 10⁻¹² |
| Sterol metabolism | 8 | 312 | 44 | 12933 | 10⁻¹¹ |
| Sterol biosynthesis | 5 | 312 | 19 | 12933 | 10⁻¹¹ |
| DNA replication and chromosome cycle | 13 | 312 | 103 | 12933 | 10⁻¹¹ |
| DNA replication | 11 | 312 | 83 | 12933 | 10⁻¹⁰ |
| RNA metabolism | 20 | 312 | 252 | 12933 | 10⁻⁸ |
| Cell cycle | 32 | 312 | 527 | 12933 | 10⁻⁷ |
| Steroid Metabolism | 10 | 312 | 92 | 12933 | 10⁻⁷ |
| RNA binding | 23 | 312 | 354 | 12933 | 10⁻⁶ |
| Cell proliferation | 36 | 312 | 692 | 12933 | 10⁻⁶ |
| Muscle development | 9 | 312 | 92 | 12933 | 10⁻⁵ |
| Pre-mRNA splicing factor activity | 5 | 312 | 35 | 12933 | 10⁻⁵ |
| Steroid biosynthesis | 6 | 312 | 49 | 12933 | 10⁻⁵ |
| Mitosis | 8 | 312 | 82 | 12933 | 10⁻⁵ |
| Nucleic acid binding | 81 | 312 | 2185 | 12933 | 10⁻⁵ |
| M phase of mitotic cell cycle | 8 | 312 | 83 | 12933 | 10⁻⁵ |
| Nucleoside, nucleotide, and nucleic acid metabolism | 79 | 312 | 2142 | 12933 | 10⁻⁴ |
| Metabolism | 157 | 312 | 5100 | 12933 | 10⁻⁴ |
| Mitotic cell cycle | 10 | 312 | 129 | 12933 | 10⁻⁴ |
| RNA processing | 13 | 312 | 196 | 12933 | 10⁻⁴ |
| Muscle contraction | 6 | 312 | 60 | 12933 | 10⁻⁴ |
| Nuclear division | 9 | 312 | 115 | 12933 | 10⁻⁴ |
| Cell growth and/or maintenance | 102 | 312 | 3061 | 12933 | 10⁻⁴ |
| Structural constituent of cytoskeleton | 8 | 312 | 98 | 12933 | 10⁻⁴ |

| Downregulated in LOI | | | | | |
|---|---|---|---|---|---|
| GO-annotation Category | Genes downregulated in category | Total GO-annotated downregulated genes | Total GO-annotated genes in category | Total GO-annotated genes | P value |
| Glucose metabolism | 7 | 73 | 76 | 12933 | 10⁻¹⁵ |
| Energy derivation by oxidation of organic compounds | 8 | 73 | 112 | 12933 | 10⁻¹⁵ |
| Energy pathways | 8 | 73 | 115 | 12933 | 10⁻¹⁵ |
| Hexose metabolism | 7 | 73 | 94 | 12933 | 10⁻¹⁵ |
| Monosaccharide metabolism | 7 | 73 | 95 | 12933 | 10⁻¹⁵ |
| Main pathways of carbohydrate metabolism | 6 | 73 | 80 | 12933 | 10⁻¹⁵ |
| Carbohydrate metabolism | 10 | 73 | 271 | 12933 | 10⁻¹¹ |
| Alcohol metabolism | 7 | 73 | 162 | 12933 | 10⁻¹⁰ |
| Lipid metabolism | 8 | 73 | 379 | 12933 | 10⁻⁴ |

A limitation of this analysis is that it was based on full thickness intestine, yet the progenitor cell compartment, i.e. crypts, are specifically altered histopathologically in LOI (Sakatani et al. (2005)). Therefore compartment-specific gene expression was examined by microdissecting an average of 8,000 crypts from each of 3 LOI(+) and 3 LOI(-) mice, yielding >2 µg RNA from each mouse, sufficient for an independent microarray experiment. This analysis revealed a more limited subset of differentially expressed genes in LOI(+) crypts, with 283 genes with increased expression and 109 genes with decreased expression (Table 3).

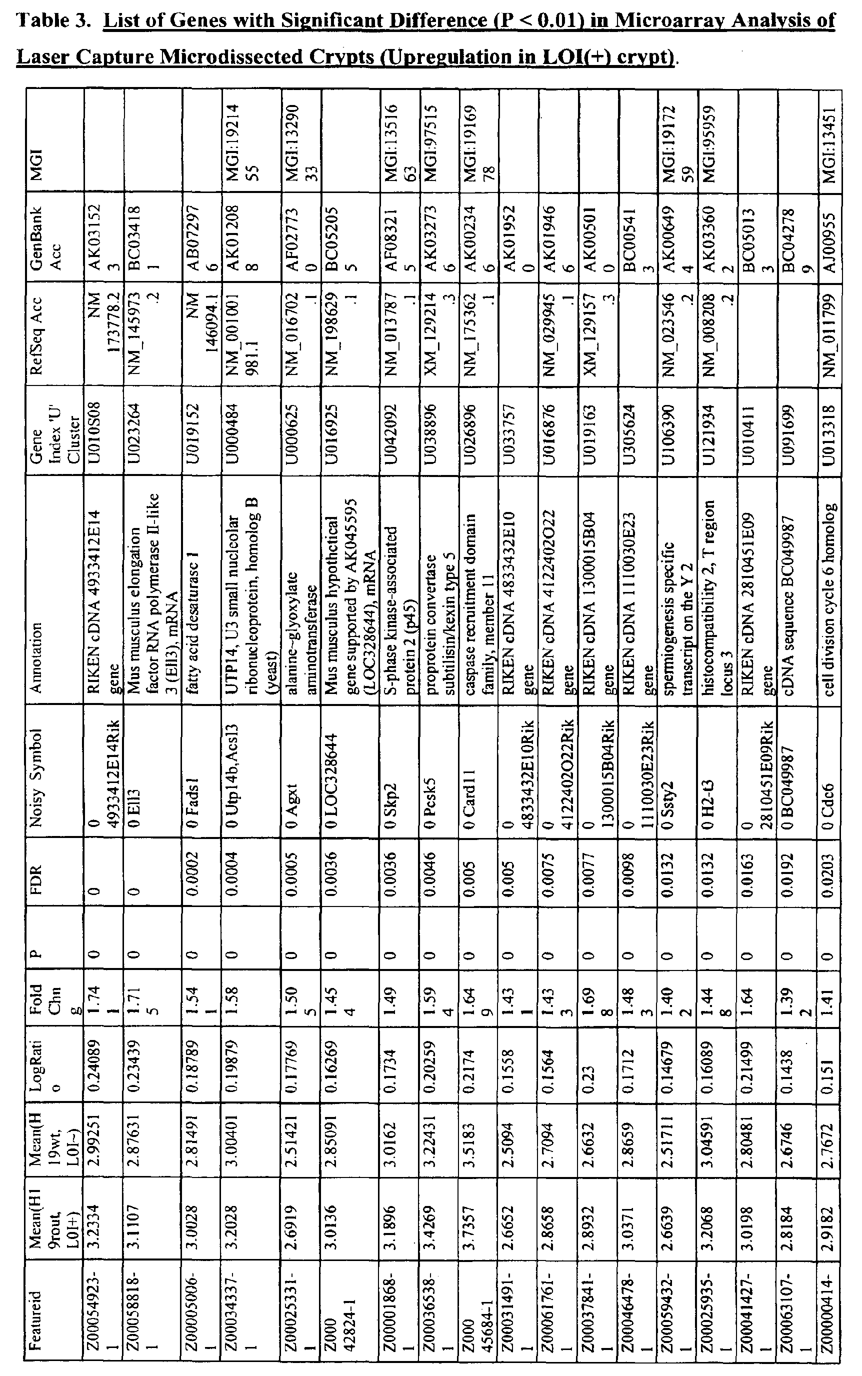

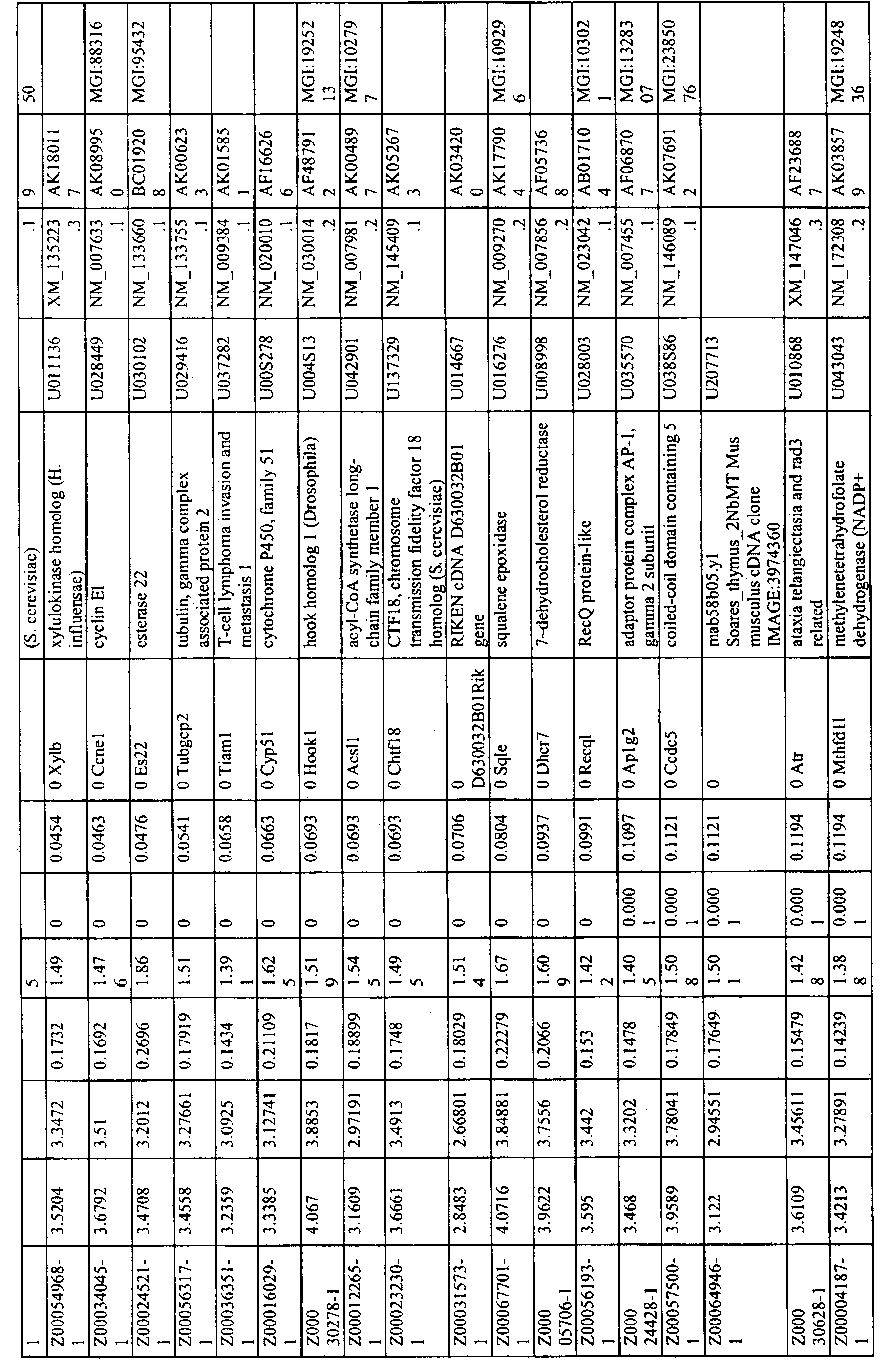

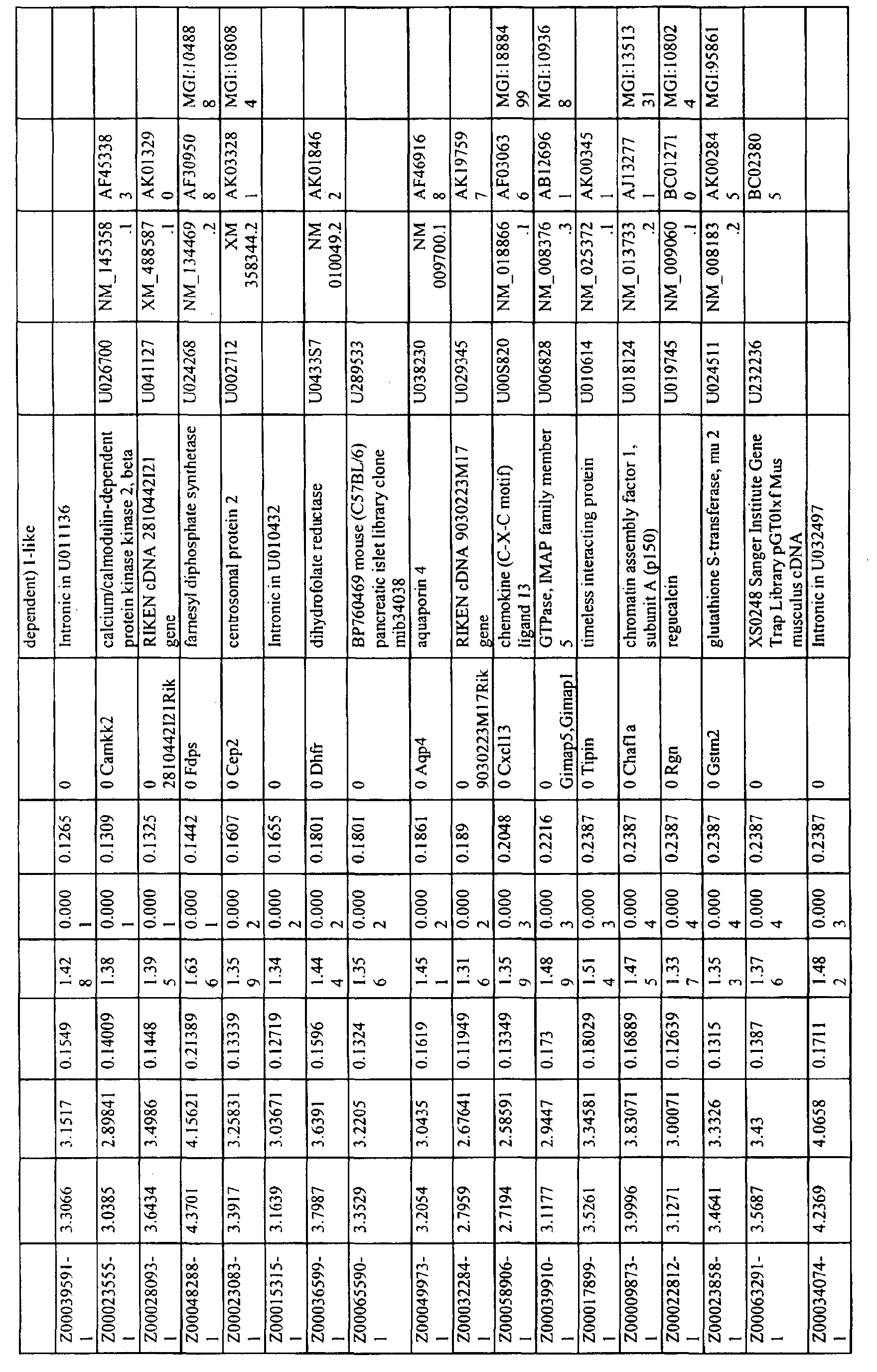

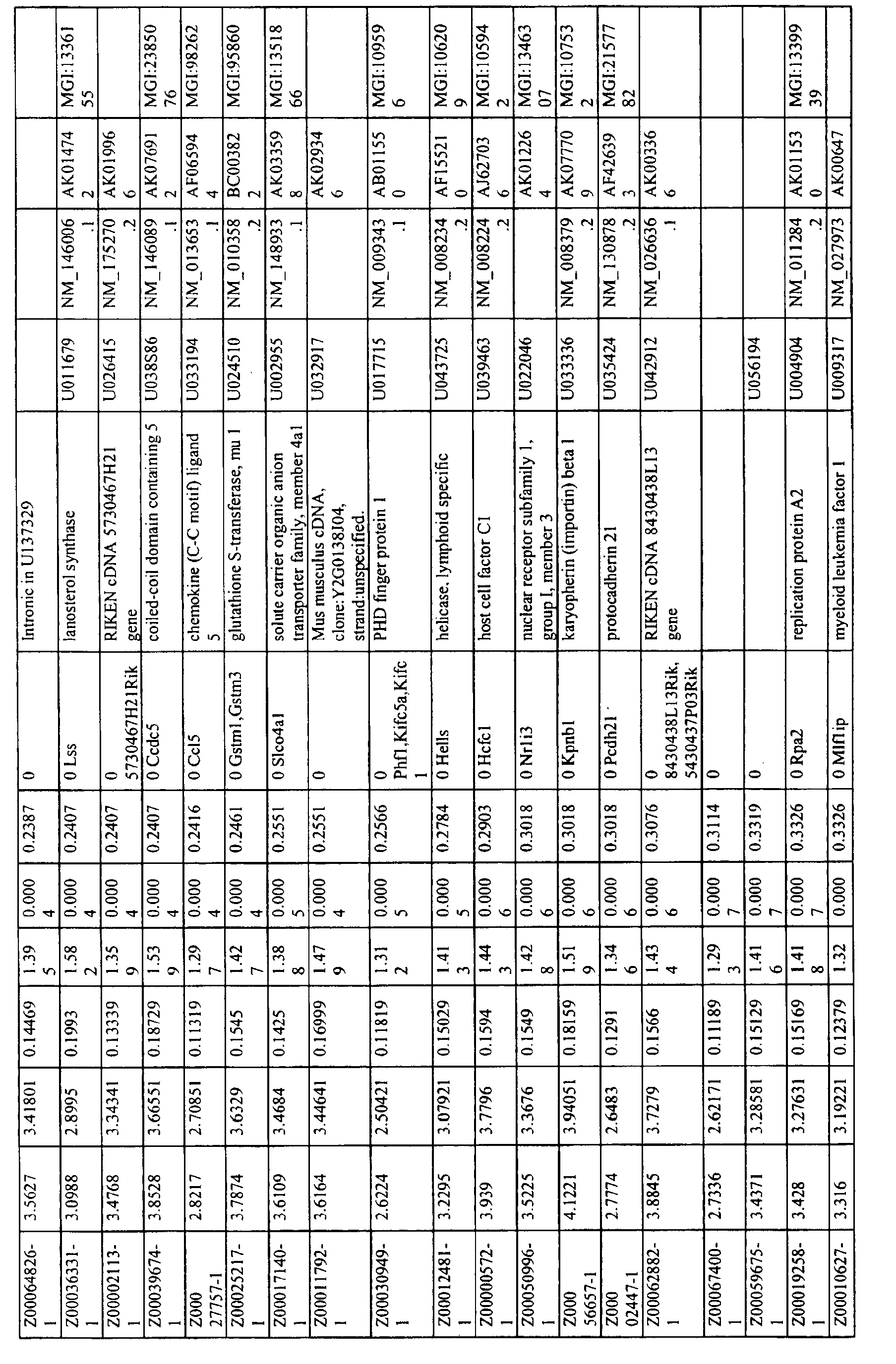

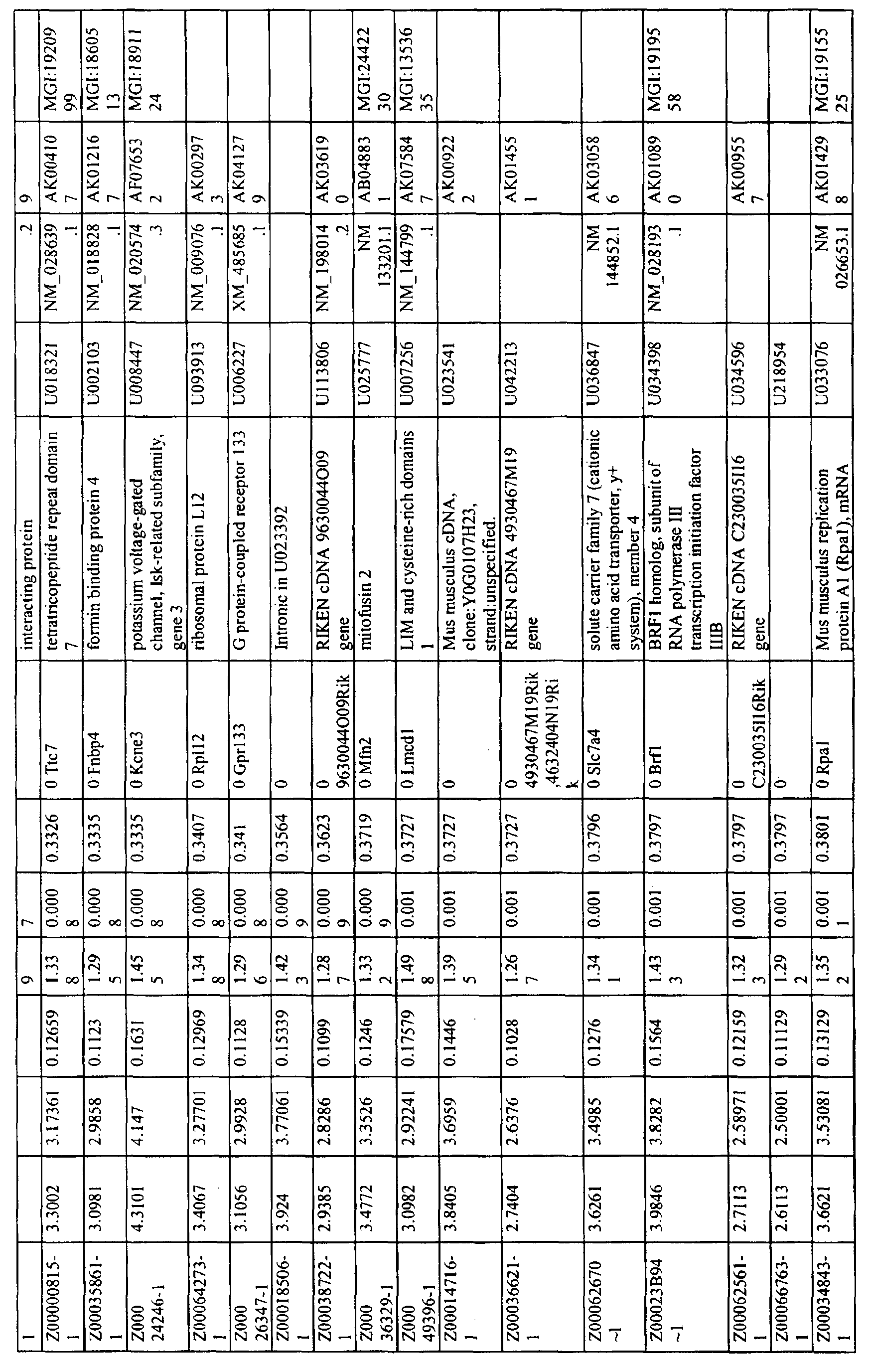

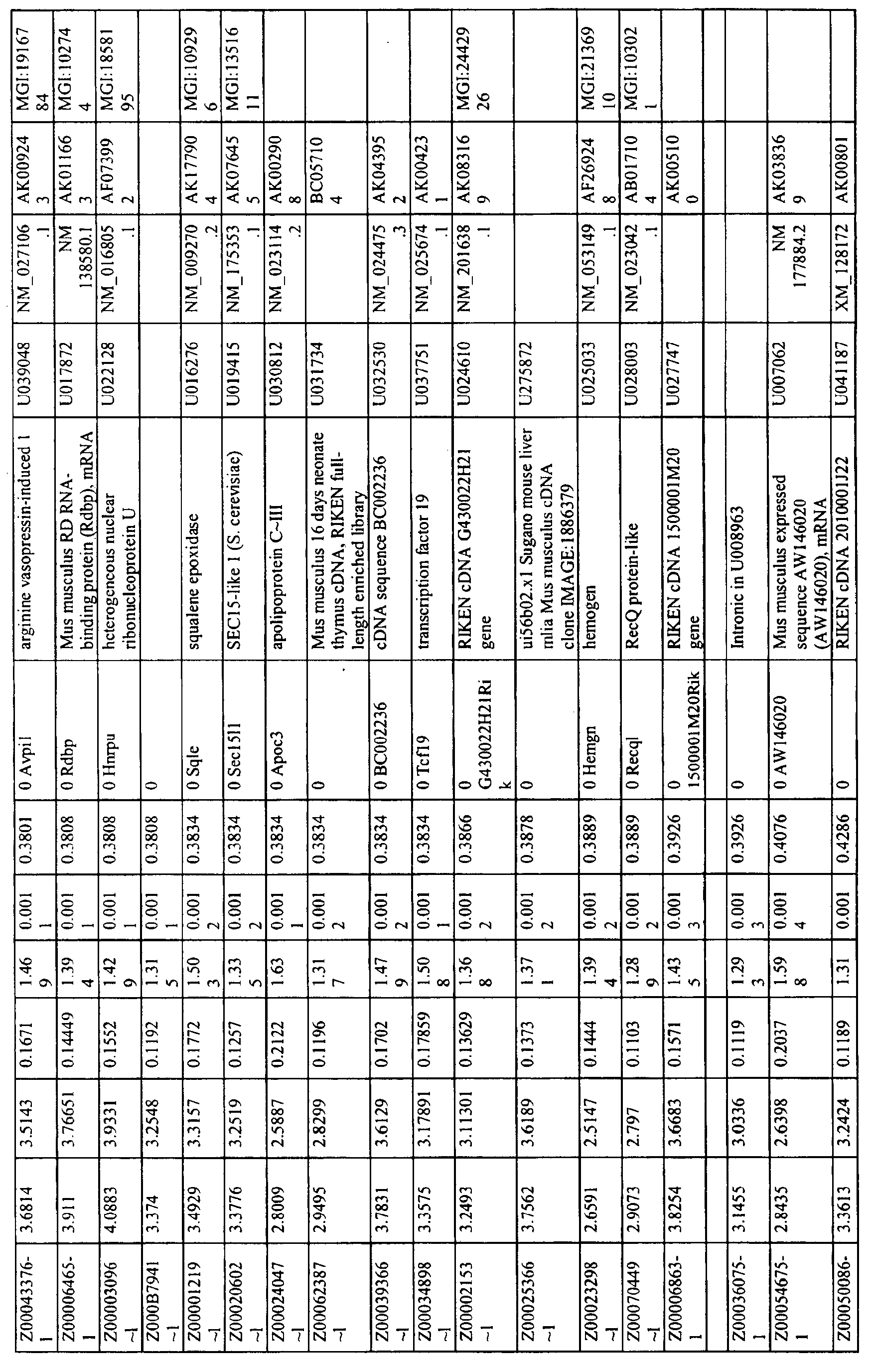

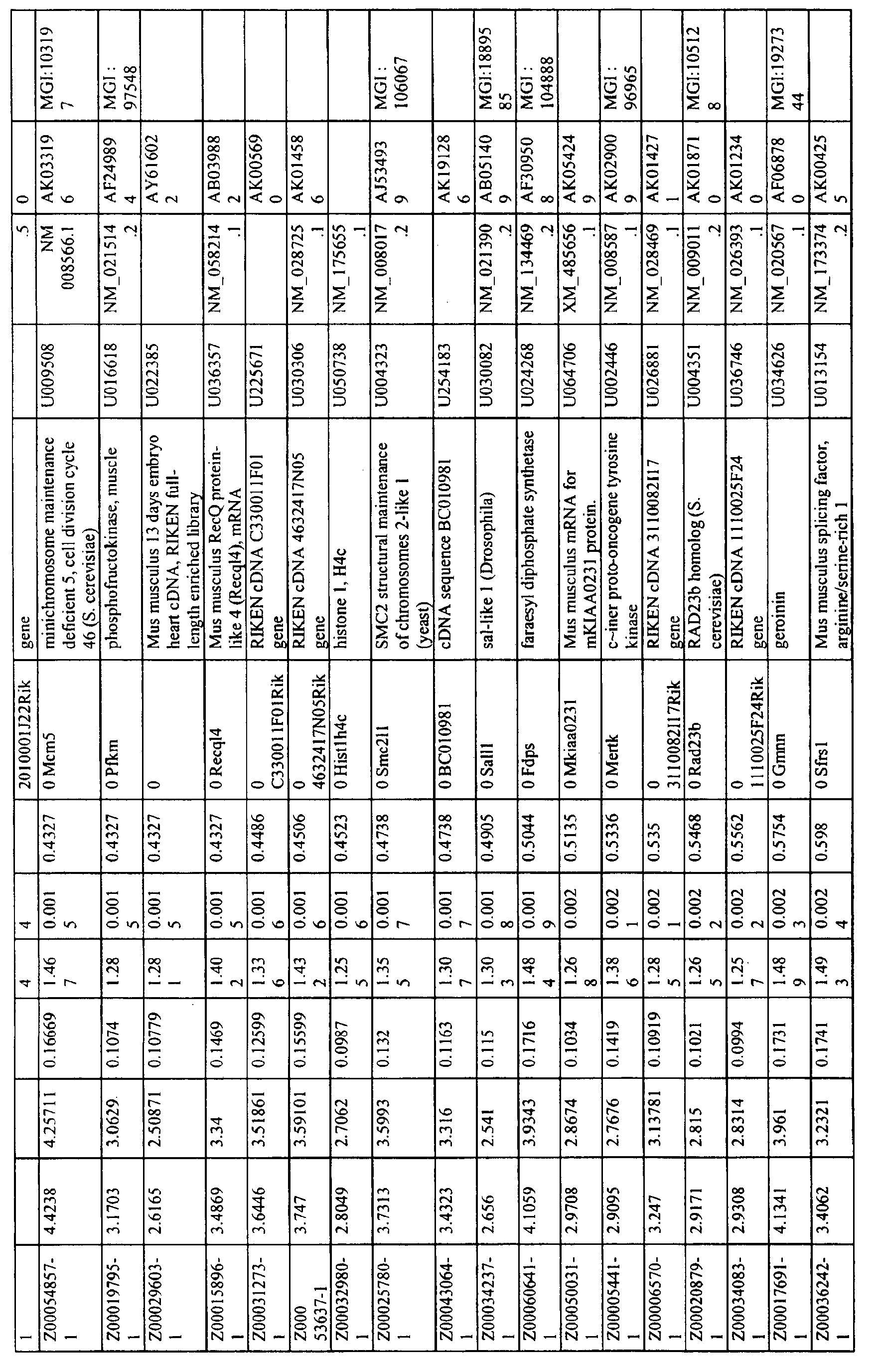

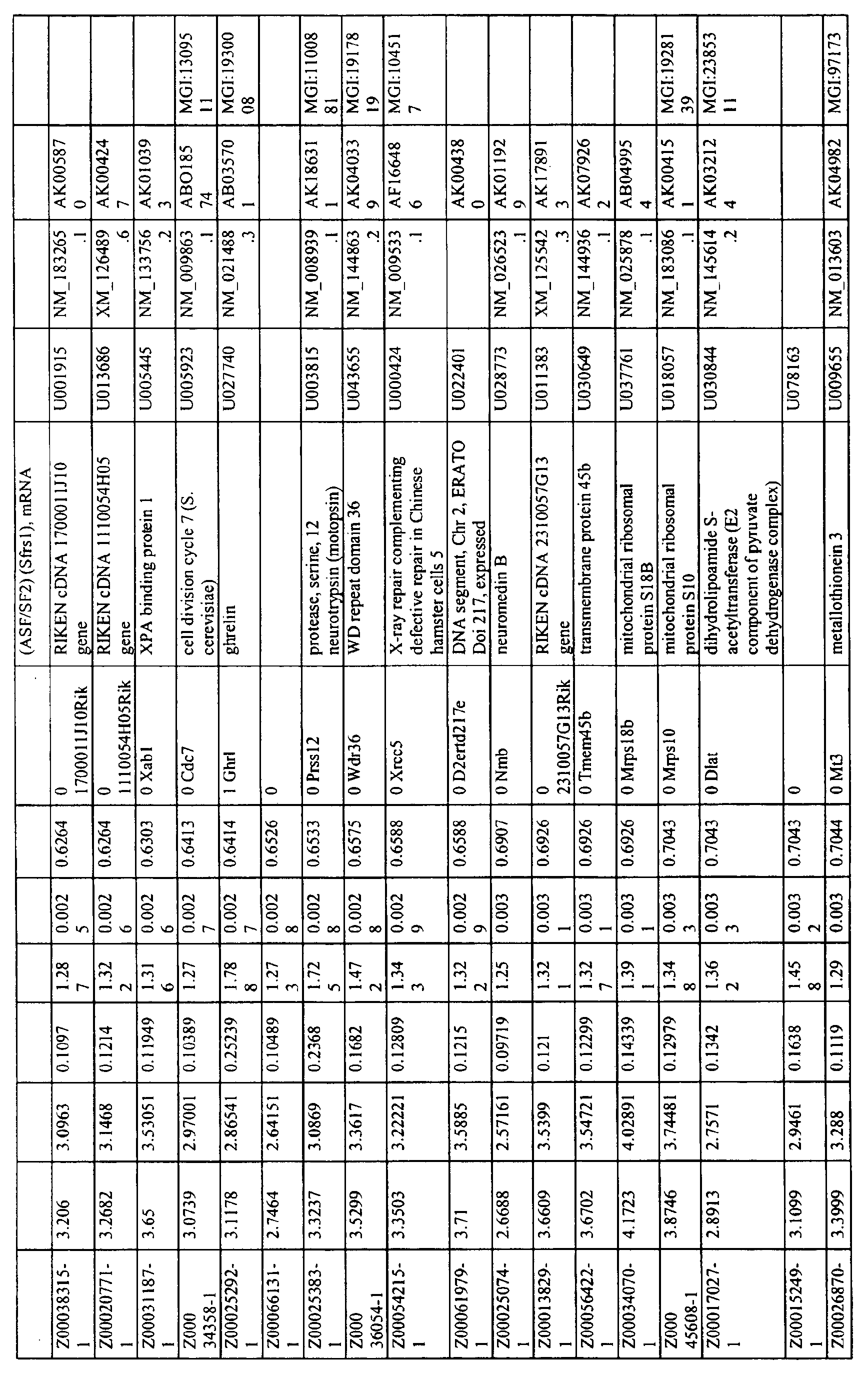

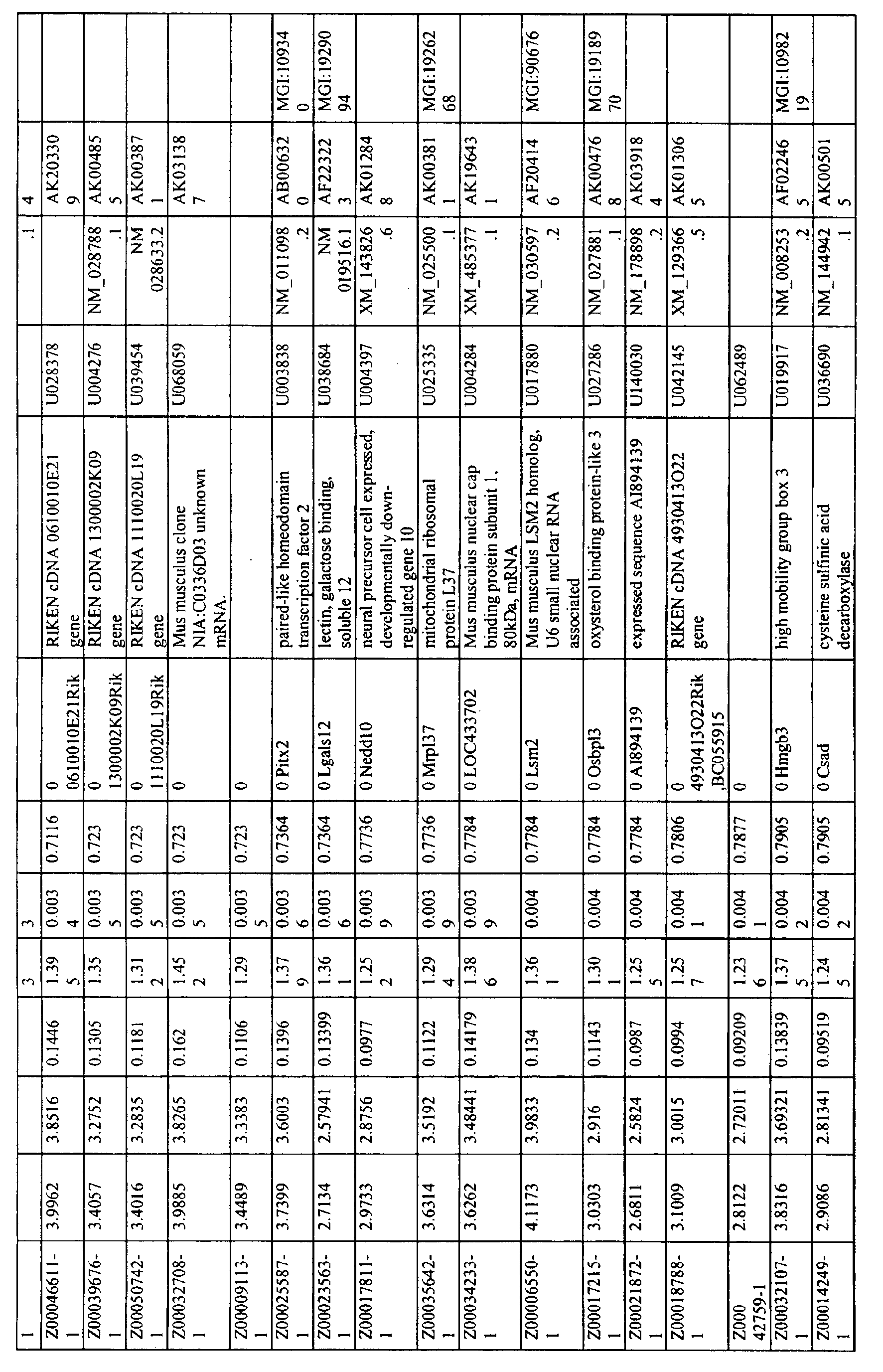

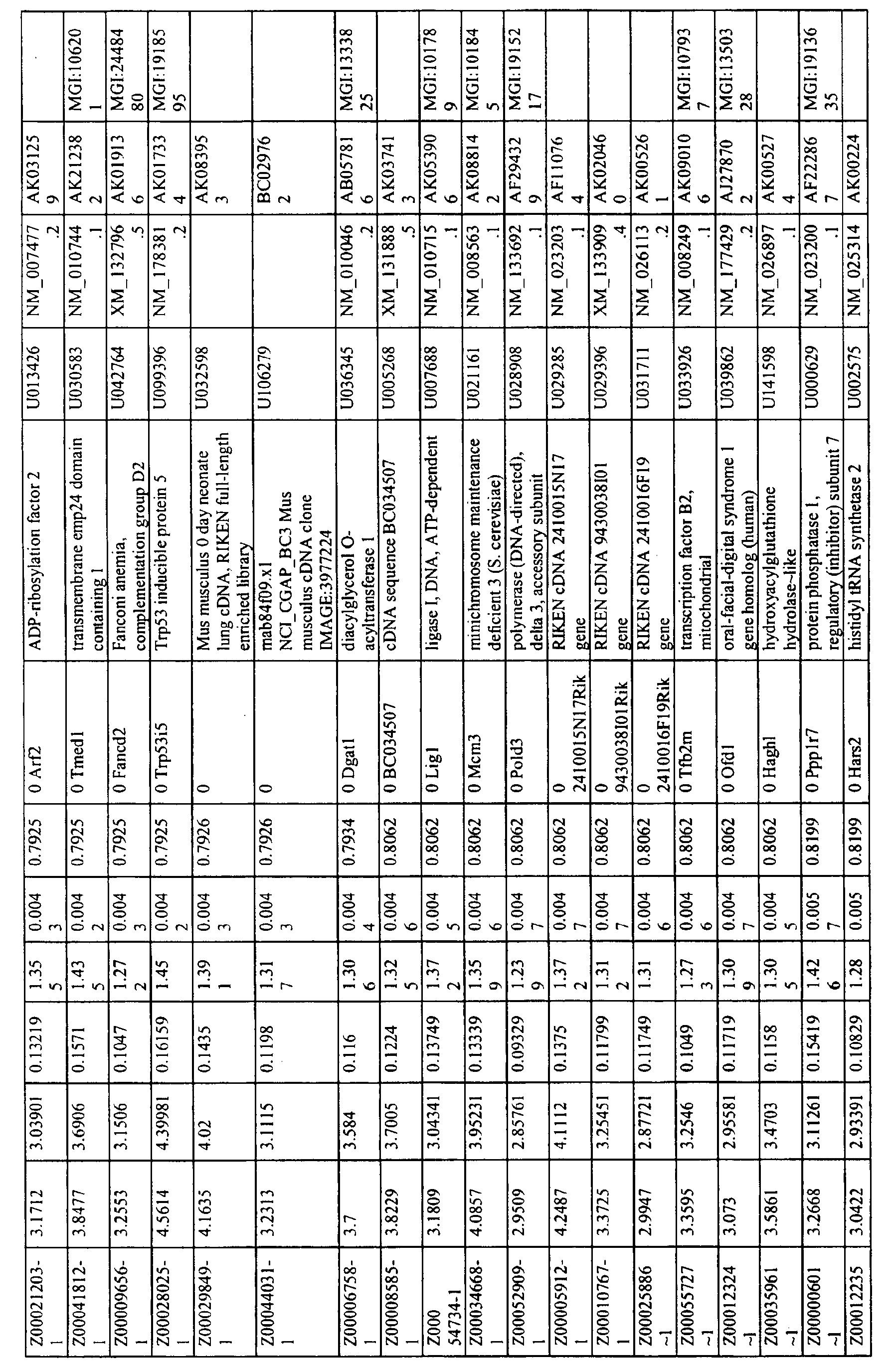

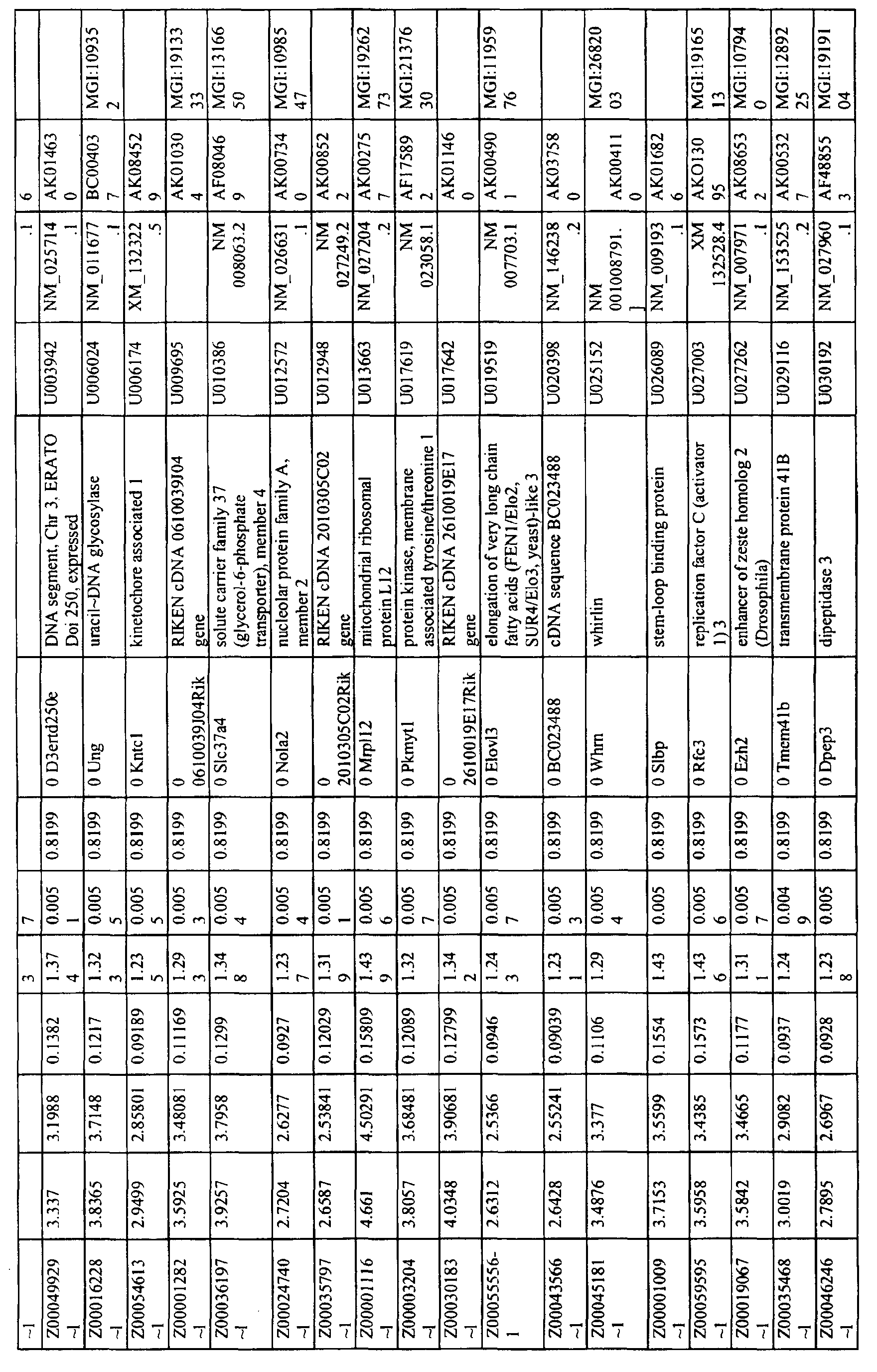

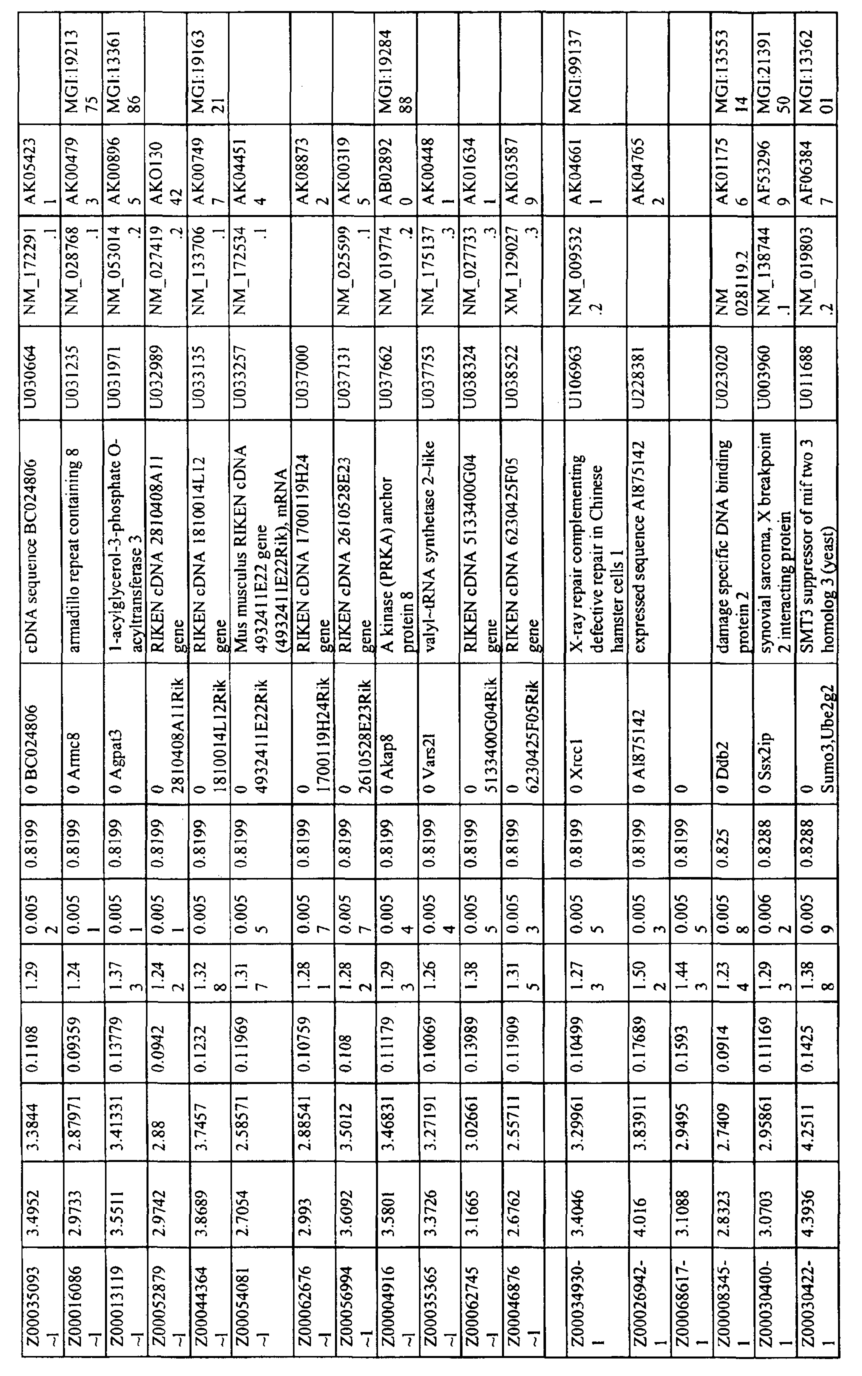

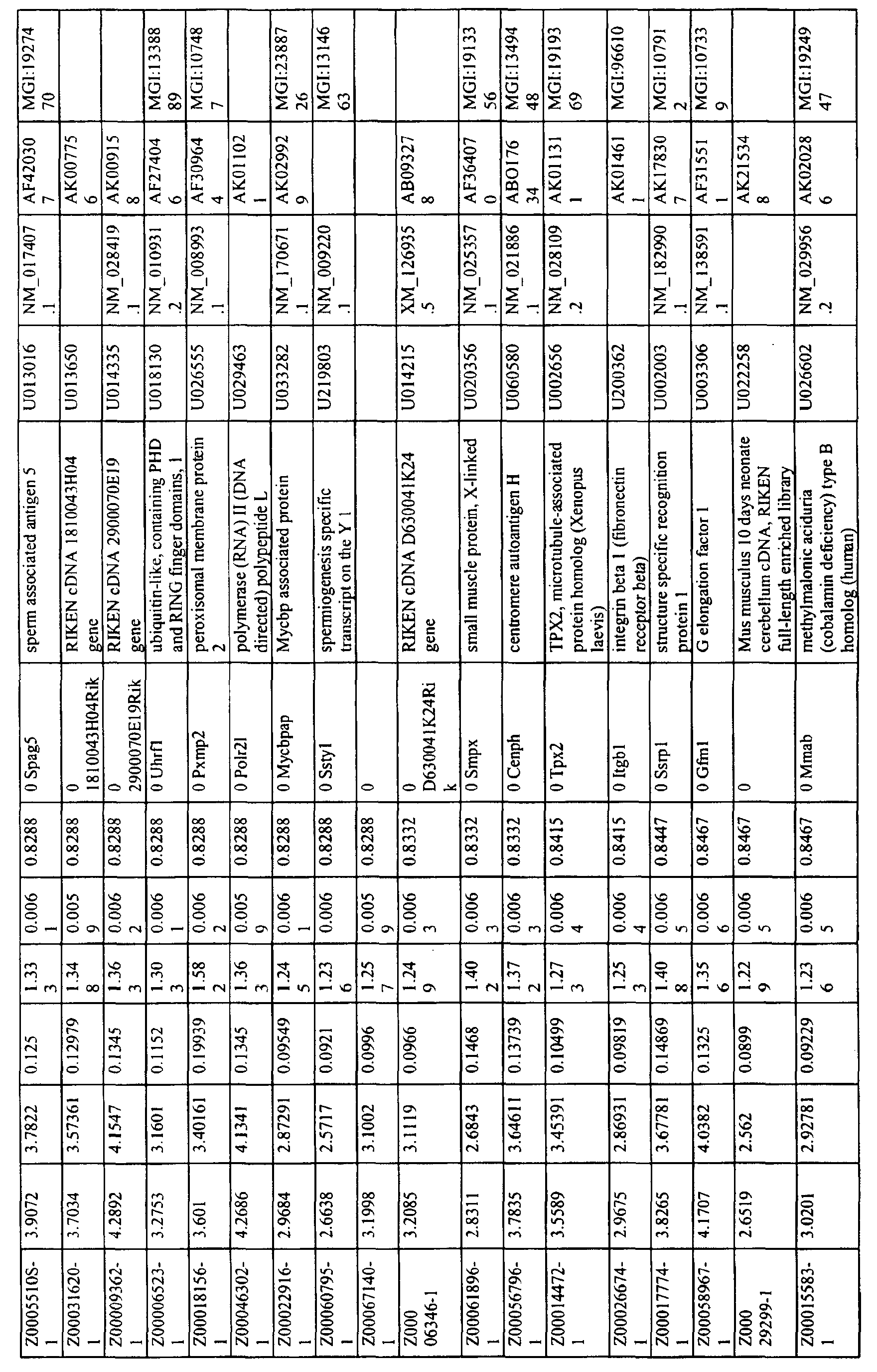

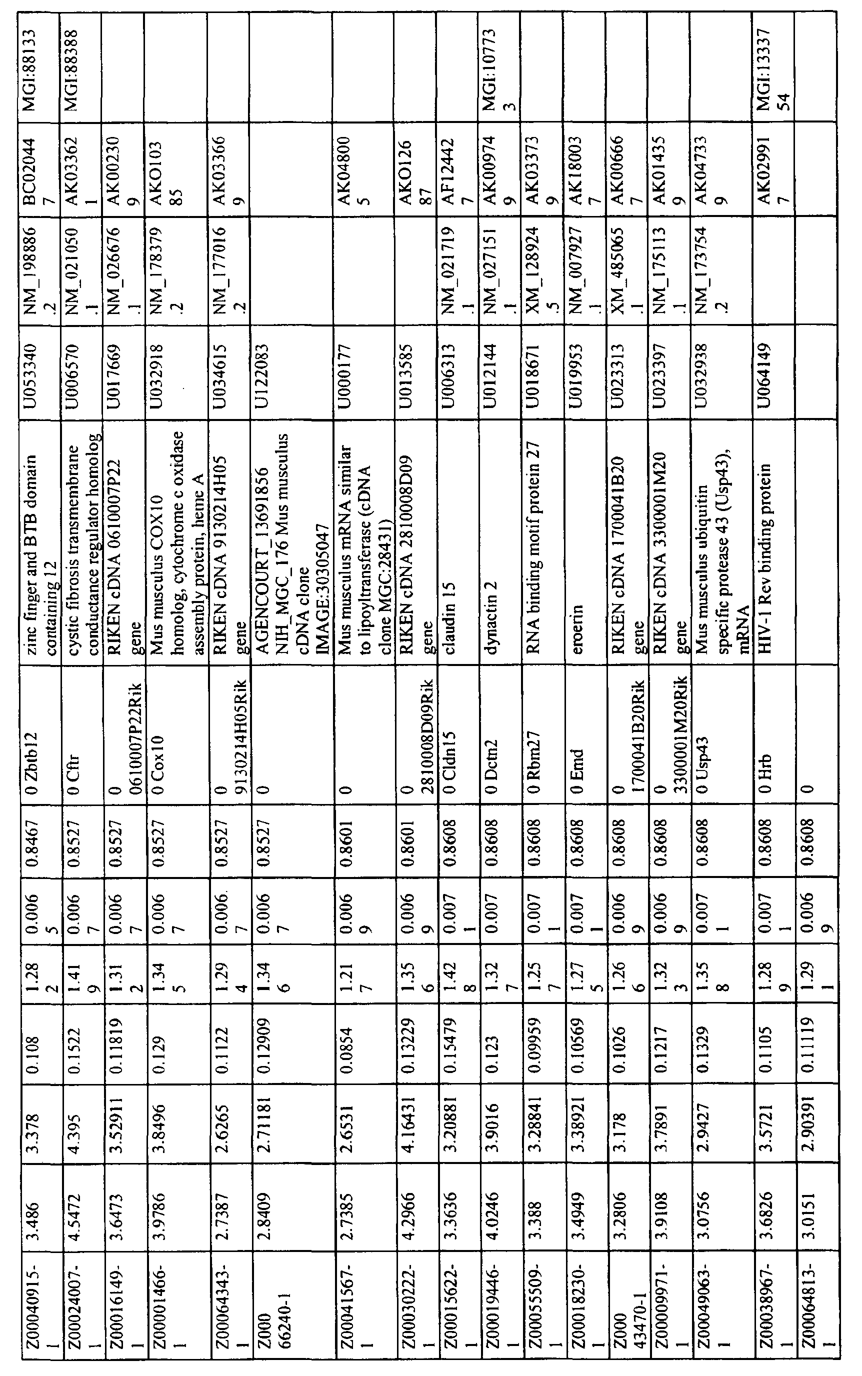

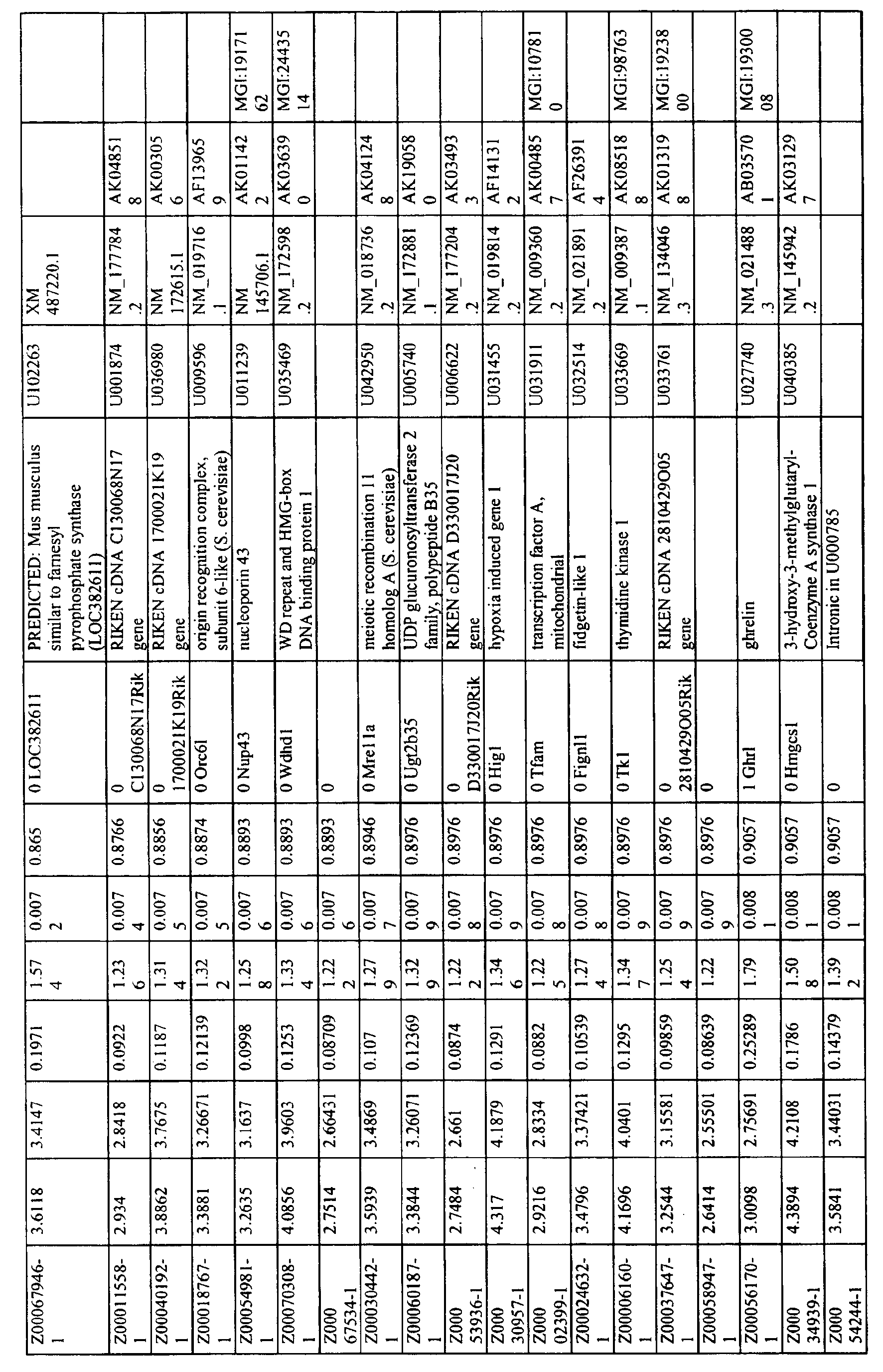

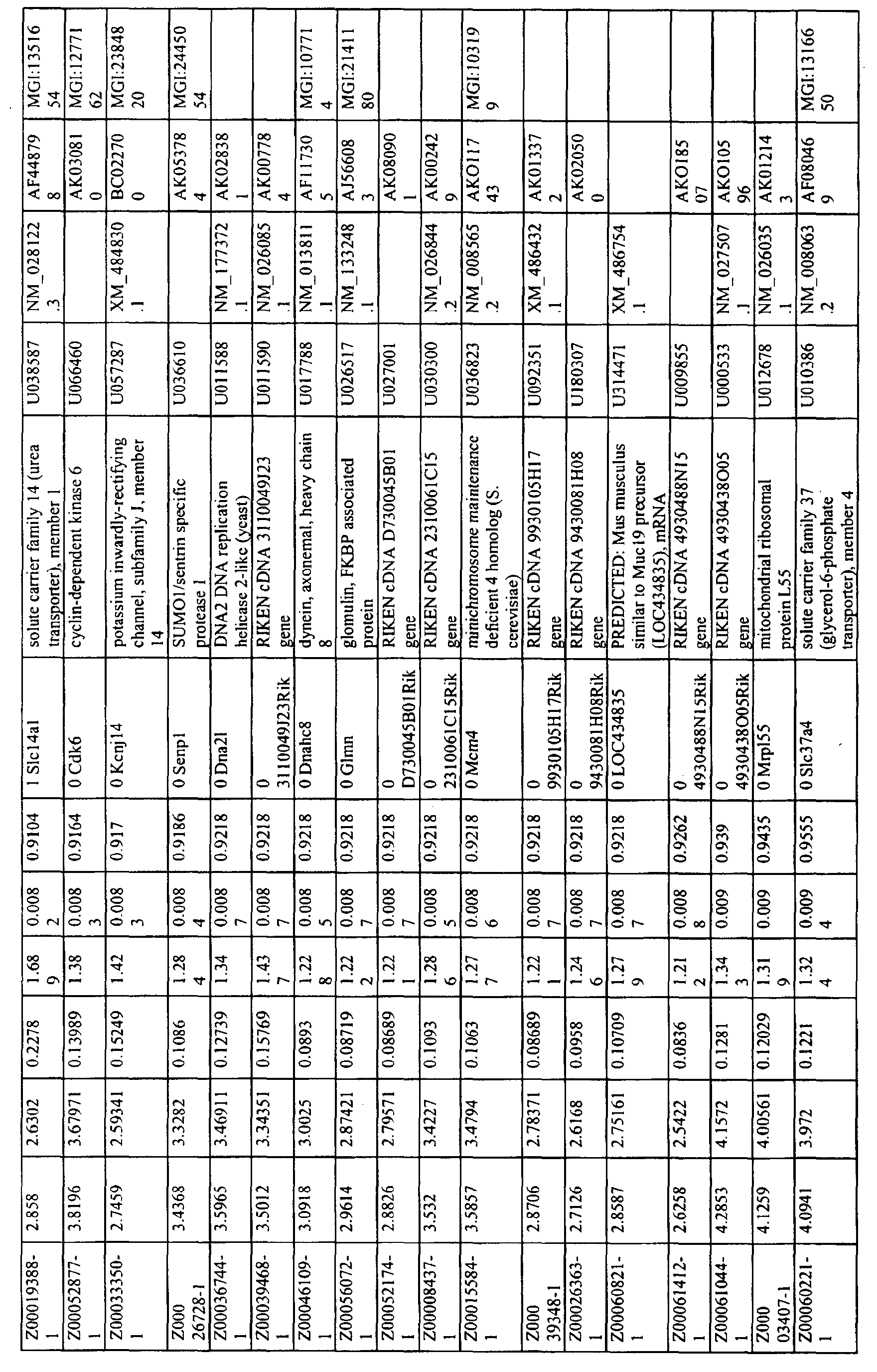

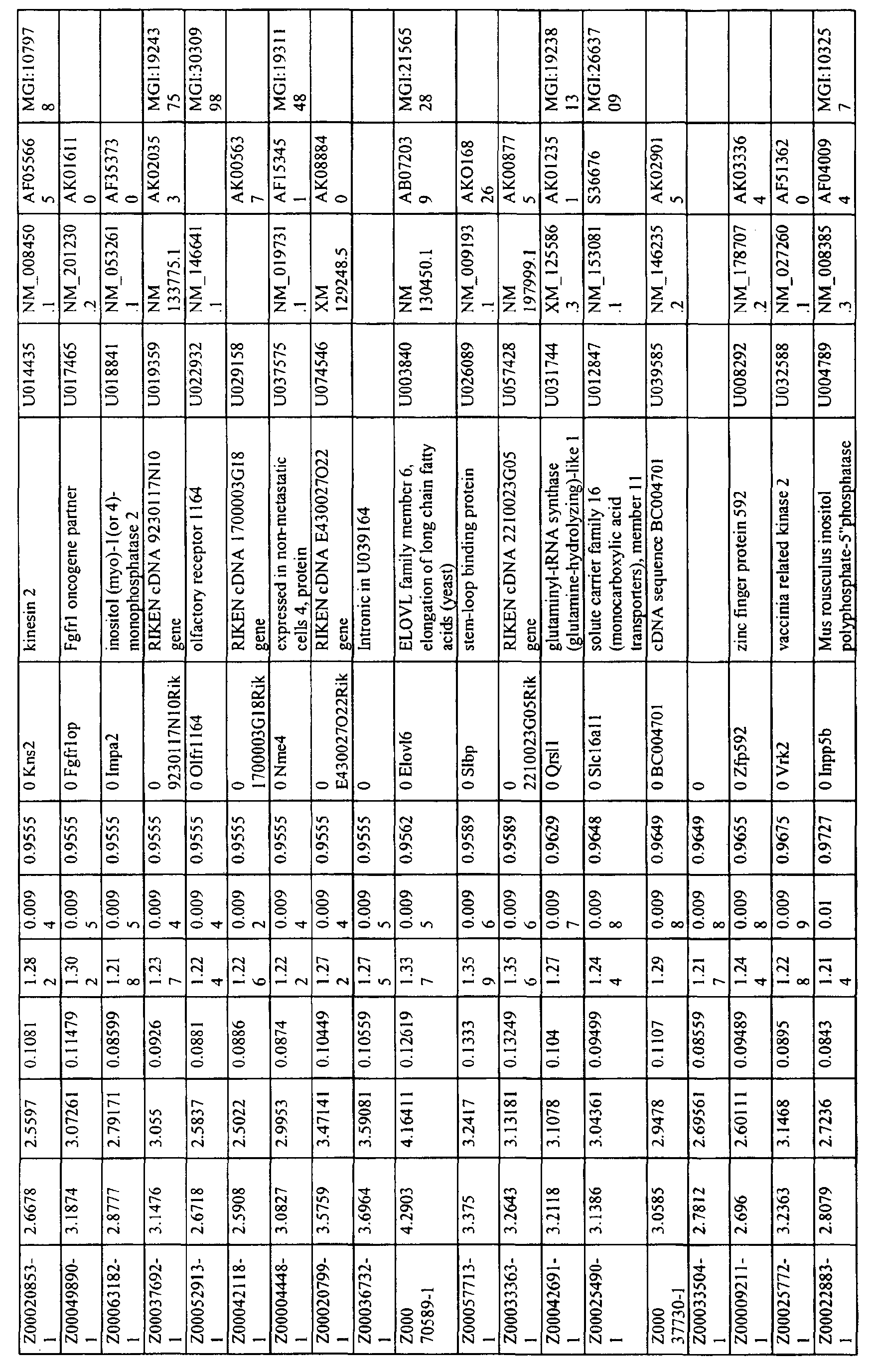

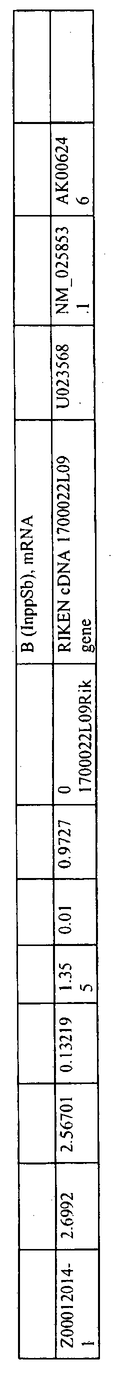

GO annotations showed a striking overrepresentation of genes showing increased expression involved with DNA replication (P < 10⁻¹⁵), cell cycle (P < 10⁻⁹), and cell proliferation (P < 10⁻⁹)(Tables 4, 5).

**Table 4. Gene Ontogeny (GO) annotation categories of genes with altered expression in microarray analyses of laser capture microdissected crypts.***

| Upregulated in LOI | | | | | |
|---|---|---|---|---|---|
| GO-annotation Category | Genes upregulated in category | Total GO-annotated upregulated genes | Total GO-annotated genes in category | Total GO-annotated genes | P value |
| DNA replication and chromosome cycle | 15 | 168 | 103 | 12933 | 10⁻¹⁵ |
| DNA replication | 13 | 168 | 83 | 12933 | 10⁻¹⁵ |
| DNA metabolism | 24 | 168 | 373 | 12933 | 10⁻¹⁵ |
| DNA-dependent DNA replication | 5 | 168 | 31 | 12933 | 10⁻¹² |
| Cell cyclc | 23 | 168 | 527 | 12933 | 10⁻⁹ |
| Cell proliferation | 27 | 168 | 692 | 12933 | 10⁻⁹ |
| Nuclear division | 8 | 168 | 115 | 12933 | 10⁻⁷ |
| M phase | 8 | 168 | 124 | 12933 | 10⁻⁶ |
| Mitotic cell cycle | 8 | 168 | 129 | 12933 | 10⁻⁶ |
| Mitosis | 6 | 168 | 82 | 12933 | 10⁻⁶ |
| M phase of mitotic cell cycle | 6 | 168 | 83 | 12933 | 10⁻⁶ |
| DNA repair | 7 | 168 | 122 | 12933 | 10⁻⁵ |
| ATP binding | 28 | 168 | 1012 | 12933 | 10⁻⁵ |
| Adenyl-nucleotide binding | 28 | 168 | 1028 | 12933 | 10⁻⁴ |
| Cytokines | 6 | 168 | 99 | 12933 | 10⁻⁴ |
| ATP-dependent helicase activity | 5 | 168 | 73 | 12933 | 10⁻⁴ |
| Purine nucleotide binding | 32 | 168 | 1293 | 12933 | 10⁻⁴ |
| Carboxylic acid metabolism | 11 | 168 | 281 | 12933 | 10⁻⁴ |
| Organic acid metabolism | 11 | 168 | 281 | 12933 | 10⁻⁴ |
| Nucleotide binding | 32 | 168 | 1313 | 12933 | 10⁻⁴ |
| Response to DNA damage stimulus | 7 | 168 | 142 | 12933 | 10⁻⁴ |
| ATPase activity | 9 | 168 | 213 | 12933 | 10⁻⁴ |
| Metabolism | 90 | 168 | 5100 | 12933 | 10⁻⁴ |
| Response to endogenous stimulus | 7 | 168 | 147 | 12933 | 10⁻⁴ |

| | | | | | |
|---|---|---|---|---|---|
| *GO annotation was analyzed at http://lgsun.grc.nia.nih.gov/geneindex4/upload.html. Shown are categories with >5 genes identified with P < 10⁻⁴. Downregulated genes were not enriched in any category. | | | | | |

**Table 5. Genes involved in the top ranking categories (DNA replication/cell cycle), upregulated in LOI(+) mice in microarray analysis of laser-capture microdissected crypts.+**

| Gene | Fold change | Function |
|---|---|---|
| *Card11* | 1.65 | Phosphorylates BCL10, inducing NF-kB activity |
| *Ccdc5* | 1.54 | Regulator of spindle function |
| *Skp2* | 1.49 | Oncogene required for S-phase entry |
| *Gmnn* | 1.49 | Accumulated in S, G2 and M, inhibiting inappropriate origin firing by CDT1 |
| *Ccne1* | 1.48 | Required for CDK2 activation, leading to proliferation |
| *Chafla* | 1.48 | Assembles histone octamer onto replicating DNA during S phase |
| *Mcm5* | 1.47 | Required for DNA replication, interacting with Cdc6 and Mcm2 |
| *Rfc3* | 1.44 | Involved in efficient elongation of DNA |
| *Rpa2* | 1.42 | 32-kD subunit of replication protein A |
| *Cdc6* | 1.42 | Essential licensing factor for DNA replication |
| *Mertk* | 1.39 | Proto-oncogene expressed in epithelial and reproductive tissues |
| *Pitx2* | 1.38 | Transcription factor required for effective cell type-specific proliferation |
| *Cenph* | 1.37 | Mitotic centromere-associated kinesin |
| *Ligl* | 1.37 | DNA ligase involved in joining Okazaki fragments |
| *Mcm3* | 1.36 | Required for DNA unwinding during DNA replication |
| *Smc211* | 1.36 | Required for mitotic chromosome condensation |
| *Rpa1* | 1.35 | Subunit of replication protein A required for DNA replication |
| *Spag5* | 1.33 | Orthologue of astrin, localized to spindle and required for mitosis |
| *Orc61* | 1.32 | Binds origins of DNA replication for initiation of DNA replication |
| *Uhrf1* | 1.30 | Required for S-phase entry, regulates Top2a expression. |
| *Mre11a* | 1.28 | Required for double-strand break repair and cell proliferation |
| *Mcm4* | 1.28 | Required for DNA replication, interacts with Mcm6 and Mcm7 |
| *Cdc7* | 1.27 | Required for G1-S transition and initiation of DNA replication |
| *Itgb1* | 1.25 | Progenitor cell marker for proliferative zone of colon crypts |
| *Pold3* | 1.24 | Subunit of DNA polymerase delta required for DNA replication. |
| *Kntc1* | 1.24 | Mitotic check point |
| *Glmn* | 1.22 | Immunophilin, natural ligand of FKBP59 and FKBP12 |

| | | |
|---|---|---|
| + Shown are genes with ≥ 1.20-fold change. | | |

Significant enrichment in other categories was not seen. In order to confirm these results by real-time quantitative PCR, LCM was performed on 15,000 additional crypts microdissected from an additional 12 LOI(+) and 9 LOI(-) mice, yielding > 300 ng of RNA from each sample. While the changes in gene expression were moderate (~1.5 fold)(Tables 4 and 5, *supra*), real-time quantitative PCR analysis of 14 genes confirmed statistically significant differences in 13 (P between 0.003 and 0.04) and the other was suggestive (P = 0.1)(Figure 1).

The top ranking genes in this analysis included Cdc6, an essential licensing factor leading to initiation of DNA replication and onset of S-phase (Dutta et al., Annu Rev Cell Dev Biol (1997) 13:293-332; Coleman et al., Cell (1996) 87:53-63), *Mcm5* and *Mcm3,* both required for DNA replication at early S-phase (Chong et al., Nature (1995) 375:418-421; Madline et al., Nature (1995) 375:421-424), *Skp2,* necessary for S-phase entry (Reed, Nat Rev Mol Cell BGiol (2003) 4:855-864; Bashir et al., Nature (2004) 428:190-193), *Ccdc5,* a regulator of spindle function (Einarson et al., Mol Cell Biol (2004) 24:3957-3971), *Chaf1a,* which assembles the histone octamer onto replicating DNA (Smith and Stillman, Cell (1989) 58:15-25), and *Rpa2,* a single strand DNA binding protein essential for DNA replication (Mass et al., Mol Cell Biol (1989) 18:6399-6407; Shao et al., Embo J (1999) 18:13971406) (Figure 1, Tables 4, 5, *supra*). These results imply that LOI causes a specific alteration in replication-associated gene expression in intestinal epithelium. Nevertheless, increased expression of some genes not associated with DNA replication per se was observed. For example, *Card11* (Figure 1) is an anti-apoptotic gene acting through phosphorylation of BCL10 and induction of NF-κB (Bunnell, Mol Interv (2002) 2:356-360). Expression of *Msi1* was also analyzed by real-time PCR, as the encoded progenitor cell marker *Musashi*-1 showed increased immunostaining in a previous study (Sakatani et al. (2005)). Expression of *Msi1* was also significantly increased (P = 0.01)(Figure 1), confirming the earlier result and supporting a pleiotropic mechanism for IGF2 in LOI. Finally, several genes showed down regulation in LOI(+) crypts, including *p21* (Figure 1), an inhibitor of cell cycle progression.

### Example 1. In vitro confirmation of the proliferative effect of IGF2 LOI(+) on progenitor cells.

It remained theoretically possible that the observed difference in gene expression simply reflected the over-representation of progenitor cells within the intestinal crypts, rather than a change in cellular gene expression per se. To confirm the latter, mouse embryonic stem (ES) cells were derived and plated in ESGRO Complete Clonal Grade defined medium (Chemicon), which contains no IGF2 and allows growth of undifferentiated ES cells without a feeder layer, which was done with or without 800 ng/ml of mouse Igf2 recombinant protein (StemCell Technologies). Consistent with the microarray experiments, Igf2 induced a 50% and 56% increase in *Cdc6* gene expression at 3 and 6 hours, respectively, and 40% and 25% at 10 and 24 hours, respectively (Figure 3). Similar results were observed for *Mcm5* (Figure 3). To confirm the specificity of this effect, these experiments were repeated by blocking IGF2 signaling with NVP-AEW541, a pyrrolo[2,3-d]pyrimidine derivative that specifically inhibits IGF1R over the related insulin receptor, and that the drug blocks IGF2 at IGF1R was also confirmed (Figure 9). NVP-AEW541 completely abrogated the IGF2-induced increased expression of *Cdc6* at all time points (Figure 3). These experiments were repeated for *Mcm5* and *Msil,* with similar results (Figure 3, Figure 10), confirming that Igf2 induced proliferation-related gene expression, as well as increased proliferation of progenitor cells in vivo.

The idea that LOI(+) progenitor cells proliferate more quickly than LOI(-) cells was then tested by deriving 4 ES lines each from both LOI(+) and LOI(-) embryos. LOI(+) ES cells showed an apparently larger colony size by light microscopy than did LOI(-)(Figure 4). In order to quantify colony size, 1,000 ES cells were seeded each from four LOI(+) and four LOI(-) lines on feeder cells and measured the size of 15-30 colonies from each, daily through day 6. LOI (+) ES cells showed a statistically significant increase in colony size over LOI(-) ES cells as early as day 3 (P = 0.001), which increased to an 86% increase by day 6 (P = 0.0009)(Figure 5).

ES cell growth was then measured directly as undifferentiated cells in ESGRO Complete Clonal Grade defined medium (Chemicon), again using four LOI(+) and four LOI(-) ES lines, with triplicate wells at days 0 through 4. LOI(+) ES cells showed a 26% increased growth rate (P = 0.01), with a 160% increase in cell number over LOI(-) ES cells by day 4 (P = 0.0003). Therefore, LOI(+) ES cells proliferate significantly more rapidly than LOI(-) ES cells, consistent with the expression data suggesting that intestinal progenitor cells with LOI also show greater proliferation (Figure 6).

### Example 2. Specific Inhibition of Aberrant Crypt Foci in LOI(+) Mice by an inhibitor of Igf1R.

Because of known strain variation in progression of these lesions, littermate controls were treated with and without LOI, in which the dams were heterozygous for a deletion of the H19 differentially methylated region (DMR); inheritance of a maternal allele lacking the DMR leads to activation of the normally silent allele of Igf2 [LOI(+)], while inheritance of a wild type maternal allele leads to normal imprinting [LOI(-)]. Eight LOI(+)and 14 LOI(-) mice were given AOM intraperitoneally weekly for 3 weeks, sacrificed at 5 weeks after the first dose, and ACF were scored by the method of Bird (1987). Histologic examination of colons from AOM-treated mice confirmed the presence of ACF, with hyperproliferative features including increased mitosis, crypt enlargement and crypt disarray (Figure 12). These results are consistent with the proliferation-specific changes in gene expression described above. An additional intriguing finding in AOM-treated LOI(+) mice was cystically dilated crypts lined by enlarged cells with atypical nuclei and that contained necrotic debris, that were reminiscent of sessile serrated adenomas (SSAs) seen in the human colon (Figure 12). SSAs also show crypt dilatation in association with cytologic atypia and are currently of immense interest for their recently recognized association with colorectal cancer (Snover et al., Am J Clin Pathol (2005) 124:380-391). It will thus be of interest to determine whether SSAs are associated with LOI in the human population.

LOI(+) mice showed 19.8 ± 2.2 ACF per colon, compared to 12.4 ± 0.9 ACF per colon in LOI(-) mice, a 60% increase (P = 0.002). An additional 9 LOI(+) mice and 9 LOI(-) control littermates were similarly exposed to AOM, adding treatment with NVP-AEW541, an IGF1R inhibitor, at a dose of 50 mg/kg by oral gavage daily for 6 weeks (twice daily except daily on weekends). LOI(-) mice showed no difference in ACF formation after NVP-AEW541 drug treatment (11.3 ± 1.6, N.S.). However, LOI(+) mice showed a striking reduction in AOM-induced ACF formation after NVP-AEW541 treatment (7.8 ± 1.2, P = 0. 0002), significantly lower even than that seen in LOI(-) AOM-treated mice (P=0.007). Thus, LOI of lgf2 increases the sensitivity to AOM through an IGF1R-dependent mechanism. Furthermore, LOI(+) mice are more sensitive to the effects of IGF1R blockade than are LOI(-) mice, suggesting an increased sensitivity to IGF2 signaling in LOI(+) mice (Figure 7).

These results taken together suggest a possible chemoprevention strategy in which patients with LOI are treated with a drug designed to inhibit IGF2 signaling, thereby reducing the increased proliferation of progenitor cells. As a proof of principle experiment, a new animal model of LOI was developed using the chemical carcinogen azoxymethane (AOM), which, unlike the Min model, is colon-specific and the exposure can be timed postnatally. The C57BI/6 strain in which the LOI model was established develops aberrant crypt foci (ACF), which are mucosal lesions with varying degrees of crypt multiplicity, elevation, and enlargement. This strain is nontumorigenic but ideally suited for study of the earliest stages of tumor development, which is the presumed target for LOI as well as our chemopreventative strategy. ACFs have been used as a model for rodent intestinal tumors for two decades (Schoonjans et al., Proc Natl Acad Sci USA (2005) 102:2058-2062; Osawa et al., Gastroenterology (2003) 124:361-367). Example 3. LOI causes Long Term Potentiation of Akt Signaling in Response to Igf2.

To determine whether cells from LOI(+) mice themselves differed in IGF2 sensitivity, a novel high throughput signal transduction assay was developed based on an immunostaining automation device comprising microfluidic chambers housing multiple cells (Wang et al., in 10th International Conference on Miniaturized Systems for Chemistry and Life Sciences (MicroTAS2006) (Tokyo, Japan, 2006). An advantage of the microfluidic chip is that all the cells can be cultured simultaneously on the same chip and under identical conditions, with exquisite control of the cell medium over the time of the experiment and subsequent analysis, allowing a much larger number of measurements than would be possible by conventional means. The device was constructed within a monolithic 2-layer PDMS chip sealed with a glass coverslip, with defined media delivery controlled by a multiplexed system of valves. Akt/PKB, a known and well characterized target of IGF2 activation, was examined and mouse embryo fibroblast (MEF) lines from LOI(+) and LOI(-) embryos were derived for this purpose. MEF lines were chosen over ES cells in this analysis to facilitate individual cell examination in microfluidic chambers rather than in densely packed colonies. Live LOI(+) and LOI(-) cells were stimulated within the chips with varying doses of IGF2, with Akt/PKB measurements at multiple time points and IGF2 concentration. For each cell type, IGF2 concentration, and time point, at least 200 individual cellular measurements were obtained by digital imaging and analysis, providing ample information for statistically significant evaluation of both the average response and cell-cell variability. The results were consistent in chip-to-chip variation analysis, with two chips used for each cell line. As a read-out immunostaining of the nuclear phosphorylated Akt (Ser 473, Upstate) was used. Previous studies have revealed importance of Akt for cell cycle regulation, with sustained activity implicated in growth factor-mediated transition through G1 (Jones et al., Curr Biol (1999) 9:512-521).

IGF2 triggered a transient Akt activation signal (peak at 10 to 40 minutes followed by a return to the baseline within 90 min.) in LOI(-) cells (Figure 8A) at all concentrations tested (400, 800, and 1600 ng/ml), comparable to levels used to support mouse fetal liver hematopoietic stem cells (500-1000 ng/ml) (Zhang and Lodish, Blood (2004) 103:2513-2521). In contrast, when subjected to the lowest (400 ng/ml) Igf2 concentration, LOI(+) cells showed markedly sustained Akt activation (> 120 minutes), which increased steadily over time after stimulation (Figure 8A). At higher IGF2 doses, the Akt signal in LOI cells became progressively more transient and less pronounced. These results demonstrate that LOI(+) cells have enhanced sensitivity to IGF2 at lower doses, and could help explain the increased sensitivity of LOI(+) mice to IGF1R inhibition.

### Example 4. LOI-Related Increase in Proliferation-Related Gene Expression is Differentially Sensitive to IGF1R Inhibition.

Earlier it had been shown that LOI leads to an increase in the progenitor cell compartment in crypt cells of Min mice (Sakatani et al. (2005)), but the mechanism was unknown. Therefore it was sought to determine what changes in gene expression occur in gastrointestinal epithelial progenitor cells in LOI mice. Gene expression was measured in laser capture microdissected crypts, comparing 8000 crypts from each of 3 LOI(+) and 3 LOI(-) mice on microarrays. 283 genes showed increased expression and 109 genes showed decreased expression (Table 3, *supra*)*.* GO annotation showed a striking overrepresentation of genes showing increased expression involved with DNA replication (P < 10⁻¹⁵), DNA metabolism (P < 10⁻¹⁵), cell cycle (P < 10⁻⁹), and cell proliferation (P < 10⁻⁹)(Tables 3 and 6), consistent with earlier observations of increased progenitor cells in LOI(+) mice (Sakatani et al., (2005)).

**Table 6. List of Genes with Dignificant difference (P < 0.01) in Microarray Analysis of Laser Capture Microdissected crypts (Downregulation in LOI(-) crypt).**

| Featureid | Mcan (H19mut, LOI +) | Mean (H19wt, LOI-) | LogRatio | FoldChng | PPFDR | Noisy Symbol | Annotation | Gene Index 'U' Cluster | RefSeqAcc | GenBankAcc | MGI |
|---|---|---|---|---|---|---|---|---|---|---|---|
| Z00027742-1 | 3.5267 | 3.8485 | - 0.321 8 | 2.097 | 0 0.0005 | 0 Grin2d | glutamate receptor, ionotropic, NMDA2D (epsilon 4) | U028546 | NM_008172.1 | AK077611 | MGI: 95823 |
| Z00005302-1 | 3.12 | 3.35259 | - 0.232 59 | 1.708 | 0 0.0015 | 0 Cdknla | cyclin-dependent kinase inhibitor 1A (P21) | U017761 | NM_007669.2 | AB017817 | MGI: 104556 |
| Z00041932-1 | 2.7672 | 2.9276 | - 0.160 4 | 1.446 | 0 0.0075 | 0 Ahnak | Mus musculus AHNAK nucleoprotein (desmovokin) | U168837 | | AK003448 | MGI: 1316648 |
| Z00027819-1 | 3.106 | 3.254 | - 0.148 | 1.406 | 0 0.0306 | 0 Gm502 | gene model 502, (NCBI) | U009440 | XM_146397.2 | BC010572 | MGI: 2685348 |
| Z00011777-1 | 2.9645 | 3.13359 | - 0.169 09 | 1.476 | 0 0.0331 | 0 A630082K20Ri k | RIKEN cDNA A630082K20 gene | U027200 | XM_145254.4 | AK035529 | |
| Z00021589-1 | 3.2141 | 3.38699 | - 0.172 89 | 1.488 | 0 0.0463 | 0 Map3k6 | mitogen-activated protein kinase kinase kinase 6 | U004915 | NM_016693.2 | AB021861 | MGI: 1855691 |
| Z00039323-1 | 2.6312 | 2.76889 | - 0.137 69 | 1.373 | 0 0.0664 | 0 A930008A22Ri k | RIKEN cDNA A930008A22 gene | U030737 | NM_172768.1 | AK020827 | |
| Z00013598-1 | 3.1007 | 3.2446 | - 0.143 | 9 1.392 | 0 0.0717 | 0 Skiip | SKI interacting protein | U034214 | NM_025507.1 | AK009218 | MGI: 1913604 |
| Z00044310-1 | 3.0685 | 3.2893 | - 0.220 8 | 1.662 | 0 0.0937 | 0 1700011H14Ri k | RIKEN cDNA 1700011H14 gene | U035491 | NM_025956.2 | AK005866 | |
| Z00035971-1 | 2.8825 | 3.0066 | - 0.124 1 | 1.33 | 0.0001 | 0 Ckap4 | cytoskeleton-associated protein 4 | U032075 | XM_125808.5 | AK030708 | MGI: 2444926 |
| | | | | | 0.1121 | | | | | | |
| Z00064631-1 | 2.6715 | 2.79369 | - 0.122 19 | 1.324 | 0.0001 | 0 | | | | | |
| | | | | | 0.1194 | | | | | | |
| Z00063868-1 | 2.5884 | 2.71369 | - 0.125 29 | 1.334 | 0.0002 | 0 Herc5 | Mus musculus hect domain and RLD 5 (Herc5), mRNA | U006930 | NM_025992.1 | AK007221 | MGI: 1914388 |
| | | | | | 0.1607 | | | | | | |
| Z00005771-1 | 3.5523 | 3.7683 | - 0.216 | 1.644 | 0.0002 | 0 Klf6 | Kruppel-like factor 6 | U014517 | NM_011803.1 | AF072403 | MGI: 1346318 |
| | | | | | 0.1655 | | | | | | |
| Z00057298-1 | 2.6089 | 2.73129 | - 0.122 39 | 1.325 | 0.0002 | 0 AI9876 | expressed sequence AI987662 | U041037 | NM_178899.3 | AK033728 | |
| | | | | | 0.1801 | 62 | | | | | |
| Z00060167-1 | 3.8979 | 4.07059 | - 0.172 69 | 1.488 | 0.0003 | 0 Chmp4 | chromatin modifying protein 4B | U002691 | NM_029362.2 | AK008205 | |
| | | | | | 0.2387 | b | | | | | |
| Z00055119~1 | 2.6125 | 2.7324 | - 0.1199 | 1.317 | 0.0003 | 0 9530033F24Ri k | Mus musculus RIKEN cDNA 9530033F24 gene (9530033F24Rik ) | U013231 | NM_201609.1 | AK053008 | |
| | | | | | 0.2387 | | | | | | |
| Z00031167-1 | 2.6209 | 2.7358 | - 0.1149 | 1.302 | 0.0004 | 0 Xlr | Mus musculus X-linked lymphocyte-regulated complex (Xlr) | U047383 | NM_011725.2 | AK012549 | |
| | | | | | 0.2407 | | | | | | |
| Z00059181-1 | 2.6076 | 2.76609 | - 0.15849 | 1.44 | 0.0005 | 0 AA536749 | Mus musculus expressed sequence AA536749 (AA536749) | U012687 | NM_012027.1 | AB093269 | MGI: 1349438 |
| | | | | | 0.2551 | | | | | | |
| Z00047700-1 | 2.9974 | 3.14889 | - 0.15149 | 1.417 | 0.0005 | 0 A830006N08Ri k | Mus musculus RIKEN cDNA A830006N08 gene (A830006N08Ri k) | U032762 | NM_183173.1 | AK043538 | |
| | | | | | 0.2551 | | | | | | |
| Z00042720-1 | 3.1281 | 3.2515 | - 0.1234 | 1.328 | 0.0005 | 0 6430527G18Ri k | RIKEN cDNA 6430527G18 gene | U034200 | NM_145836.1 | AF525300 | |
| | | | | | 0.2551 | | | | | | |
| Z00021452-1 | 4.1943 | 4.36869 | - 0.17439 | 1.494 | 0.0005 | 0 Gdpd1 | glycerophosphod iestcr phosphodiesteras e domain containing 1 | U033233 | NM_025638.1 | AK011487 | |
| | | | | | 0.2588 | | | | | | |
| Z00033810-1 | 3.137 | 3.302 | - 0.165 | 1.462 | 0.0006 | 0 | | U064815 | | | |
| | | | | | 0.2903 | | | | | | |
| Z00027804-1 | 2.7244 | 2.9361 | - 0.2117 | 1.628 | 0.0006 | 0 Gpr120 | Mus musculus G protein-coupled receptor 120 (Gpr120) | U100866 | NM_181748.1 | AB115769 | |
| | | | | | 0.3018 | | | | | | |
| Z00041224-1 | 3.2239 | 3.3967 | - 0.1728 | 1.488 | 0.0006 | 0 BC025076 | cDNA sequence BC025076 | U104957 | | AK087807 | |
| | | | | | 0.3018 | | | | | | |
| Z00025480-1 | 2.5353 | 2.6646 | - 0.1293 | 1.346 | 0.0007 | 0 Bmper | BMP-binding endothelial regulator | U010170 | NM_028472.1 | AF454954 | MGI: 1920480 |
| | | | | | 0.3319 | | | | | | |
| Z00004154-1 | 2.8682 | 2.9804 | - 0.1122 | 1.294 | 0.0008 | 0 Ssfa2 | sperm specific antigen 2 | U001964 | NM_080558.3 | AB093303 | MGI: 1917849 |
| | | | | | 0.3335 | | | | | | |
| Z00024307-1 | 2.8206 | 2.93119 | - 0.11059 | 1.29 | 0.0008 | 0 Ly6d | lymphocyte antigen 6 complex, locus D | U036290 | NM_010742.1 | BC025135 | MGI: 96881 |
| | | | | | 0.3457 | | | | | | |
| Z00040747-1 | 2.9132 | 3.0818 | - 0.1686 | 1.474 | 0.0009 | 0 B430201A12Ri k | RIKEN cDNA B430201A12 gene | U024539 | XM_283903.2 | AK005412 | |
| | | | | | 0.349 | | | | | | |
| Z00040571-1 | 2.5402 | 2.64659 | - 0.10639 | 1.277 | 0.0009 | 0 Atp8bl | ATPase, class I, type 8B, member 1 | U038499 | NM_00100148 8.1 | AF395823 | MGI: 1859665 |
| | | | | | 0.3646 | | | | | | |
| Z00026784-1 | 2.974 | 3.08809 | - 0.114 09 | 1.3 | 0.001 | 0 Naaladl 1 | N-acetylated alpha-linked acidic dipeptidase-like 1 | U085390 | NM_001009546.1 | | MGI: 2685810 |
| | | | | | 0.3792 | | | | | | |
| Z00042521-1 | 4.2784 | 4.50009 | - 0.221 69 | 1.666 | 0.0011 | 0 1110006O24Rik | RIKEN cDNA 1110006O24 gene | U026636 | NM_021417.1 | AB041800 | |
| | | | | | 0.3834 | | | | | | |
| Z00016189-1 | 2.6902 | 2.82019 | - 0.129 99 | 1.348 | 0.0012 | 0 Thbs1 | thrombospondin 1 | U002275 | NM_011580.2 | AK080686 | MGI: 98737 |
| | | | | | 0.3878 | | | | | | |
| Z00064702-1 | 2.6282 | 2.73519 | - 0.106 99 | 1.279 | 0.0012 | 0 | | | | | |
| | | | | | 0.3878 | | | | | | |
| Z00023675-1 | 2.5968 | 2.70509 | - 0.108 29 | 1.283 | 0.0013 | 0 Cd3g | CD3 antigen, gamma polypeptide | U030789 | NM_009850.1 | BC027528 | MGI: 88333 |
| | | | | | 0.3949 | | | | | | |
| Z00008387-1 | 3.3617 | 3.4825 | - 0.120 8 | 1.32 | 0.0013 | 0 D16Ertd480e | DNA segment, Chr 16, ERATO Doi 480, expressed | U017162 | NM_144550.2 | AK048789 | |
| | | | | | 0.405 | | | | | | |
| Z00058668-1 | 2.7168 | 2.841 | - 0.124 2 | 1.331 | 0.0015 | 0 2410195B05Rik | RIKEN cDNA 2410195B05 gene | U006185 | NM_030241.2 | AK008845 | |
| | | | | | 0.4327 | | | | | | |
| Z00029694-1 | 3.8014 | 4.0834 | - 0.282 | 1.914 | 0.0015 | 0 5730442P18Rik | RIKEN cDNA 5730442P18 gene | U013415 | | AF215666 | |
| | | | | | 0.4399 | | | | | | |
| 200015708-1 | 2.9609 | 3.08009 | - 0.119 19 | 1.315 | 0.0016 | 0 Elovl7 | ELOVL family member 7, elongation of long chain fatty acids (yeast) | U015234 | NM_029001.2 | AK018616 | |
| | | | | | 0.4486 | | | | | | |
| Z00027266-1 | 2.5555 | 2.6589 | - 0.103 4 | 1.268 | 0.0017 | 0 4930535E21Rik | RIKEN cDNA 4930535E21 gene | U030932 | NM_029212.1 | ABO48860 | MGI: 1922464 |
| | | | | | 0.4579 | | | | | | |
| ZO0032444~1 | 2.7836 | 2.8868 | - 0.103 2 | 1.268 | 0.0019 | 0 Mylc2b | myosin light chain, regulatory B | U038037 | NM_023402.1 | AK002885 | MGI: 107494 |
| | | | | | 0.5016 | | | | | | |
| Z00014295-1 | 3.141 | 3.25059 | - 0.109 59 | 1.287 | 0.0019 | 0 Hsd12 | hydroxysteroid dehydrogenase like 2 | U004381 | NM_024255.1 | AJ293845 | |
| | | | | | 0.5061 | | | | | | |
| Z00024114-1 | 2.5759 | 2.7035 | - 0.127 6 | 1.341 | 0.002 | 0 Cdx1 | caudal type homeo box 1 | U038465 | NM_009880.2 | BC019986 | MGI: 88360 |
| | | | | | 0.5224 | | | | | | |
| Z00009280-1 | 3.419 | 3.543 | - 0.124 | 1.33 | 0.0021 | 0 Nhsl1 | NHS-like 1 | U011305 | NM_173390.2 | AK043447 | MGI: 106390 |
| | | | | | 0.5336 | | | | | | |
| Z00023930-1 | 2.835 | 2.9775 | - 0.142 5 | 1.388 | 0.0021 | 0 Gdf15 | growth differentiation factor 15 | U029924 | NM_011819.1 | AF159571 | MGI: 1346047 |
| | | | | | 0.5336 | | | | | | |
| Z00025459-1 | 2.8792 | 2.9792 | -0.1 | 1.258 | 0.0027 | 0 Arid3a | AT rich interactive domain 3A (Bright like) | U011730 | NM_007880.1 | AK034824 | MGI:13283 60 |
| | | | | | 0.6414 | | | | | | |
| Z00006544-1 | 3.4135 | 3.53379 | - 0.120 29 | 1.319 | 0.0027 | 0 Npepps | aminopeptidase puromycin sensitive | U033337 | NM_008942.1 | BC009653 | MGI:1101358 |
| | | | | | 0.6414 | | | | | | |
| Z00024111-1 | 3.3292 | 3.4378 | - 0.108 6 | 1.284 | 0.0028 | 0 Syt10 | synaptotagmin 10 | U105649 | NM_018803.1 | AK051232 | |
| | | | | | 0.6568 | | | | | | |
| Z00033010-1 | 2.7361 | 2.8298 | - 0.093 7 | 1.24 | 0.0029 | 0 Efemp2 | epidermal growth factor-containing fibulin-like extracellular matrix protein 2 | U051376 | NM_021474.2 | AF104223 | MGI:1891209 |
| | | | | | 0.6608 | | | | | | |
| Z00056587-1 | 2.6828 | 2.7909 | - 0.108 1 | 1.282 | 0.0032 | 0 1810003N24Ri k | RIKEN cDNA 1810003N24 gene | U032536 | NM_025443.1 | AF349950 | |
| | | | | | 0.7043 | | | | | | |
| Z00057248-1 | 2.6492 | 2.7745 | - 0.125 3 | 1.334 | 0.0033 | 0 Xlr | Mus musculus X-linked lymphocyte-regulated complex (Xlr) | U040096 | NM_027510.1 | | |
| | | | | | 0.7043 | | | | | | |
| Z00023103-1 | 2.9645 | 3.08749 | - 0.122 99 | 1.327 | 0.0033 | 0 Clic5 | chloride intracellular channel 5 | U043605 | NM_172621.1 | AK017800 | |
| | | | | | 0.7043 | | | | | | |
| Z00060328-1 | 3.1619 | 3.32199 | - 0.160 09 | 1.445 | 0.0033 | 0 Bzwl | basic leucine zipper and W2 domains 1 | U000306 | NM_025824.2 | AK004784 | |
| | | | | | 0.7116 | | | | | | |
| Z00036150-1 | 3.1027 | 3.20829 | - 0.105 59 | 1.275 | 0.0034 | 0 Fcgrt | Fc receptor, IgG, alpha chain transporter | U028514 | NM_010189.1 | AK008167 | MGI:103017 |
| | | | | | 0.7116 | | | | | | |
| Z00060861-1 | 2.5462 | 2.6452 | - 0.099 | 1.256 | 0.0034 | 0 1810047C23Ri k | RIKEN cDNA 1810047C23 gene | U009309 | NM_138668.1 | AK075795 | |
| | | | | | 0.7166 | | | | | | |
| Z00062597-1 | 2.7107 | 2.80359 | - 0.092 89 | 1.238 | 0.0038 | 0 Fst | foliistatin | U035199 | NM_008046.1 | AK079 916 | |
| | | | | | 0.7706 | | | | | | |
| Z00068378-1 | 3.2094 | 3.3546 | - 0.145 2 | 1.397 | 0.0039 | 0 | | U058896 | | | |
| | | | | | 0.7736 | | | | | | |
| Z00022408-1 | 3.4615 | 3.5743 | - 0.112 8 | 1.296 | 0.0039 | 0 Cited2 | Cbp/p300-interacting transactivator, with Glu/Asp-rich carboxy-terminal domain, 2 | U011296 | NM_010828.1 | AK177398 | MGI:1306784 |
| | | | | | 0.7784 | | | | | | |
| Z00022297-1 | 2.9252 | 3.0228 | - 0.097 6 | 1.251 | 0.004 | 0 Rhoj | ras homolog gene family, member J | U014096 | NM_023275.1 | ABO60651 | MGI:1931551 |
| | | | | | 0.7784 | | | | | | |
| Z00003251-1 | 2.7864 | 2.8812 9 | - 0.094 89 | 1.244 | 0.0041 | 0 | UI-M-FY0-ccp-j-21-0-Ul.r1 NIH_BMAP_FY 0 Mus musculus cDNA clone IMAGE:6822742 | U271068 | | | |
| | | | | | 0.7806 | | | | | | |
| Z00001120-1 | 2.7223 | 2.8132 9 | - 0.090 99 | 1.233 | 0.0042 | 0 Thop 1 | thimct oligopeptidase 1 | U011773 | NM_022653.2 | AF314187 | MGI:1354165 |
| | | | | | 0.7925 | | | | | | |
| Z00015596-1 | 2.922 | 3.1291 9 | - 0.207 19 | 1.611 | 0.0044 | 0 Oact 1 | O-acyltransferase (membrane bound) domain containing 1 | U014692 | NM_153546.1 | AK020281 | |
| | | | | | 0.7934 | | | | | | |
| Z00055229-1 | 2.8801 | 3.0093 9 | - 0.129 29 | 1.346 | 0.0044 | 0 LOC38 0843 | PREDICTED: Mus musculus similar to hypothetical protein FLJ30829 | U014794 | XM_354752.2 | | |
| | | | | | 0.7972 | | | | | | |
| Z00061294-1 | 2.8519 | 2.9483 9 | - 0.096 49 | 1.248 | 0.0044 | 0 | | | | | |
| | | | | | 0.8005 | | | | | | |
| Z00036109-1 | 2.5678 | 2.66 | - 0.092 2 | 1.236 | 0.0046 | 0 Lamb1-1 | laminin B1 subunit 1 | U013837 | NM_008482.1 | AK013952 | MGI:96743 |
| | | | | | 0.8062 | | | | | | |
| Z00018830-1 | 2.8117 | 2.9050 9 | - 0.093 39 | 1.239 | 0.0047 | 0 C1r | complement component 1, r subcomponent | U020779 | NM_023143.1 | AF148216 | |
| | | | | | 0.8062 | | | | | | |
| Z00016016-1 | 2.9404 | 3.0663 9 | - 0.125 99 | 1.336 | 0.0047 | 0 Plec1 | plectin 1 | U036336 | NM_011117.1 | AF188006 | MGI:1277961 |
| | | | | | 0.8062 | | | | | | |
| Z00058245-1 | 3.2352 | 3.3367 | - 0.101 5 | 1.263 | 0.0048 | 0 LOC38 2906 | PREDICTED: Mus musculus similar to Ac2-008 (LOC382906), mRNA | U056292 | XM_356744.1 | | |
| | | | | | 0.8194 | | | | | | |
| Z00059003-1 | 2.6504 | 2.7513 | - 0.100 9 | 1.261 | 0.0056 | 0 Slit2 | slit homolog 2 (Drosophila) | U005564 | NM_178804.2 | AF074960 | MGI:1315205 |
| | | | | | 0.8199 | | | | | | |
| Z00009502-1 | 2.5168 | 2.6107 9 | - 0.093 99 | 1.241 | 0.0057 | 0 Hspb1 | heat shock protein 1 | U006295 | NM_013560.1 | AF047377 | MGI:96240 |
| | | | | | 0.8199 | | | | | | |
| Z00034606-1 | 3.1133 | 3.2264 9 | - 0.113 19 | 1.297 | 0.0055 | 0 Kdelr2 | KDEL (Lys-Asp-Glu-Leu) endoplasmic reticulum protein retention receptor 2 | U006405 | NM_025841.1 | AJ278133 | |
| | | | | | 0.8199 | | | | | | |
| Z00020266-1 | 3.3706 | 3.4799 9 | - 0.109 39 | 1.286 | 0.005 | 0 Arfrp2 | ADP-ribosylation factor related protein 2 | U015265 | NM_172595.1 | AK039965 | |
| | | | | | 0.8199 | | | | | | |
| Z00060766-1 | 2.6172 | 2.7079 | - 0.090 7 | 1.232 | 0.0057 | 0 1300007B12Ri k | RIKEN cDNA 1300007B12 gene | U021785 | NM_020588.1 | AB041592 | |
| | | | | | 0.8199 | | | | | | |
| Z000386 71-1 | 2.6457 | 2.73339 | - 0.08769 | 1.223 | 0.005 | 0 D5Bwg 0834e | DNA segment, Chr 5, Brigham & Women's Genetics 0834 expressed | U026739 | NM_144819.1 | AK028192 | |
| | | | | | 0.8199 | | | | | | |
| Z00056132-1 | 3.3804 | 3.4904 | -0.11 | 1.288 | 0.0057 | 0 Wasl | Wiskott-Aldrich syndrome-like (human) | U027087 | NM_028459.1 | AJ318416 | MGI:1920428 |
| | | | | | 0.8199 | | | | | | |
| Z00024939-1 | 2.5325 | 2.65519 | - 0.12269 | 1.326 | 0.0049 | 0 Irf7 | interferon regulatory factor 7 | U029451 | NM_016850.1 | AK002830 | |
| | | | | | 0.8199 | | | | | | |
| Z00015996-1 | 2.7647 | 2.8515 | - 0.0868 | 1.221 | 0.0052 | 0 Akap2, Palm2 | A kinase (PRKA) anchor protein 2 | U042535 | NM_009649.1 | AF033274 | MGI:1306795 |
| | | | | | 0.8199 | | | | | | |
| Z00068882-1 | 2.5079 | 2.6034 | - 0.0955 | 1.245 | 0.005 | 0 LOC432634 | PREDICTED: Mus musculus LOC432634 (LOC432634), mRNA | U092058 | XM_488625.1 | | |
| | | | | | 0.8199 | | | | | | |
| Z00066890-1 | 2.6898 | 2.7805 | - 0.0907 | 1.232 | 0.0054 | 0 | BY 122082 RIKEN full-length enriched, adult male brain Mus musculus cDNA clone L630004J03 | U149831 | | | |
| | | | | | 0.8199 | | | | | | |
| Z00055441-1 | 3.4335 | 3.5426 | - 0.1091 | 1.285 | 0.0061 | 0 Atp6v0c | ATPase, H+ transporting, V0 subunit | U017709 | NM_025272.2 | AK007610 | MGI:1328318 |
| | | | | | 0.8288 | | | | | | |
| Z00005843-1 | 3.8481 | 4.03849 | - 0.19039 | 1.55 | 0.0061 | 0 Aphla | anterior pharynx defective 1a homolog (C. elegans) | U021958 | | AK178736 | |
| | | | | | 0.8288 | | | | | | |
| Z00043229-1 | 3.0792 | 3.19029 | - 0.11109 | 1.291 | 0.006 | 0 2600009E05Rik | RIKEN cDNA 2600009E05 gene | U023361 | XM_485067.1 | AK011170 | |
| | | | | | 0.8288 | | | | | | |
| | | | | | | | | | | | |
| Z00007361~1 | 2.5133 | 2.60639 | - 0.09309 | 1.239 | 0.0062 | 0 E130014J05Rik | RIKEN cDNA E130014J05 gene | U024673 | | AK033781 | |
| | | | | | 0.8288 | | | | | | |
| Z00035126-1 | 3.0869 | 3.188 | - 0.1011 | 1.262 | 0.0062 | 0 Wasl | Wiskott-Aldrich syndrome-like (human) | U027087 | NM_028459.1 | AJ318416 | |
| | | | | | 0.8288 | | | | | | |
| Z00060705-1 | 2.8653 | 2.9904 | - 0.1251 | 1.333 | 0.0062 | 0 Cd2ap | CD2-associated protein | U037819 | NM_009847.2 | AF077003 | |
| | | | | | 0.8288 | | | | | | |
| Z00067827-1 | 2.8661 | 2.9568 | - 0.0907 | 1.232 | 0.0062 | 0 | | U076078 | | | |
| | | | | | 0.8288 | | | | | | |
| Z00030081~1 | 2.9433 | 3.1123 | - 0.169 | 1.475 | 0.0062 | 0 | | | | | |
| | | | | | 0.8288 | | | | | | |
| Z00063055-1 | 2.9086 | 3.0162 | - 0.1076 | 1.281 | 0.0066 | 0 | Mus musculus cDNA clone | U009314 | | AB120968 | |
| Z00023416-1 | 2.72 64 | 2.8117 | - 0.0853 | 1.217 | 0.0068 | 0 Tnfrsf1 4 | tumor necrosis factor receptor superfamily, member 14 (herpesvirus entry mediator) | U025864 | NM_178931.2 | AF515707 | |
| | | | | | 0.8527 | | | | | | |
| Z00021381-1 | 3.26 92 | 3.38189 | - 0.11269 | 1.296 | 0.0068 | 0 Gyk | glycerol kinase | U039513 | NM_008194.2 | AK008186 | |
| | | | | | 0.8601 | | | | | | |
| Z00022714-1 | 3.28 62 | 3.3849 | - 0.0987 | 1.255 | 0.0071 | 0 Grcc3f | gene rich cluster, C3f gene | U007393 | NM_145130.1 | AK083687 | |
| | | | | | 0.8608 | | | | | | |
| Z00024151-1 | 2.68 91 | 2.7904 | - 0.1013 | 1.262 | 0.007 | 0 Rasgrfl | RAS protein-specific guanine nucleotide-releasing factor 1 | U010844 | NM_011245.1 | AF169826 | |
| | | | | | 0.8608 | | | | | | |
| Z00011773-1 | 2.50 31 | 2.5948 | - 0.0917 | 1.235 | 0.007 | 0 Adamts 2 | a disintegrin-like and metalloprotease (reprolysin type) with thrombospondin type 1 motif, 2 | U012555 | | AK084657 | |
| | | | | | 0.8608 | | | | | | |
| Z00035615-1 | 3.06 93 | 3.15869 | - 0.08939 | 1.228 | 0.0072 | 0 Cobl | cordon-bleu | U032518 | NM_172496.2 | AK028833 | |
| | | | | | 0.8608 | | | | | | |
| Z00066214-1 | 4.02 45 | 4.168 | - 0.1435 | 1.391 | 0.0072 | 0 | | | | | |
| | | | | | 0.8608 | | | | | | |
| Z00043624-1 | 2.55 83 | 2.7086 | - 0.1503 | 1.413 | 0.0073 | 0 Chmp4b | chromatin modifying protein 4B | U002691 | NM_029362.2 | AK008205 | |
| | | | | | 0.8667 | | | | | | |
| Z00040734-1 | 2.84 16 | 2.94469 | - 0.10309 | 1.267 | 0.0073 | 0 4930432B04Rik | RIKEN cDNA 4930432B04 gene | U002126 | XM_130287.6 | AK014169 | |
| | | | | | 0.8701 | | | | | | |
| Z00026828-1 | 3.52 52 | 3.7835 | - 0.2583 | 1.812 | 0.0077 | 1 E030010A14 | Mus musculus hypothetical protein E030010A14 (E030010A14), mRNA | U019314 | NM_183160.1 | AK086895 | |
| | | | | | 0.8976 | | | | | | |
| Z00055606-1 | 3.12 44 | 3.29819 | - 0.17379 | 1.492 | 0.0078 | 0 Tsx | testis specific X-linked gene | U020099 | NM_009440.1 | AK018925 | |
| | | | | | 0.8976 | | | | | | |
| Z00008080-1 | 3.19 37 | 3.38399 | - 0.19029 | 1.549 | 0.008 | 0 Prkcbp1 | protein kinase C bindingprotein 1 | U023664 | NM_027230.2 | AB093284 | |
| | | | | | 0.8993 | | | | | | |
| Z00057842-1 | 2.77 6 | 3.2209 | - 0.4449 | 2.785 | 0.008 | 1 Nptx2 | neuronal pentraxin 2 | U130883 | NM_016789.2 | AF049124 | |
| | | | | | 0.8993 | | | | | | |
| Z00025293-1 | 2.95 41 | 3.043 | - 0.0889 | 1.227 | 0.008 | 0 Gdf1,Lass1 | growth differentiation factor 1 | U127722 | NM_008107.2 | AK053885 | |
| | | | | | 0.9032 | | | | | | |
| Z00030376-1 | 2.9933 | 3.10439 | - 0.11109 | 1.291 | 0.0083 | 0 2700062C07Rik | RIKEN cDNA 2700062C07 gene | U018482 | NM_026529.2 | AK011228 | |
| | | | | | 0.9164 | | | | | | |
| Z00049596-1 | 2.7014 | 2.8426 | - 0.1412 | 1.384 | 0.0083 | 0 9130218O11Rik | RIKEN cDNA 9130218011 gene | U036369 | NM_177820.2 | BC038135 | |
| | | | | | 0.9164 | | | | | | |
| Z00054220-1 | 3.4174 | 3.5219 | - 0.10379 | 1.269 | 0.0084 | 0 Dnajb1 0 | DnaJ (Hsp40) homolog, subfamily B, member 10 | U000470 | NM_020266.1 | AB028858 | |
| | | | | | 0.9186 | | | | | | |
| Z00016776-1 | 2.8349 | 2.92109 | - 0.08619 | 1.219 | 0.0085 | 0 Ssb | Sjogren syndrome antigen B | U001875 | NM_009278.1 | AK017822 | |
| | | | | | 0.9218 | | | | | | |
| Z00026542-1 | 2.6124 | 2.70329 | - 0.09089 | 1.232 | 0.0086 | 0 D130060C09Rik | RIKEN cDNA D130060C09 gene | U002357 | NM_177054.3 | AK080364 | |
| | | | | | 0.9218 | | | | | | |
| Z00036665-1 | 3.5566 | 3.67839 | - 0.12179 | 1.323 | 0.0085 | 0 Atp2c1 | ATPase, Ca++-sequestering | U031277 | NM_175025.2 | AJ551270 | |
| | | | | | 0.9218 | | | | | | |
| Z00018373-1 | 3.6543 | 3.77009 | - 0.11579 | 1.305 | 0.0087 | 0 Smad4 | MAD homolog 4 (Drosophila) | U038549 | NM_008540.2 | AK004804 | |
| | | | | | 0.9218 | | | | | | |
| Z00019928-1 | 2.6567 | 2.7401 | - 0.0834 | 1.211 | 0.009 | 0 D330010C22Rik | RIKEN cDNA 330010C22 gene | U025841 | XM_131865.5 | AK052224 | |
| | | | | | 0.939 | | | | | | |
| Z00009274-1 | 3.0732 | 3.19439 | - 0.12119 | 1.321 | 0.0091 | 0 Sulf2 | sulfatase 2 | U023667 | XM_358343.2 | AK008108 | |
| | | | | | 0.9435 | | | | | | |
| Z00032201-1 | 3.3134 | 3.45679 | - 0.14339 | 1.391 | 0.0095 | 0 | | U025166 | | | |
| | | | | | 0.9555 | | | | | | |
| Z00040312-1 | 2.8784 | 2.9664 | - 0.088 | 1.224 | 0.0093 | 0 Abi3 | ABI gene family, member 3 | U033309 | NM_025659.1 | AK008928 | |
| | | | | | 0.9555 | | | | | | |
| Z00057502-1 | 2.9689 | 3.0567 | - 0.0878 | 1.224 | 0.0095 | 0 Znhit2 | zinc finger, HIT domain containing 2 | U038703 | NM_013859.2 | AF119498 | |
| | | | | | 0.9555 | | | | | | |
| Z00070092-1 | 2.6803 | 2.77349 | - 0.09319 | 1.239 | 0.0095 | 0 | | | | | |
| | | | | | 0.9555 | | | | | | |
| Z00006417-1 | 2.4712 | 2.55929 | - 0.08809 | 1.224 | 0.0096 | 0 Hs3st3b 1 | Mus musculus heparan sulfate (glucosamine) 3-0-sulfotransferase 3B1 (Hs3sL3b1), mRNA | U032915 | NM_018805.1 | AF168992 | |
| | | | | | 0.9593 | | | | | | |
| Z00006170-1 | 3.2905 | 3.39939 | - 0.10889 | 1.284 | 0.0097 | 0 Cpne3 | copine III | U051430 | NM_027769.1 | AK017357 | |
| | | | | | 0.9593 | | | | | | |
| Z00024368-1 | 4.3807 | 4.93979 | - 0.559 09 | 3.623 | 0.0099 | 1 Slc6a9 | solute carrier family 6 (neurotransmitter transporter, glycine), member 9 | U004714 | NM_008135.1 | AK014572 | |
| | | | | | 0.9675 | | | | | | |
| Z00036435-1 | 2.6826 | 2.7806 | - 0.098 | 1.253 | 0.0099 | 0 Pcdhal 1 and others | protocadherin alpha 11 | U018601 | NM_00100367 1.1 | AB008178 | |
| | | | | | 0.9675 | | | | | | |

These results were confirmed by real-time quantitative RT-PCR analysis of 15,000 additional crypts microdissected from an additional 12 LOI(+) and 9 LOI(-) mice (Fig. 2A). The expected doubling of Igf2 mRNA levels was also confirmed in LOI(+) mice (Fig. 2A). The top ranking genes showing altered expression with LOI included: Cdc6, 1.55-fold (P = 0.003), an essential licensing factor leading to initiation of DNA replication and onset of S-phase (Dutta (1997); Coleman (1996)); *Mcm5,* 1.47-fold (P = 0.007) and *Mcm3,* 1.49-fold (P = 0.002), both required for DNA replication at early S-phase (18, 19); *Chaf1a,* 1.61-fold (P = 0.009), which assembles the histone octamer onto replicating DNA (20); Lig1, 1.54-fold (P = 0.008), DNA ligase involved in joining Okazaki fragments during DNA replication (Tomkinson and Mackey, Mutat Res (1998) 407:1-9); and *Ccne1,* 1.38-fold (P = 0.04), which stimulates replication complex assembly by cooperating with *Cdc6* (Coverley et al., Nat Cell Biol (2992) 4:523-528)) (Fig. 2A, Table 4, *supra*).

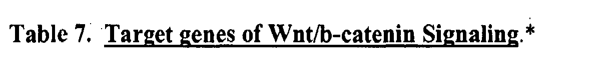

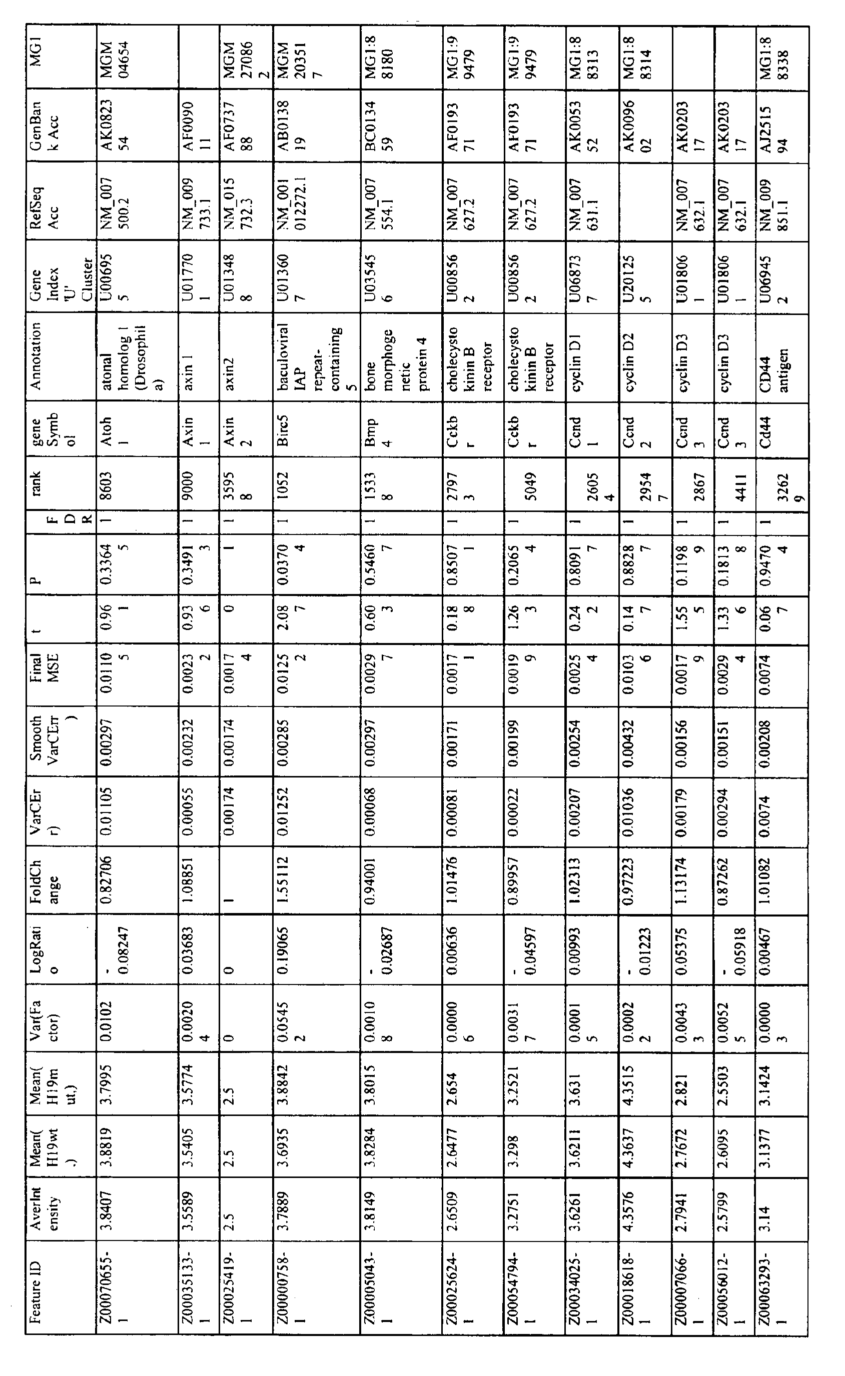

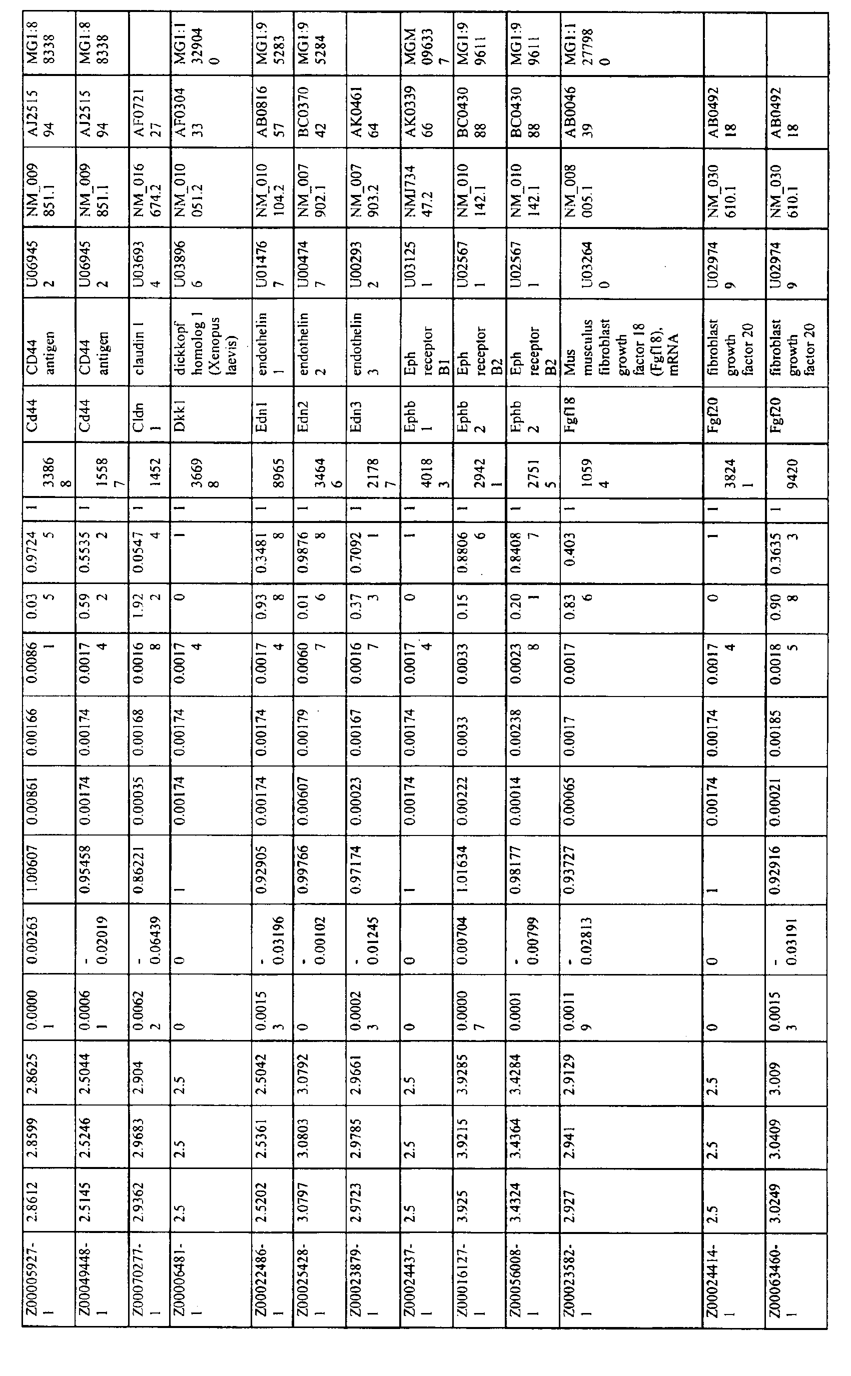

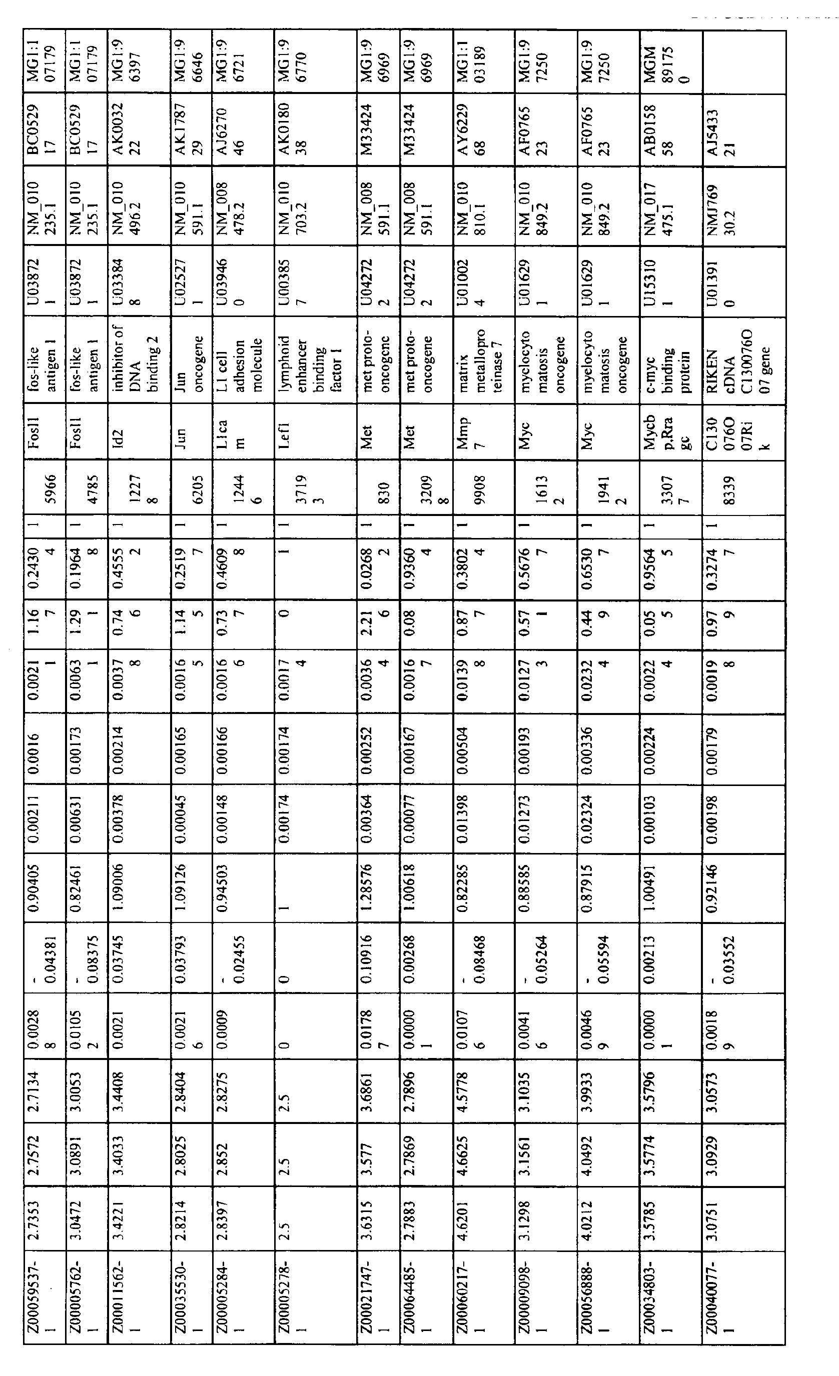

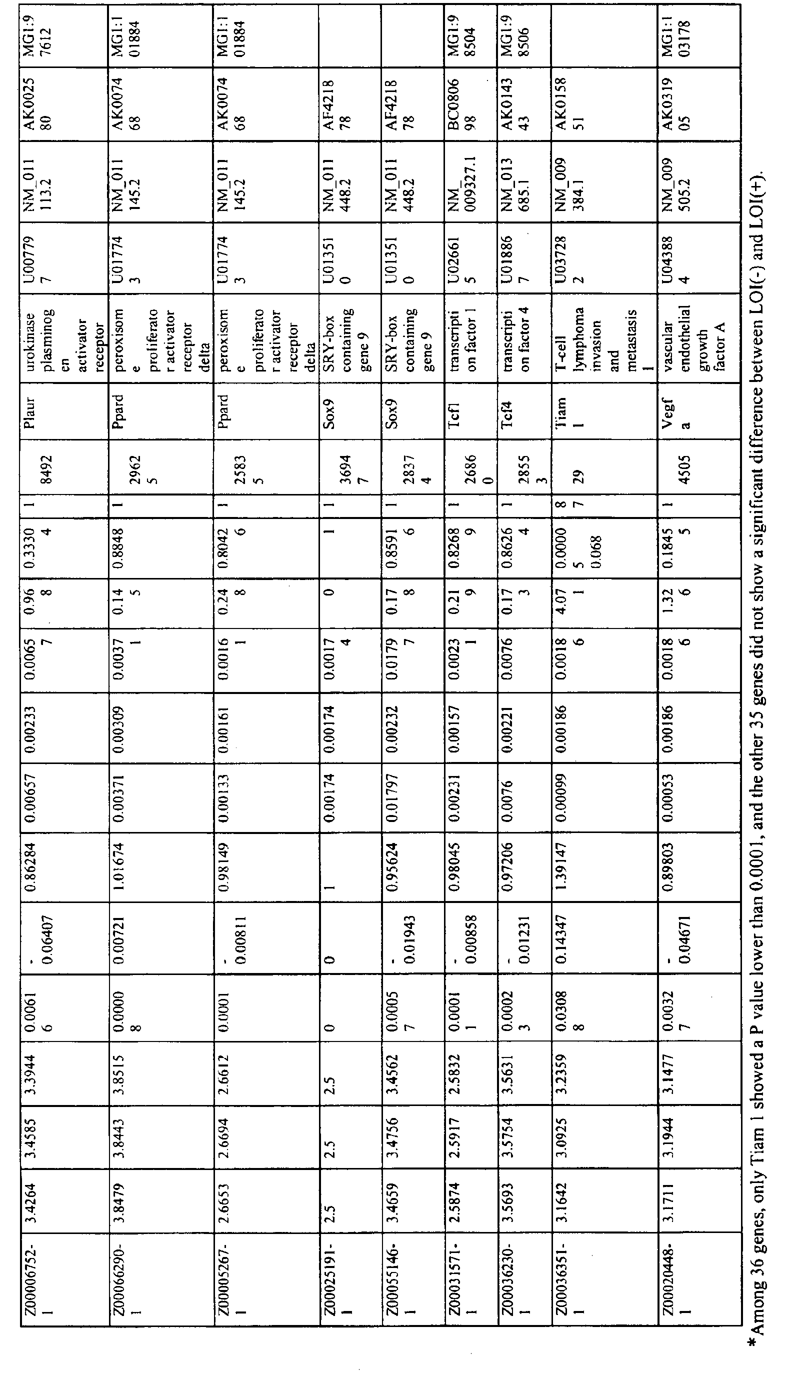

Four LOI(+) and four LOI(-) mice were also treated with NVP-AEW541 to inhibit IGF2 signaling, at a dose of 50 mg/kg by oral gavage daily for 3 weeks (twice daily except daily on weekends). NVP-AEW541 is an ATP-competitive inhibitor of IGF-IR which blocks signaling at the IGF1 receptor, which mediates IGF2 signaling (Garcia-Echeverria et al., Cancer Cell (2004) 5:231-239). That NVP-AEW541 blocks IGF2 at IGF1R in vitro (Fig. 9) was also confirmed. Interestingly, NVP-AEW541 had a dramatic effect on expression of proliferation-related genes in LOI(+) crypts, with reduction to levels even lower than those seen in LOI(-) crypts (5 of 6 genes statistically significant): *Cdc6,* 0.49-fold (P = 0.048); *Mcm5,* 0.48-fold (P = 0.007); *Mcm3,* 0.65-fold (P = 0.1); *Chaf1a,* 0.42-fold (P = 0.010); *Lig1,* 0.42-fold (P = 0.029); and *Ccne1,* 0.57-fold (P = 0.030)(Fig. 2B). Thus, LOI-induced changes in proliferation-related gene expression were mediated, at least in part, through IGF2 signaling itself. The drug-induced decrease in the expression of proliferation-related genes did not occur simply due simply to changes in numbers of proliferating crypt cells, because there were approximately the same number of cells in this short term treatment.

These results imply that LOI causes a specific alteration in replication-associated gene expression in intestinal epithelium. Nevertheless, increased expression of some genes not associated with DNA replication per se was observed. For example, *Card11* (1.44-fold, P = 0.04; Fig. 11) is an anti-apoptotic gene acting through phosphorylation of BCL10 and induction of NF-κB (Narayan et al., Mol Cell Biol (2006) 26:2327-2336). Expression of *Msi1* was also analyzed by real-time PCR, as the encoded progenitor cell marker *Musashi-1* showed increased immunostaining in previous studies. Expression of *Msi1* was also significantly increased (1.49-fold, P = 0.01, Fig. 11), supporting a pleiotropic mechanism for IGF2 in LOI. In addition, several genes showed down regulation in LOI(+) crypts (Table 2, *supra*), including *p21* (0.55-fold, P = 0.007, Fig. 11), an inhibitor of cell cycle progression (Gartel et al., Proc Soc Exp Biol Med (1996) 213:138-149).

### Example 5. Enhanced Sensitivity of the IGF2 Signaling Network in LOI.

The in vivo experiments described led to the determination of whether LOI(+) cells have differential sensitivity to IGF2 and the NVP-AEW541 where a high throughput signal transduction assay was performed based on an immunostaining automation device comprising microfluidic chambers housing multiple cells. An advantage of the microfluidic chip is that all the cells can be cultured simultaneously in the same chip and under internally controlled conditions, with precise determination of the cell micro-environment over the time of the experiment and subsequent analysis, allowing a much larger number of measurements than would be possible by conventional means. The device was constructed within a monolithic 2-layer PDMS chip sealed with a glass coverslip, with defined media delivery controlled by a multiplexed system of valves. Signaling of Akt/PKB and Erk2, two canonical signaling pathways activated by IGF2, was also examined, having derived for this purpose mouse embryo fibroblast (MEF) lines from LOI(+) and LOI(-) embryos. Live LOI(+) and LOI(-) cells were stimulated with varying doses of IGF2, for varying periods of time, fixed and processed for Akt/PKB measurements, with all steps performed within the chip.

For each cell type, IGF2 concentration, and time point, at least 200 individual cellular measurements were obtained by digital imaging and analysis, providing ample information for statistically significant evaluation of both the average response and cell-cell variability. The results were consistent in chip-to-chip variation analysis, with two chips used for each cell line. As a read-out we used immunostaining of the nuclear phosphorylated Akt (Ser 473) due to its nuclear activity in regulation of FOXO (Shao et al., Embo J (1999) 18:1397-1406) and other proteins controlling cell cycle progression, as well as possible interactions with modifiers of histone modulation (Garcia Esheverria et al., Cancer Cell (20040) 5:231-239).

IGF2 triggered a transient Akt activation signal (peak at 10 to 40 minutes followed by a return to the baseline within 90 min.) in LOI(-) cells (Fig. 8A) at all concentrations tested (400, 800, and 1600 ng/ml), comparable to levels used to support mouse fetal liver hematopoietic stem cells (500-1000 ng/ml) (Zhang and Lodish (2004)). In contrast, when subjected to the lowest (400 ng/ml) IGF2 concentration, LOI(+) cells showed markedly sustained Akt activation (> 120 minutes), which increased steadily over time after stimulation (Fig. 8A). At higher IGF2 doses, the Akt signal in LOI(+) cells became progressively more transient and less pronounced (Fig. 8A). Furthermore, if NVP-AEW541 was added alongside IGF2, the Akt activation was inhibited to the baseline levels in LOI(-) cells and significantly below the baseline in LOI(+) cells (Fig. 8B). Signaling differences in Erk2 between LOI(-) and LOI(+), though statistically significant, were very small compared to the effect of LOI on Akt activation (Fig. 8C), suggesting that Akt has a particularly important role in IGF2 response in these cells. These results demonstrate that Akt response in LOI(+) cells has enhanced sensitivity to IGF2 at lower doses as well as hypersensitivity to IGF1R inhibition.

One potential mechanism of this hypersensitivity might be based on differential expression of the components of the underlying signaling network, e.g., the IGF1 receptor, which is the primary signaling receptor for IGF2, or members of the insulin receptor family sensitive to IGF2 (33). The expression of Igf1r, *IgJ2r*, whose protein product is a sink for IGF2, and Insr, in MEFs showing the altered signaling response was analyzed. Strikingly, a doubling of Igf1r expression and Insr in LOI(+)cells (Fig. 8D) was observed. Although, the reasons for altered expression of Igf1r and Insr are not clear at this point, this change in the expression of these receptors provides an intriguing model for alterations in signaling sensitivity in LOI.

### Example 6. LOI Increases Premalignant Lesion Formation in the AOM/LOI Model, Which Shows Enhanced Sensitivity to IGF2 Signaling Inhibition.

Based on these results, it was of interest to determine whether IGF2 signaling inhibition would inhibit in vivo carcinogenesis, or even show an enhanced chemopreventive effect. Treatment with NVP-AEW541 requires twice daily gavage, and Min mice develop lesions over a longer period of time than was practical for use of this drug. In addition, a limitation of the Min model is that it does not reflect the human situation, in which LOI occurs in normal cells before the Apc mutation is present (Cui et al., Nat Med (1998) 4:1276-1280). Accordingly, the azoxymethane (AOM) model was used in which the carcinogen is administered postnatally, and premalignant lesions termed aberrant crypt foci (ACF) appear 5 weeks after the first dose. An additional advantage is that the AOM is a widely studied rodent colon cancer model (Bissahoyo et al., Toxicol Sci (2005) 88:340-345; Bird (1987)).

Eight LOI(+)and 14 LOI(-) mice were given AOM intraperitoneally weekly for 3 weeks, sacrificed at 5 weeks after the first dose, and ACFs were scored as described (Bird (1987)). Histologic examination of colons from AOM-treated mice confirmed the presence of ACFs, with hyperproliferative features including increased mitosis, crypt enlargement and crypt disarray (Fig. 14A, B). LOI(+) mice showed 19.8 ± 2.2 ACF per colon, compared to 12.4 ± 0.9 ACF per colon in LOI(-) mice, a 60% increase (P = 0.002; Fig. 14A).

An additional 9 LOI(+) mice and 9 LOI(-) control littermates to AOM were similarly exposed, adding treatment with NVP-AEW541 to inhibit IGF2 signaling, at a dose of 50 mg/kg by oral gavage daily for 6 weeks (twice daily except daily on weekends), starting one week prior to AOM administration. LOI(-) mice showed no difference in ACF formation after NVP-AEW541 drug treatment (11.3 ± 1.6, N.S.; Fig. 14A). Surprisingly, LOI(+) mice showed a striking reduction in AOM-induced ACF formation after NVP-AEW541 treatment (7.8 ± 1.2, P = 0.0002; Fig. 14A), significantly lower even than that seen in LOI(-) AOM-treated mice (P = 0.007).

Since LOI also leads to an increase in birth weight and therefore potentially in the size of the colon, the number of ACFs to colon surface area was normalized. A similar increase in ACFs in LOI(+) mice, 59% (P = 0.004) was observed, as was a similar decrease in LOI(+) mice treated with the inhibitor, 56% (P = 0.0008), but there was no decrease in ACFs with inhibitor in LOI(-) mice (Fig. 14B). Thus, LOI of Igf2 increased the sensitivity to AOM through an IGF1R-dependent mechanism, and LOI(+) mice were more sensitive to the effects of IGF1R blockade than were LOI(-) mice.

An additional intriguing finding in AOM-treated LOI(+) mice was cystically dilated crypts lined by enlarged cells with atypical nuclei and containing necrotic debris, that were reminiscent of sessile serrated adenomas (SSAs) seen in the human colon (SI Fig. 13C,D). SSAs also show crypt dilatation in association with cytologic atypia and are currently of immense interest for their recently recognized association with colorectal cancer (Sonver et al. (2005)).

Although the invention has been described with reference to the above examples, it will be understood that modifications and variations are encompassed within the spirit and scope of the invention. Accordingly, the invention is limited only by the following claims.

Since it was reported that IGF2 caused relocation of β-catenin to the nucleus in vitro and activated transcription of target genes of the β-catenin/TCF4 complex, (Morali et al., Oncogene (2001) 20:4942-4950)) whether Wnt signaling is activated in LOI(+) intestinal crypts was determined. However, among 36 target genes of Wnt/β-catenin signaling, only Tiam1, a Wnt-responsive Rac GTPase activator (Malliri et al., J Biol Chem (2006) 281:543-548), showed a P value lower than 0.0001, and the other 35 genes did not show significant differences between LOI(-) and LOI(+) crypts (Table 7, *supra*). Furthermore, no significant increase was detected in real-time RT-PCR of Tiam1 (1.16-fold, P = 0.5), nor was the well known target gene *Axin2* (Yan et al., Proc Natl Acad Sci USA (2001) 98:14973-14978; Vogt et al., Cell Cycle (2005) 4:908-913 29) (1.19-fold, P = 0.4) (Fig. 10). Thus activation of *Wnt* signaling does not appear to be involved in the increase of progenitor cells in LOI(+) crypts.

## Claims

1. A method of determining a pharmacological intervention strategy for an individual, the method comprising
determining an insulin-like growth factor 2 (IGF2) loss of imprinting (LOI) status from a sample taken from the individual; and
determining a pharmacological intervention strategy for the individual based on the determined LOI status, wherein if the individual has LOI and therefore has or is at risk of developing colorectal cancer, the intervention strategy comprises administering to the individual an inhibitor of signal pathway activation by IGF2, and if the individual does not have LOI, the intervention strategy does not comprise administering said inhibitor to the individual.

2. A method of identifying an increased risk of developing colorectal cancer in a subject comprising:
a) contacting a progenitor cell in a sample from a subject with insulin-like growth factor 2 (IGF2;
b) determining the IGF2 sensitivity of the progenitor cell by measuring a change in the IRS-1/PI3K/AKT signal pathway or the GRB2/Ras/ERK signal pathway; and
c) comparing the IGF2 sensitivity of the progenitor cell to the IGF2 sensitivity of a cell lacking IGF2 LOI; wherein an increase in the IGF2 sensitivity of the progenitor cell as compared to the cell lacking IGF2 LOI correlates with an increased risk of developing colorectal cancer.

3. The method of claim 2, further comprising contacting the progenitor cell with IGF2 in the presence of an inhibitor of IGF1 receptor.

4. The method of claim 3, wherein the inhibitor is an agent selected from the group consisting of a tyrphostin, a pyrrolo[2,3-d]-pyrimidine, and a monoclonal antibody.

5. A method of assessing the efficacy of a chemotherapeutic regimen comprising:
a) periodically isolating a progenitor cell in a sample from a subject receiving a chemotherapeutic, the subject having, or being at risk of developing colorectal cancer;
b) contacting the progenitor cell in the sample with insulin-like growth factor 2 (IGF2); and
c) determining the sensitivity of the progenitor cell to IGF2 by measuring a change in the IRS-1/PI3K/AKT signal pathway or the GRB2/Ras/ERK signal pathway, wherein a reduction in sensitivity to IGF2 as compared to a previously determined level of sensitivity exhibited by a progenitor cell from the individual correlates with increased efficacy of the regimen.

## Patentansprüche

1. Verfahren zum Bestimmen einer pharmakologischen Interventionsstrategie für ein Individuum, wobei das Verfahren Folgendes umfasst:
Bestimmen eines Imprinting-Verlust-Status ("Loss of Imprinting" (LOI)-Status) des insulinartigen Wachstumsfaktors 2 ("Insulin-like growth factor" 2, IGF2) aus einer dem Individuum entnommenen Probe; und Bestimmen einer pharmakologischen Interventionsstrategie für das Individuum auf der Grundlage des bestimmten LOI-Status, wobei die Interventionsstrategie das Verabreichen eines Hemmstoffes der Signalwegaktivierung durch IGF2 umfasst, wenn das Individuum LOI aufweist und daher Kolorektalkrebs hat oder einem Risiko zur Entwicklung von Kolorektalkrebs ausgesetzt ist, und wobei die Interventionsstrategie das Verabreichen des Hemmstoffes an das Individuum nicht umfasst, wenn das Individuum keinen LOI aufweist.

2. Verfahren des Identifizierens eines erhöhten Risikos zur Entwicklung von Kolorektalkrebs in einem Individuum, umfassend:
a) In-Berührung-bringen einer Vorläuferzelle in einer Probe von einem Individuum mit insulinartigem Wachstumsfaktor 2 ("Insulin-like growth factor" 2, IGF2);
b) Bestimmen der IGF2-Empfindlichkeit der Vorläuferzelle durch Messen einer Veränderung im IRS-1/PI3K/AKT-Signalweg oder im GRB2/Ras/ERK-Signalweg; und
c) Vergleichen der IGF2-Empfindlichkeit der Vorläuferzelle mit der IGF2-Empfindlichkeit einer Zelle ohne IGF2-LOI; wobei eine Erhöhung der IGF2-Empfindlichkeit der Vorläuferzelle im Vergleich zu der Zelle ohne IGF2-LOI mit einem erhöhten Risiko zur Entwicklung von Kolorektalkrebs korreliert.

3. Verfahren nach Anspruch 2, ferner umfassend das In-Berührung-bringen der Vorläuferzelle mit IGF2 in Gegenwart eines Hemmstoffes des IGF1-Rezeptors.

4. Verfahren nach Anspruch 3, wobei der Hemmstoff ein Stoff ist, der aus der Gruppe ausgewählt ist, die aus Tyrphostin, einem Pyrrolo[2,3-d]-pyrimidin und einem monoklonalen Antikörper besteht.

5. Verfahren des Beurteilens der Wirksamkeit einer chemotherapeutischen Behandlung, umfassend:
a) regelmäßiges Isolieren einer Vorläuferzelle in einer Probe von einem Individuum, das ein Chemotherapeutikum erhält, wobei das Individuum Kolorektalkrebs hat oder einem Risiko zur Entwicklung von Kolorektalkrebs ausgesetzt ist;
b) In-Berührung-bringen der Vorläuferzelle in der Probe mit insulinartigem Wachstumsfaktor 2 ("Insulin-like growth factor" 2, IGF2);
c) Bestimmen der Empfindlichkeit der Vorläuferzelle auf IGF2 durch Messen einer Veränderung im IRS-1/PI3K/AKT-Signalweg oder im GRB2/Ras/ERK-Signalweg, wobei eine Verringerung der Empfindlichkeit auf IGF2 im Vergleich zu einem zuvor bestimmten Grad der Empfindlichkeit, die eine Vorläuferzelle von dem Individuum aufweist, mit einer erhöhten Wirksamkeit der Behandlung korreliert.

## Revendications

1. Une méthode de sélection de la stratégie d'intervention pharmacologique à adopter pour un patient, méthode impliquant de :
déterminer l'état de la diminution de l'expression du facteur de croissance insulinomimétique de type 2 (IGF2) à partir d'un échantillon prélevé sur le patient ; et
sélectionner la stratégie d'intervention à adopter pour le patient en fonction de l'état déterminé pour la diminution d'expression : si le patient souffre d'une diminution d'expression et, par conséquent, souffre d'un cancer colorectal ou risque d'en développer un, la stratégie d'intervention implique l'administration au patient d'un inhibiteur de l'activation des voies de signalisations par l'IGF2 ; tandis que si le patient ne souffre pas de diminution d'expression, la stratégie d'intervention n'implique pas l'administration dudit inhibiteur.

2. Une méthode d'identification du risque accru de développer un cancer colorectal chez un patient, méthode impliquant :
a) de contacter une cellule souche dans un échantillon prélevé sur un patient possédant le facteur de croissance insulinomimétique de type 2 (IGF2) ;
b) de déterminer la sensibilité de la cellule souche à l'IGF2 en mesurant une modification de la voie de signalisation IRS-1/P13K/Akt ou de la voie de signalisation GRB2/Ras/ERK; et
c) de comparer la sensibilité de la cellule souche à l'IGF2 à la sensibilité à l'IGF2 d'une cellule ne souffrant pas d'une perte d'expression de l'IGF2 ; auquel cas une augmentation de la sensibilité de la cellule souche à l'IGF2 par rapport à celle d'une cellule ne souffrant pas d'une perte d'expression de l'IGF2 est corrélée à un risque accru de développer un cancer colorectal.

3. La méthode décrite à la revendication 2, impliquant en outre de contacter la cellule souche avec IGF2 en présence d'un inhibiteur du récepteur de l'IGF1.

4. La méthode décrite à la revendication 3, dans le cadre de laquelle l'inhibiteur est un agent sélectionné au sein d'un groupe composé d'une tyrphostine, d'une pyrrolo[2,3-d]-pyrimidine et d'un anticorps monoclonal.

5. Une méthode d'évaluation de l'efficacité d'un traitement chimiothérapeutique impliquant de :
a) isoler périodiquement une cellule souche dans un échantillon prélevé sur un patient recevant une chimiothérapie, ledit patient souffrant d'un cancer colorectal ou risquant d'en développer un ;
b) de contacter dans l'échantillon la cellule souche possédant le facteur de croissance insulinomimétique de type 2 (IGF2) ; et
c) de déterminer la sensibilité de la cellule souche à l'IGF2 en mesurant une modification de la voie de signalisation IRS-1/PI3K/Akt ou de la voie de signalisation GRB2/Ras/ERK, sachant qu'une diminution de la sensibilité de la cellule souche à l'IGF2 par rapport à la sensibilité précédemment démontrée par une cellule souche du patient est corrélée à une augmentation de l'efficacité du traitement.


### SEQUENCE LISTING

<110> THE JOHNS HOPKINS UNIVERSITY FEINBERG, Andrew P. LEVCHENKO, Andre LONGO, Dan L. KO, Minoru S.H.
<120> CANCER CHEMOPREVENTION STRATEGY BASED ON LOSS OF IMPRINTING OF IGF2
<130> JHU3010-1WO
<150> US 60,873,830
   <151> 2006-12-08
<160> 39
<170> PatentIn version 3.4
<210> 1
   <211> 23
   <212> DNA
   <213> Artificial sequence
<220>
   <223> Primer
<400> 1
   tcccctcgcc tagtctggaa gca 23
<210> 2
   <211> 21
   <212> DNA
   <213> Artificial sequence
<220>
   <223> Primer
<400> 2
   gaactgttcg ccaggctcaa g 21
<210> 3
   <211> 23
   <212> DNA
   <213> Artificial sequence
<220>
   <223> Primer
<400> 3
   acagcagaca gcaaggggag ggt 23
<210> 4
   <211> 20
   <212> DNA
   <213> Artificial sequence
<220>
   <223> Primer
<400> 4
   catcgtggaa gagtgctgct 20
<210> 5
   <211> 19
   <212> DNA
   <213> Artificial sequence
<220>
   <223> Primer
<400> 5
   gggtatctgg ggaagtcgt 19
<210> 6
   <211> 21
   <212> DNA
   <213> Artificial sequence
<220>
   <223> Primer
<400> 6
   aacacagaag acagctcctc a 21
<210> 7
   <211> 22
   <212> DNA
   <213> Artificial sequence
<220>
   <223> Primer
<400> 7
   gtctgaatcg atggtaaacc tg 22
<210> 8
   <211> 21
   <212> DNA
   <213> Artificial sequence
<220>
   <223> Primer
<400> 8
   cacttcaagg agcagctcag c 21
<210> 9
   <211> 20
   <212> DNA
   <213> Artificial sequence
<220>
   <223> Primer
<400> 9
   gctcagtcga tcctctccac 20
<210> 10
   <211> 21
   <212> DNA
   <213> Artificial sequence
<220>
   <223> Primer
<400> 10
   atggatgttc gtcagtgggt t 21
<210> 11
   <211> 22
   <212> DNA
   <213> Artificial sequence
<220>
   <223> Primer
<400> 11
   ccagaggaat gatcttaaag gc 22
<210> 12
   <211> 20
   <212> DNA
   <213> Artificial sequence
<220>
   <223> Primer
<400> 12 20
   gaagacgagg tgctcaatgc 20
<210> 13
   <211> 20
   <212> DNA
   <213> Artificial sequence
<220>
   <223> Primer
<400> 13
   cctttgtccc ttggtgtgaa 20
<210> 14
   <211> 22
   <212> DNA
   <213> Artificial sequence
<220>
   <223> Primer
<400> 14
   gggacatcag cctggtaata ga 22
<210> 15
   <211> 22
   <212> DNA
   <213> Artificial sequence
<220>
   <223> Primer
<400> 15
   cttagacaga ttggcaggtg aa 22
<210> 16
   <211> 20
   <212> DNA
   <213> Artificial sequence
<220>
   <223> Primer
<400> 16
   tgtggagtcg gatgtcagga 20
<210> 17
   <211> 21
   <212> DNA
   <213> Artificial sequence
<220>
   <223> Primer
<400> 17
   gggatatgtg agcaagacca a 21
<210> 18
   <211> 21
   <212> DNA
   <213> Artificial sequence
<220>
   <223> Primer
<400> 18
   ccaggtcatg ctcaagtcag a 21
<210> 19
   <211> 22
   <212> DNA
   <213> Artificial sequence
<220>
   <223> Primer
<400> 19
   gaatggagat acgagtagcc tt 22
<210> 20
   <211> 23
   <212> DNA
   <213> Artificial sequence
<220>
   <223> Primer
<400> 20
   cgcagagaga ctacttggac ttc 23
<210> 21
   <211> 21
   <212> DNA
   <213> Artificial sequence
<220>
   <223> Primer
<400> 21
   agccgatact ggttgtcact g 21
<210> 22
   <211> 20
   <212> DNA
   <213> Artificial sequence
<220>
   <223> Primer
<400> 22
   agtcaagggc aaagggagtg 20
<210> 23
   <211> 21
   <212> DNA
   <213> Artificial sequence
<220>
   <223> Primer
<400> 23
   gaggcacaga caggaaaaga t 21
<210> 24
   <211> 24
   <212> DNA
   <213> Artificial sequence
<220>
   <223> Primer
<400> 24
   tcccagtgaa gaggttaata caag 24
<210> 25
   <211> 23
   <212> DNA
   <213> Artificial sequence
<220>
   <223> Primer
<400> 25
   gatgtgtctt cctcaacttt ctc 23
<210> 26
   <211> 22
   <212> DNA
   <213> Artificial sequence
<220>
   <223> Primer
<400> 26
   cggacatttg agaagattgc gg 22
<210> 27
   <211> 23
   <212> DNA
   <213> Artificial sequence
<220>
   <223> Primer
<400> 27
   agatagagaa cagggagcaa gtc 23
<210> 28
   <211> 23
   <212> DNA
   <213> Artificial sequence
<220>
   <223> Primer
<400> 28
   tgaaaataag gatgttggag acc 23
<210> 29
   <211> 22
   <212> DNA
   <213> Artificial sequence
<220>
   <223> Primer
<400> 29
   gccacttctt tccaatactg ag 22
<210> 30
   <211> 22
   <212> DNA
   <213> Artificial sequence
<220>
   <223> Primer
<400> 30
   ccaccttgaa gttaatccca gt 22
<210> 31
   <211> 23
   <212> DNA
   <213> Artificial sequence
<220>
   <223> Primer
<400> 31
   tgtccacctc tgtcaataat acc 23
<210> 32
   <211> 22
   <212> DNA
   <213> Artificial sequence
<220>
   <223> Primer
<400> 32
   agttcttctg gattggctga tg 22
<210> 33
   <211> 23
   <212> DNA
   <213> Artificial sequence
<220>
   <223> Primer
<400> 33
   gtaacgatca aagaagtcct gtg 23
<210> 34
   <211> 19
   <212> DNA
   <213> Artificial sequence
<220>
   <223> Primer
<400> 34
   tgctgggtat tgggatgct 19
<210> 35
   <211> 20
   <212> DNA
   <213> Artificial sequence
<220>
   <223> Primer
<400> 35
   tcggggaact ggtaggtgta 20
<210> 36
   <211> 23
   <212> DNA
   <213> Artificial sequence
<220>
   <223> Primer
<400> 36
   acagcgatat ccagacattc aga 23
<210> 37
   <211> 20
   <212> DNA
   <213> Artificial sequence
<220>
   <223> Primer
<400> 37
   cgaagagaca acggcacact 20
<210> 38
   <211> 20
   <212> DNA
   <213> Artificial sequence
<220>
   <223> Primer
<400> 38
   taccaccatg tacccaggca 20
<210> 39
   <211> 20
   <212> DNA
   <213> Artificial sequence
<220>
   <223> Primer
<400> 39
   ggaggagcaa tgatcttgat 20